(19) **Europäisches Patentamt**
**European Patent Office**
**Office européen des brevets**

(11) **EP 3 043 706 B1**

(12) **FASCICULE DE BREVET EUROPEEN**

(45) Date de publication et mention
de la délivrance du brevet:
**25.10.2017 Bulletin 2017/43**

(21) Numéro de dépôt: **14756088.2**

(22) Date de dépôt: **14.08.2014**

(51) Int Cl.:
*A61B 5/107* *(2006.01)*    *A61B 5/00* *(2006.01)*
*G06F 19/00* *(2011.01)*

(86) Numéro de dépôt international:
**PCT/FR2014/000187**

(87) Numéro de publication internationale:
**WO 2015/036661 (19.03.2015 Gazette 2015/11)**

(54) **DISPOSITIF ET PROCÉDÉ D'ÉVALUATION ET DE SURVEILLANCE DE DOULEURS PHYSIQUES**

VORRICHTUNG UND VERFAHREN ZUR BEWERTUNG UND ÜBERWACHUNG VON PHYSISCHEM SCHMERZ

DEVICE AND METHOD FOR EVALUATING AND MONITORING PHYSICAL PAIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**ME**

(30) Priorité: **13.09.2013 FR 1358850**

(43) Date de publication de la demande:
**20.07.2016 Bulletin 2016/29**

(73) Titulaire: **Centre Hospitalier Universitaire de Poitiers**
**86000 Poitiers (FR)**

(72) Inventeurs:
• **RIGOARD, Philippe**
**86000 Poitiers (FR)**
• **GUETARNI, Farid**
**86300 Chauvigny (FR)**

(74) Mandataire: **Cabinet Netter**
**36, avenue Hoche**
**75008 Paris (FR)**

(56) Documents cités:
**EP-A1- 0 618 528       EP-A1- 2 518 594**
**WO-A2-2004/041080    US-A1- 2009 005 649**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

L'invention, qui est définie dans la revendication indépendante 1 et les revendications dépendantes 2 à 17, se rapporte au domaine médical, et notamment à l'étude de la douleur. Elle s'applique notamment, mais non exclusivement, à la technique de stimulation médullaire, en particulier pour la prise en charge des douleurs rachidiennes post-opératoires.

## État de la technique

1. Lorsqu'un praticien veut mieux connaître la douleur de son patient, le plus simple est qu'il lui demande de la décrire, en usant au besoin d'outils auxiliaires comme l'échelle visuelle analogique (EVA), l'échelle verbale simple (EVS), l'échelle numérique (EN), ou d'autres échelles plutôt destinées aux douleurs chroniques.

Ces échelles sont des estimations de l'intensité de la douleur, assez approximatives. Elles ne donnent pas d'informations sur l'étendue de la douleur dans l'espace et ses variations d'intensité locales, lesquelles vont être le plus souvent décrites verbalement par le patient, de façon assez approximative, également.

De plus, cette description de la douleur par le patient est subjective, et ne correspond pas forcément aux véritables points de douleur du patient, remarque étant faite qu'il existe plusieurs types de douleur.

2. La stimulation médullaire est une technique de neurostimulation implantée pour atténuer ou supprimer des douleurs chroniques réfractaires, évoluant sur un territoire cutané limité. Cette technique consiste à implanter une électrode de stimulation électrique en regard de la partie postérieure de la moelle épinière (appelée « cordons postérieurs »), électrode reliée à un générateur électrique programmable sous-cutané, fournissant l'énergie nécessaire à la stimulation pendant plusieurs années en règle générale. Les cordons postérieurs qui constituent la cible de la stimulation médullaire ont pour fonction d'intégrer les influx sensitifs périphériques jusqu'au cerveau, où les sensations sont analysées et le message douloureux perçu. La stimulation électrique des cordons postérieurs agit sur les fibres du tact fin, collectant les influx sensitifs des territoires sous-jacents à l'emplacement de l'électrode. Il en résulte deux effets physiques :

- cette stimulation des fibres du tact fin, dites « épicritiques », court-circuite la transmission du message douloureux au niveau de la moelle épinière, et donc sur le territoire douloureux correspondant. Ce « court-circuit » est rendu possible par le fait que cette stimulation s'adresse à des fibres à transmission rapide, de gros calibre, fortement myélinisées (fibres de type A beta), lesquelles conduisent plus rapidement que les fibres véhiculant les influx douloureux, de type nociceptifs (fibres A delta et C). En activant ce « filtre » médullaire, la stimulation médullaire atténue les sensations douloureuses sur les territoires stimulés. A noter que les mécanismes d'action de la stimulation médullaire sont complexes, et ne se limitent pas à l'activation de ce filtre médullaire.

- cette stimulation des fibres du tact épicritique génère de manière collatérale et systématique une sensation agréable de « paresthésies » (s'apparentant à des fourmis bienveillantes) perçues par le patient sur le territoire stimulé (idéalement douloureux), et qu'il assimile à la stimulation médullaire. Dès lors qu'on parle de stimulation médullaire « conventionnelle », c'est à dire utilisant des fréquences de stimulation comprises entre 10 et 1500 Hz, il ne saurait y avoir de stimulation électrique efficace des cordons postérieurs de la moelle sans paresthésies perçues au niveau périphérique et il ne saurait y avoir d'efficacité antalgique clinique de cette stimulation sur un territoire douloureux donné sans paresthésies générées sur ce même territoire. On admet ainsi communément qu'il faut pouvoir « couvrir » le territoire douloureux cible grâce à la technique de neurostimulation utilisée, pour prétendre le soulager ultérieurement.

Or, indépendamment des difficultés pour assurer une évaluation comparative et fiable de la douleur tout au long du parcours de soins d'un patient douloureux chronique, il n'existe pas aujourd'hui de dispositif ou de procédé fiable, renseignant sur l'étendue de la couverture paresthésiante, générée par une technique de neurostimulation médullaire implantée chez un patient, utilisant des variables quantitatives et reproductibles, et permettant éventuellement une évaluation comparative de:

- l'efficacité de couverture paresthésiante d'une technique de neurostimulation,
- la sélectivité d'une couverture paresthésiante d'une technique de neurostimulation,
- la « transduction » entre une efficacité quantifiée et mesurable d'une couverture électrique liée à l'utilisation d'un dispositif antalgique et son efficacité antalgique clinique.

Le brevet US 8,046,241 (Dodson) a proposé un outil informatique pour évaluer la douleur ("Computer Pain Assessment Tool") sous la forme d'une quantité dite "Objective Pain Value". Le caractère objectif de cette quantité est discutable. De plus, ce document utilise un dessin de la douleur sur une silhouette d'humain, qui n'est pas rapportée aux mensurations du patient dans l'espace réel. Autrement dit, la métrique n'est pas satisfaisante.

Le brevet US 7,374,536 (Taylor) a proposé un procédé informatique pour l'analyse d'images de douleur. Ce document utilise un dessin de la douleur sur une silhouette d'humain sur papier, dessin qui est ensuite scanné. Là aussi, la métrique n'est pas satisfaisante.

Le document US 2009/0005649 (Baird et al.) divulgue un système et un procédé pour cartographier la profondeur d'une douleur. Pour cela, il est utilisé une silhouette d'humain tridimensionnelle sur laquelle l'utilisateur peut indiquer la localisation de la douleur. Une coupe transversale de la silhouette permet ensuite au patient d'indiquer la profondeur de la douleur. Mais la silhouette et la coupe ne sont pas rapportées aux mensurations réelles du patient. La métrique n'est donc pas satisfaisante.

Le document US 2001/0007950 (North et al.) divulgue un système et un procédé de neurostimulation interactive utilisant la coopération du patient. Le système ou procédé utilise plusieurs éléments de neurostimulation implantés sur un patient, lequel dessine, sur une silhouette standard, ses sensations au vu des stimulations, comparativement à l'étendue de sa douleur. Mais la silhouette n'est pas rapportée aux mensurations réelles du patient. Encore une fois, la métrique n'est pas satisfaisante.

Le document WO 2004/041080 divulgue un outil d'aide à l'évaluation/surveillance de la douleur selon le préambule de la revendication 1.

La présente invention vient améliorer la situation, en particulier au niveau de la précision de dessin, et de son rapport avec une métrique réelle, et sur de nombreux autres aspects dont il sera question plus loin. Il s'agit notamment d'attribuer des métriques aux douleurs pour leur analyse descriptive et aux paresthésies générées par une technique de neurostimulation implantée en rapport avec lesdites douleurs, pour leur analyse comparative ; on pourra ensuite procéder à des analyses statistiques sur des groupes de patients.

Selon un premier aspect, l'invention propose un outil d'aide à l'évaluation/surveillance de la douleur, comprenant :

- un outil graphique, tel qu'une tablette, équipé d'un écran et capable de dialoguer avec un serveur,
- une application locale installée dans l'outil graphique, pour afficher à l'écran une représentation graphique du corps d'un patient, tout en permettant à un usager tel que le patient de délimiter une zone sur la représentation graphique affichée à l'écran,
- une application pilote coopérant avec l'application locale pour enregistrer chaque zone délimitée, en association avec des données de surface correspondantes.

L'application pilote est agencée pour piloter une fonction d'auto-calibration patient, capable de déterminer les écarts entre une pluralité de points-cibles désignés par contact sur le corps physique du patient, et des points correspondants indiqués par pointage par le patient sur la représentation graphique affichée à l'écran. Et la délimitation sur l'écran d'une zone sur la représentation graphique par le patient est modifiée en fonction de ces écarts d'auto-calibration patient. Ceci permet de prendre en compte le fait qu'un patient perçoit de façon imprécise les points de son corps, notamment lorsqu'il a mal.

Les points-cibles peuvent être désignés successivement par contact sur le corps physique du patient, tandis que le patient indique à chaque fois sur l'écran de la tablette graphique le point de la représentation graphique de son corps où il ressent le contact. Les points-cibles peuvent être choisis parmi une liste prédéfinie de repères morphologiques. Enfin, la fonction d'auto-calibration patient est répétée plusieurs fois pour un même patient. Chacune de ces caractéristiques améliore les choses en pratique.

Selon un autre aspect, l'invention propose un outil qui comprend un écran tactile. L'application pilote est également agencée pour piloter une fonction de calibration patient/écran, capable de déterminer les écarts entre des points à viser sur l'écran et les zones de l'écran tactile que le patient touche effectivement. Ces écarts peuvent être combinés avec les écarts d'auto calibration patient. Dans ce cas, les points à viser sur l'écran peuvent comprendre au moins quatre points proches de quatre coins de l'écran et un point au centre de l'écran.

Selon un autre aspect, l'application locale est agencée pour permettre une délimitation par le patient de zones de douleur sur la représentation graphique du corps ou silhouette et d'afficher à l'écran lesdites zones de douleur, et le calcul de données de surface correspondantes. Ces données de douleur pourront être rapprochées de données de traitement.

De préférence, la délimitation graphique de zones de douleur est différenciée d'après l'intensité de la douleur d'une part, et le type de douleur d'une autre part. Pour les types de douleurs, on peut notamment différencier parmi certains au moins des types du groupe : Douleur Nociceptive, Douleur "Trigger" ("Gâchette"), Douleur Neuropathique, Douleur Mécanique.

Selon un autre aspect, l'application locale est agencée pour permettre également une délimitation graphique d'au moins une zone de ressenti de traitement, notamment de paresthésies.

Très avantageusement, l'application locale est agencée pour effectuer des calculs comparatifs entre la ou les surfaces de paresthésies et certaines au moins des surfaces de zones de douleur.

Chaque délimitation graphique peut être réalisée par remplissage de zone avec des attributs graphiques sélectifs, en particulier de couleur, certaines au moins des zones étant partiellement transparentes.

Selon un autre aspect, l'application locale est agencée pour permettre d'associer une métrique réelle auxdites délimitations graphiques. Cela permet d'obtenir des surfaces réelles.

La métrique réelle peut être obtenue en appliquant une échelle réelle à la silhouette du patient, à partir d'une grandeur réelle mesurée, telle que la taille du patient. Elle peut aussi être obtenue par traitement d'images du patient, issues de l'imagerie médicale.

Selon un autre aspect, les données de délimitation graphique et de surfaces sont rangées dans un historique daté rapporté à une fiche patient.

Dans un mode de réalisation intéressant, l'outil graphique est monté en réseau avec au moins un autre ordinateur, d'une manière permettant la mobilité.

## BREVE DESCRIPTION DES FIGURES

D'autres caractéristiques et avantages de l'invention apparaîtront à l'examen de la description détaillée ci-après, et des dessins annexés. Certaines figures sont présentées à la fois sous une forme en couleur, et sous une forme en noir et blanc, la version couleur représentant beaucoup mieux les avantages de l'invention.

Sur les dessins :

- La figure 1 illustre l'affichage d'une silhouette (représentation corporelle) sur un écran, selon un mode de réalisation de la cartographie proposée ;

- La figure 2 illustre le dessin d'une zone douloureuse sur la silhouette de la Fig. 1 et, dans le bandeau de droite, des éléments de calcul surfacique de la taille de la surface douloureuse ;

- La figure 3 illustre l'affichage de dermatomes (bandes de surface cutanée innervées par un même nerf périphérique) représentés sur la silhouette, et le calcul de ratios de surface cutanée douloureuse correspondant à l'implication de chacun de ces dermatomes ;

- La figure 4 illustre des étapes d'un mode de réalisation du procédé de cartographie proposé ;

- La figure 5 illustre des étapes d'un procédé d'évaluation de l'efficacité d'une stimulation médullaire, tel que proposé ;

- La figure 6 représente schématiquement un système de cartographie et un système d'évaluation de l'efficacité d'une stimulation médullaire, tels que proposés ;

- La figure 7 est un schéma de principe des équipements proposés, avec leur contexte médical ;

- La figure 8 est une copie d'écran convertie en noir et blanc illustrant un menu pour la saisie du patient ;

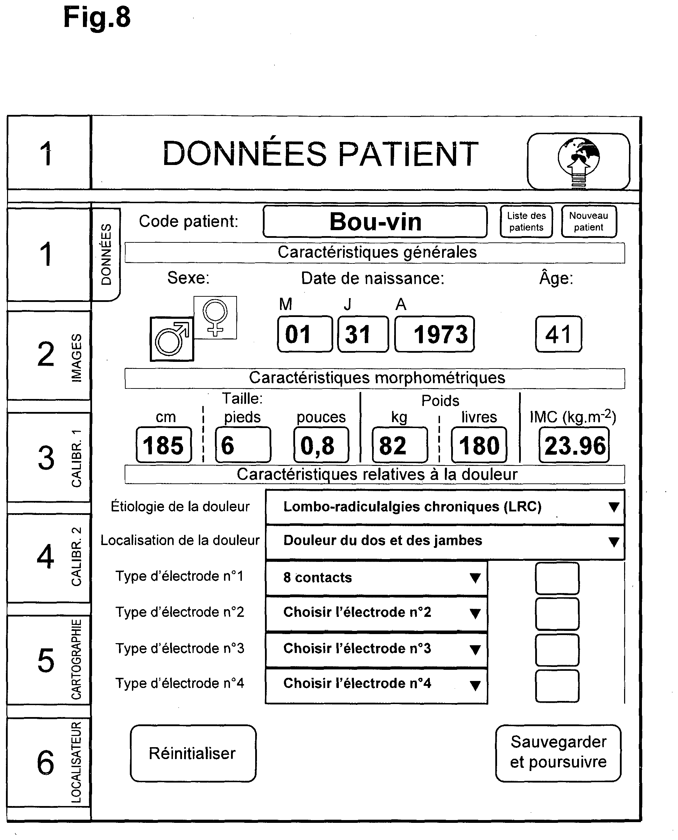

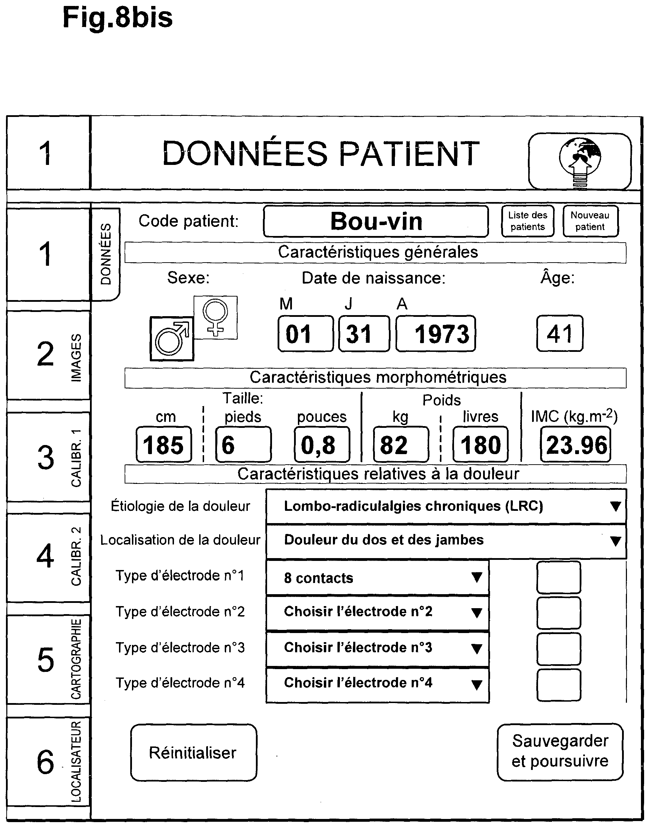

- La figure 8bis est l'original en couleur de la figure 8 ;

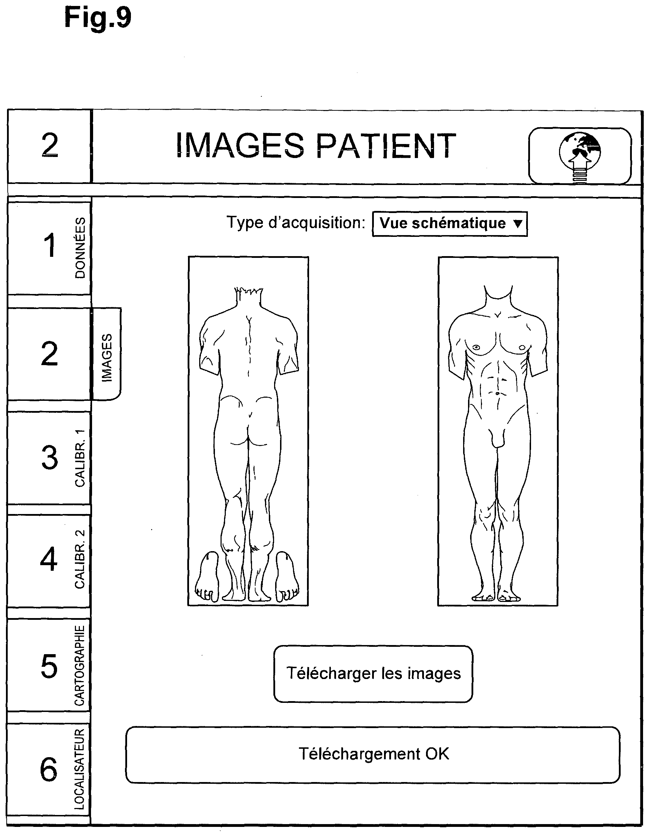

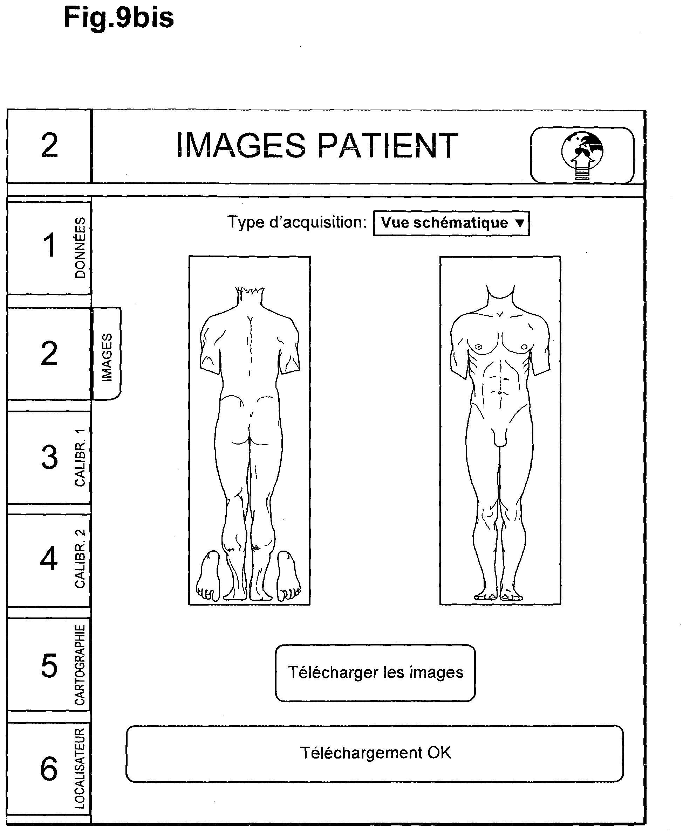

- La figure 9 est une copie d'écran illustrant un menu pour définir l'image utilisée pour représenter le patient ;

- La figure 9bis est l'original en couleur de la figure 9 ;

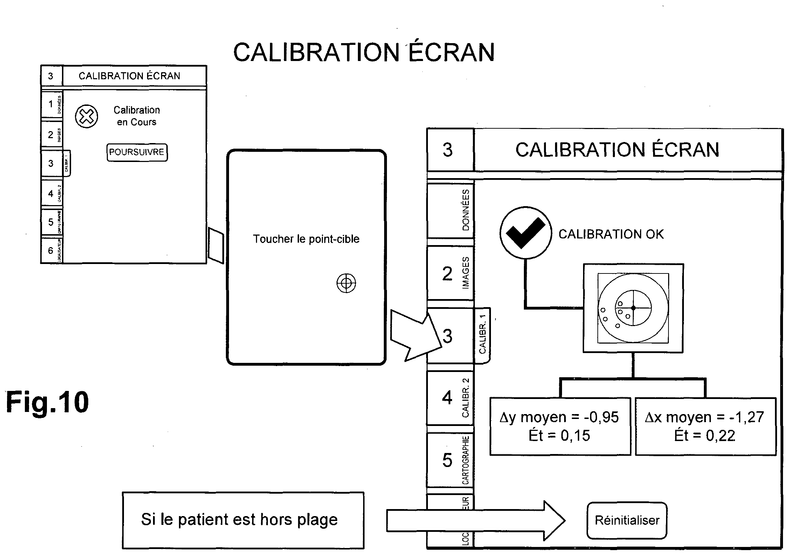

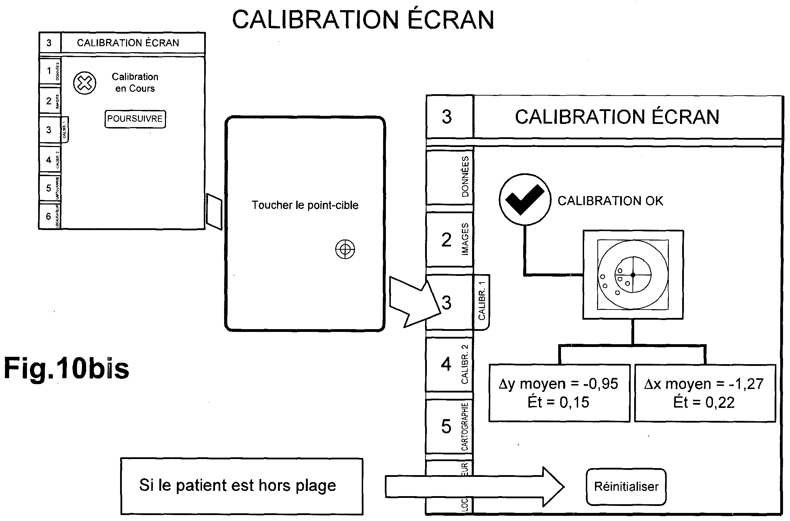

- La figure 10 est une copie d'écran illustrant le résultat de l'opération dite "Calibration 1" ci-après ;

- La figure 10bis est l'original en couleur de la figure 10 ;

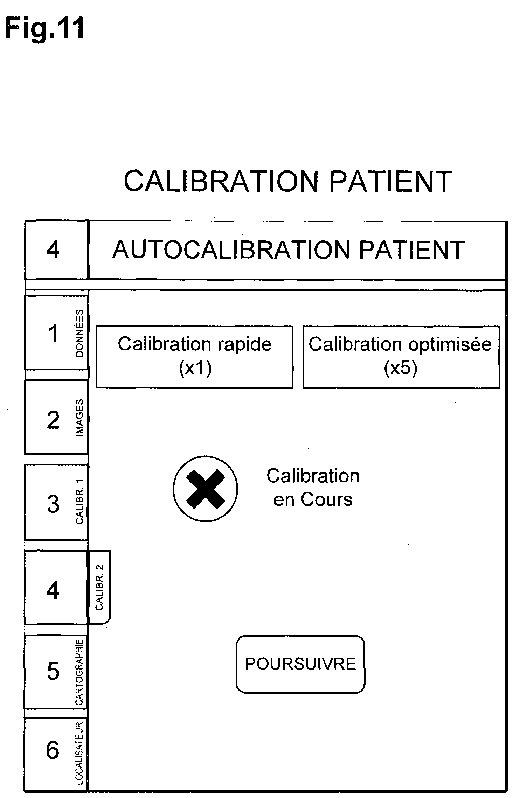

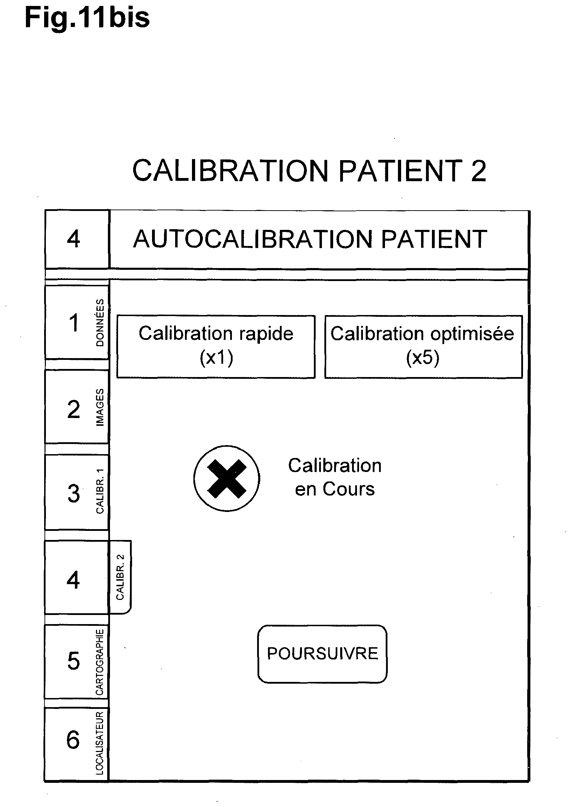

- La figure 11 est une copie d'écran illustrant le lancement de l'opération dite "Calibration 2" ci-après ;

- La figure 11bis est l'original en couleur de la figure 11 ;

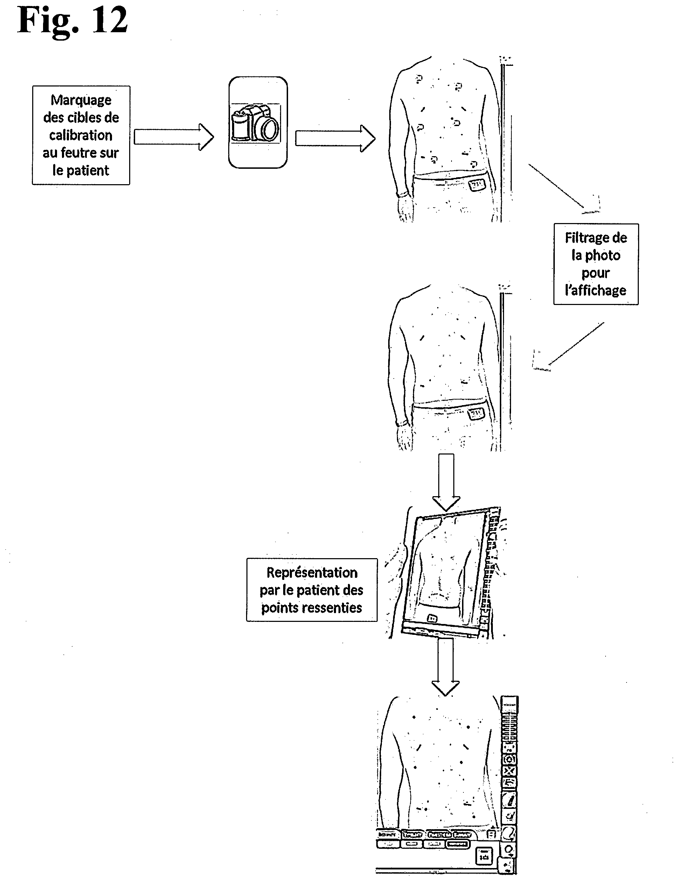

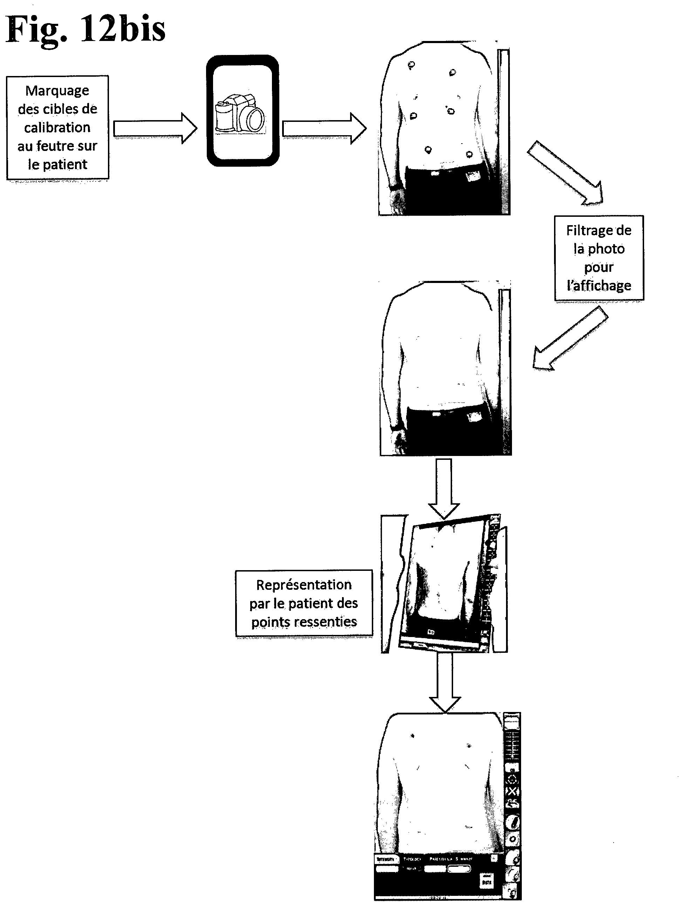

- La figure 12 illustre un mode de réalisation de base de l'opération dite "Calibration 2" ci-après ;

- La figure 12bis est l'original en couleur de la figure 12 ;

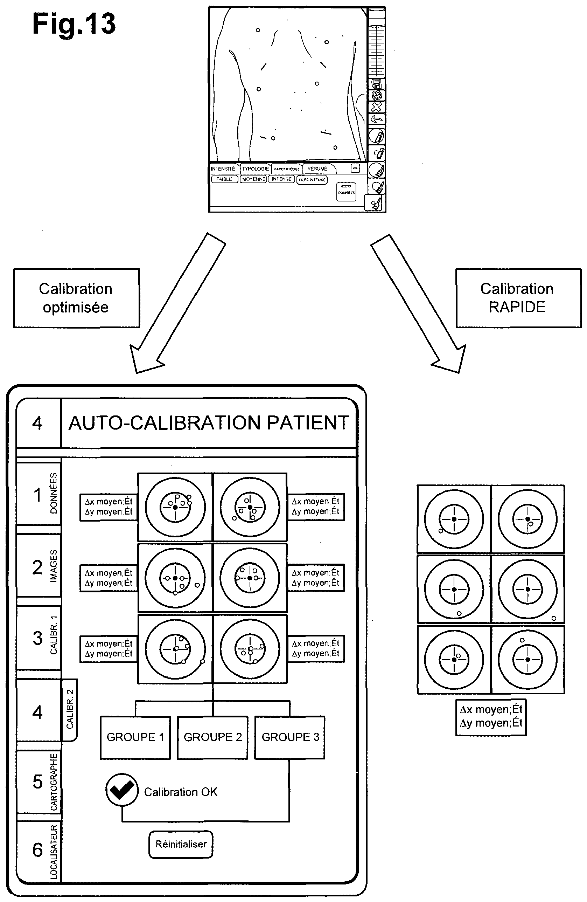

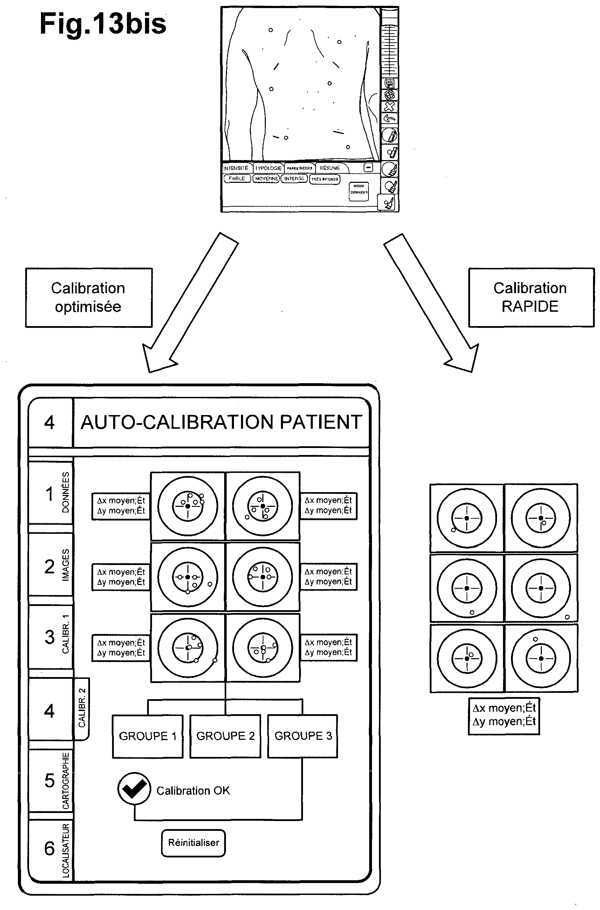

- La figure 13 illustre deux modes d'exploitation de l'opération de la figure 12 ;

- La figure 13bis est l'original en couleur de la figure 13 ;

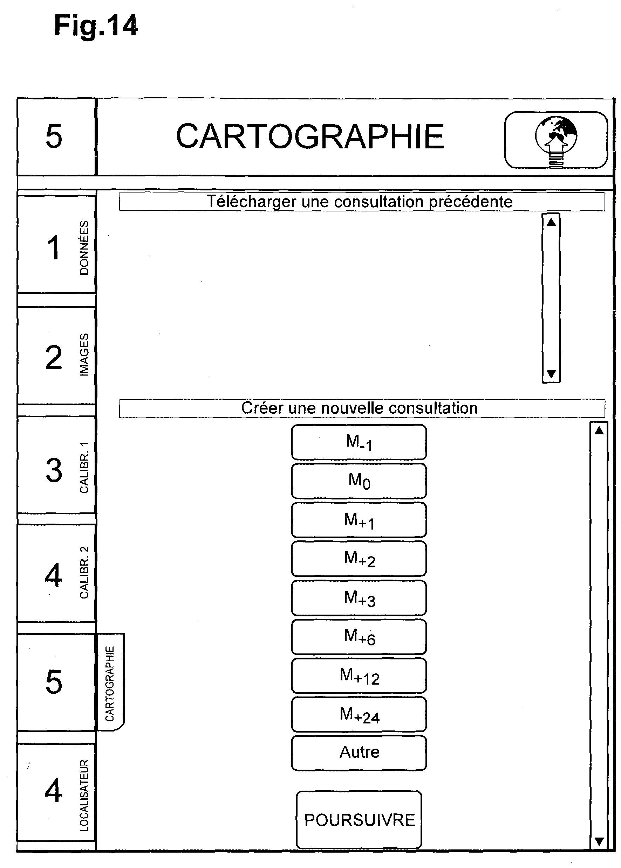

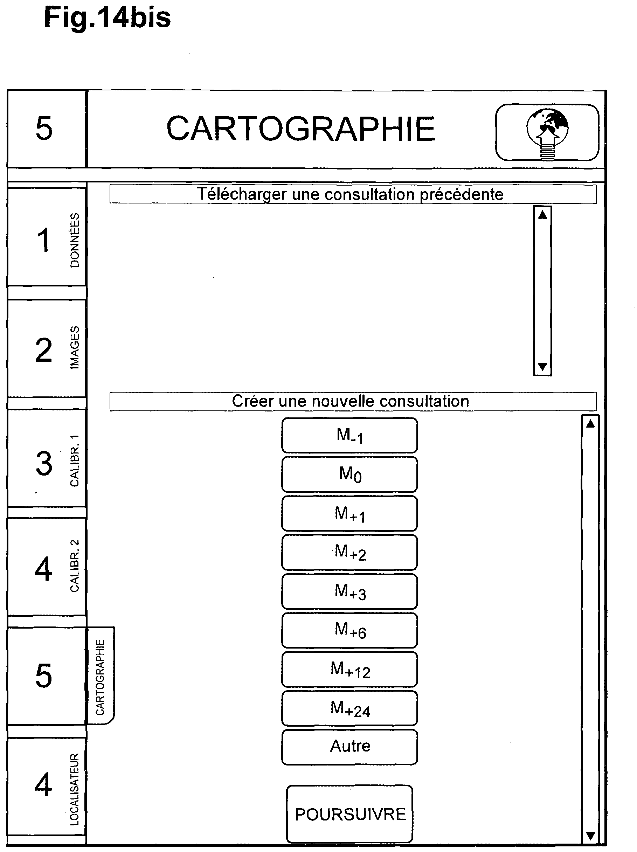

- La figure 14 est une copie d'un écran d'accueil qui gère un historique ;

- La figure 14bis est l'original en couleur de la figure 14 ;

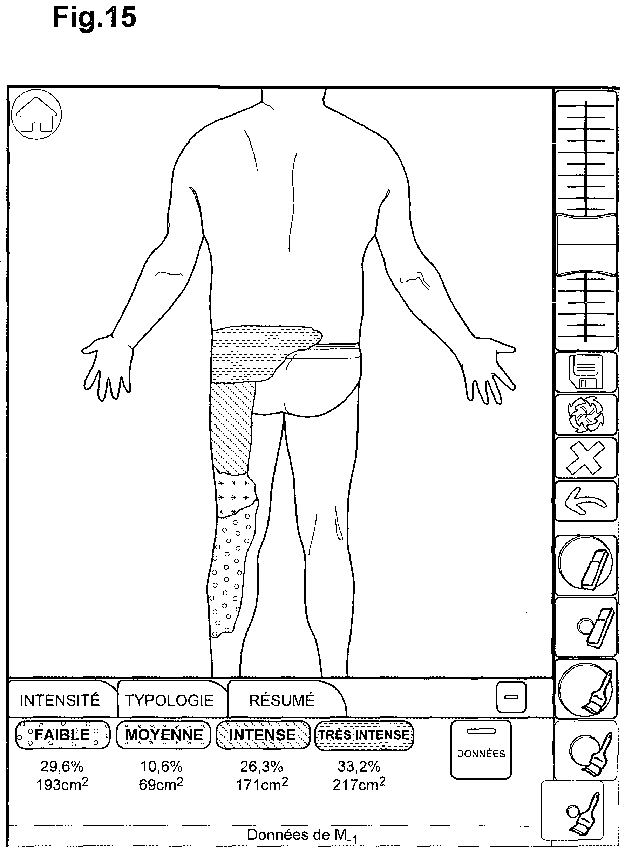

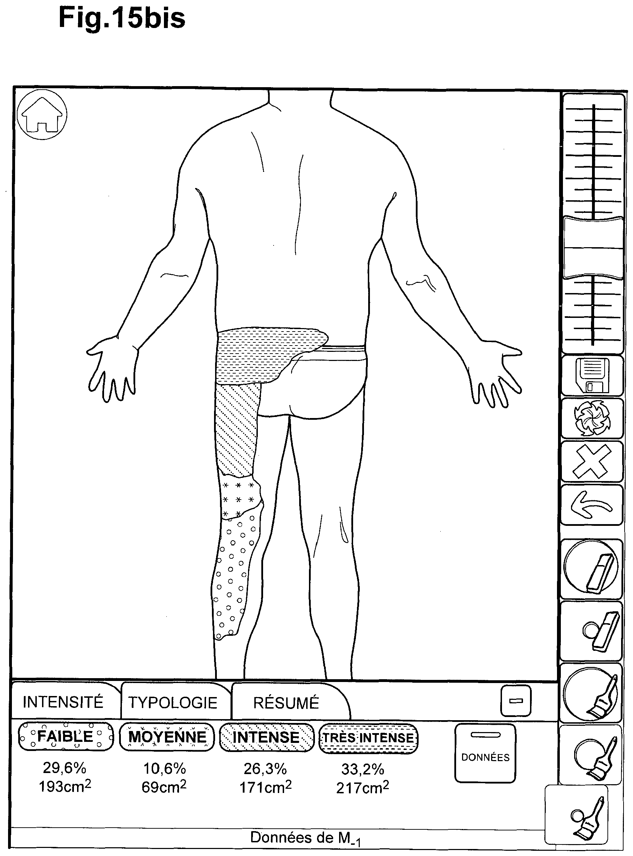

- La figure 15 est une copie d'écran illustrant un exemple de cartographie de l'intensité de douleur ;

- La figure 15bis est l'original en couleur de la figure 15 ;

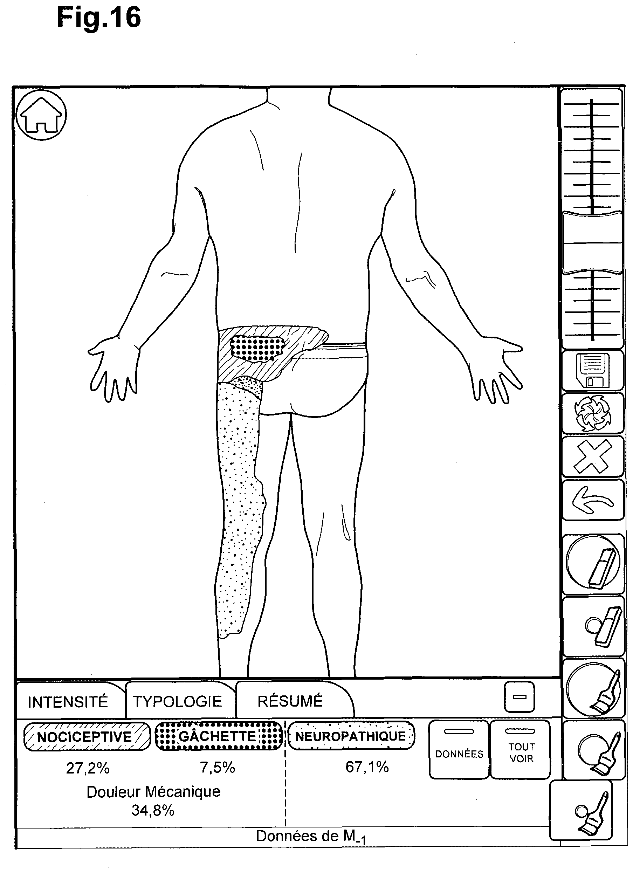

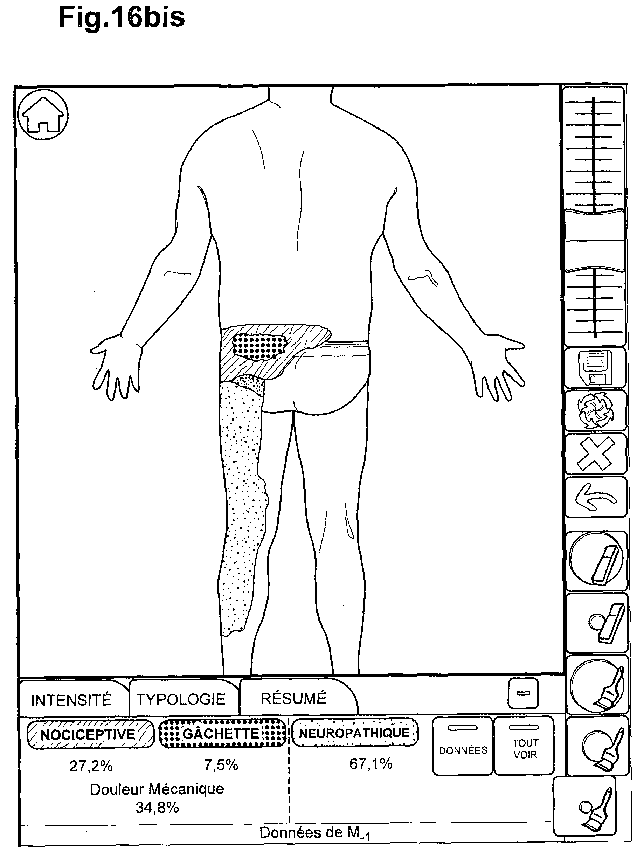

- La figure 16 est une copie d'écran illustrant un exemple de cartographie de type de douleurs ;

- La figure 16bis est l'original en couleur de la figure 16 ;

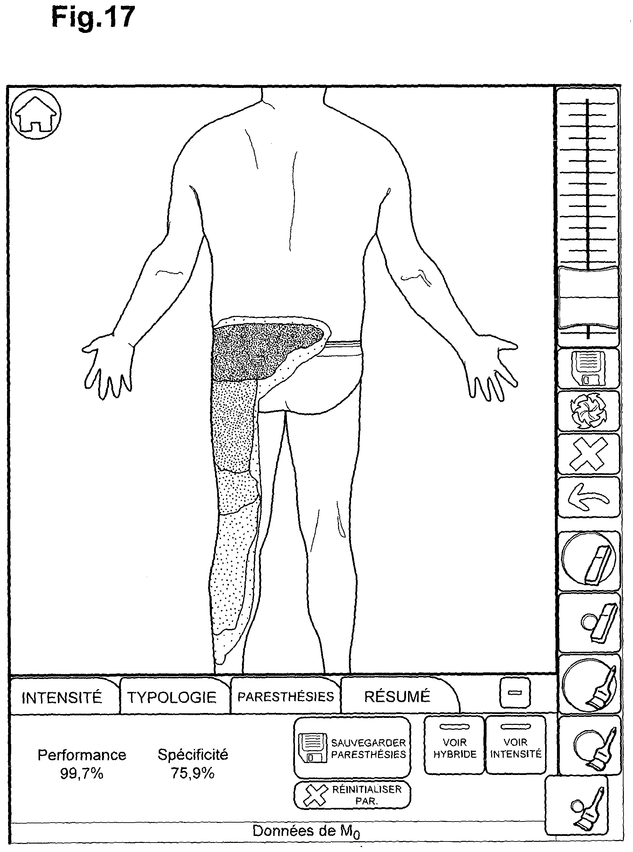

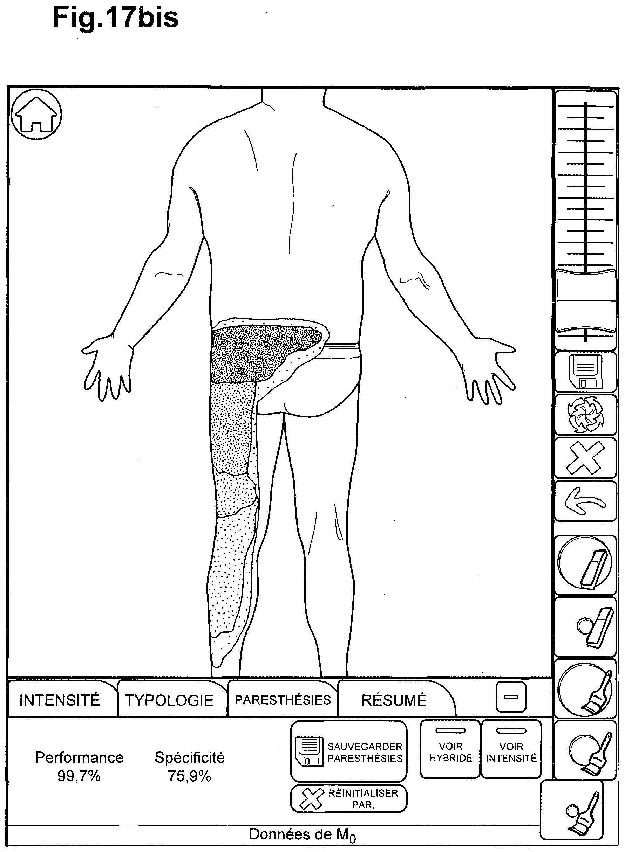

- La figure 17 est une copie d'écran illustrant un exemple de cartographie de traitement de la douleur par une stimulation implantée, générant une « couverture de la zone douloureuse », dessinée par le patient et représentant la sensation créée par le dispositif de neurostimulation ou « paresthésies » ;

- La figure 17bis est l'original en couleur de la figure 17 ;

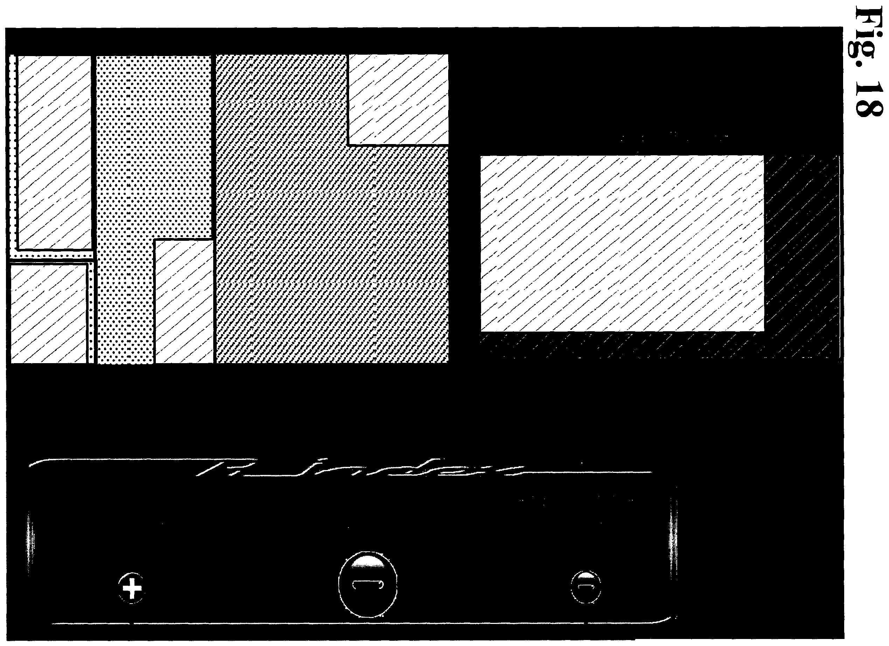

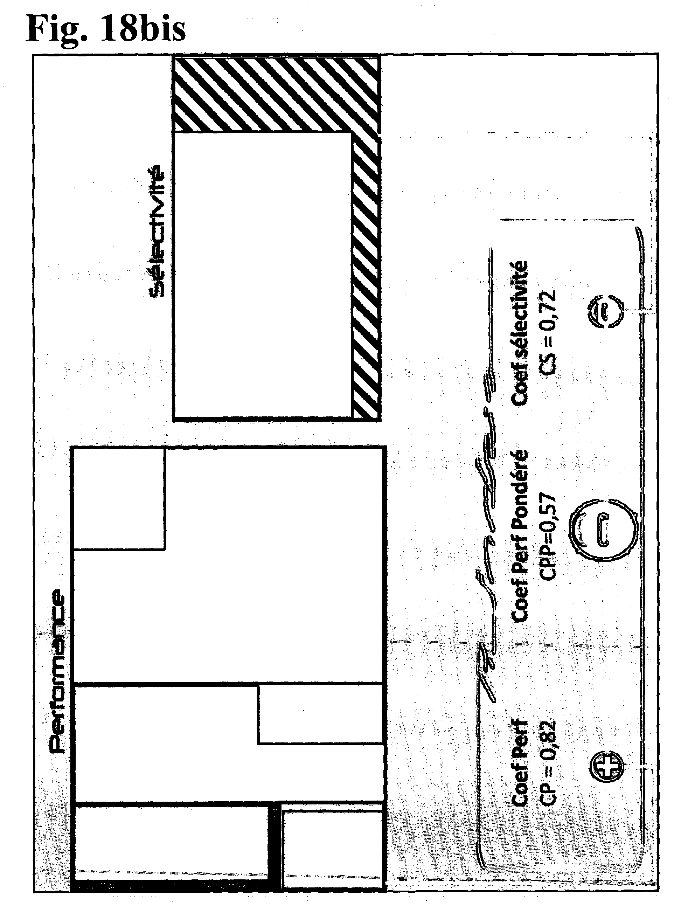

- La figure 18 est une copie d'écran illustrant un outil particulièrement avantageux, dit "diagramme de Manoé";

- La figure 18 bis est l'original en couleur de la figure 18 ;

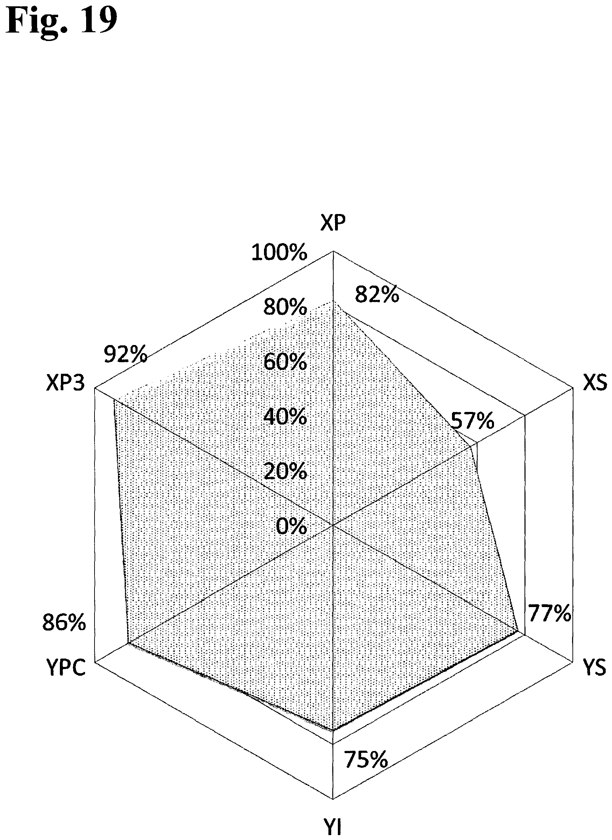

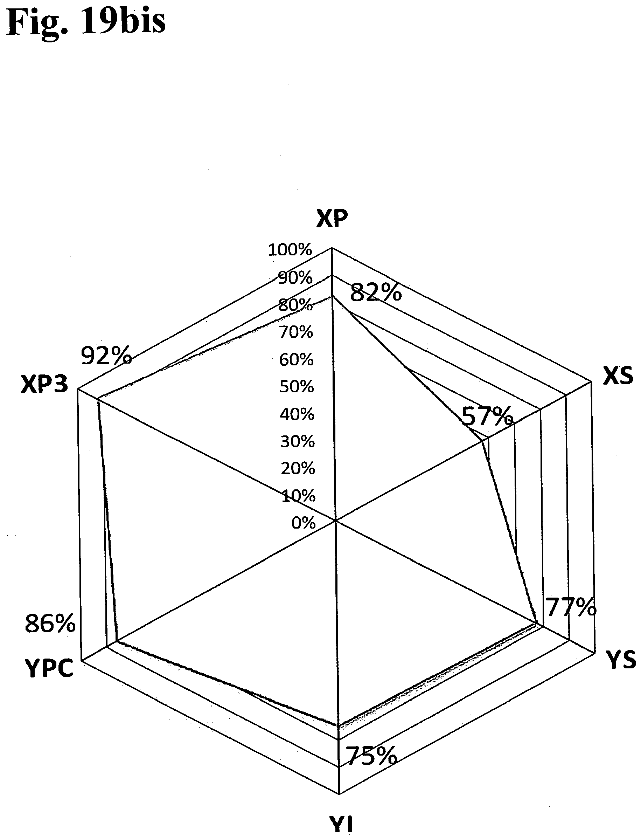

- La figure 19 est une copie d'écran montrant un diagramme hexagonal multiaxial pour une électrode et un patient;

- La figure 19 bis est l'original en couleur de la figure 19 ;

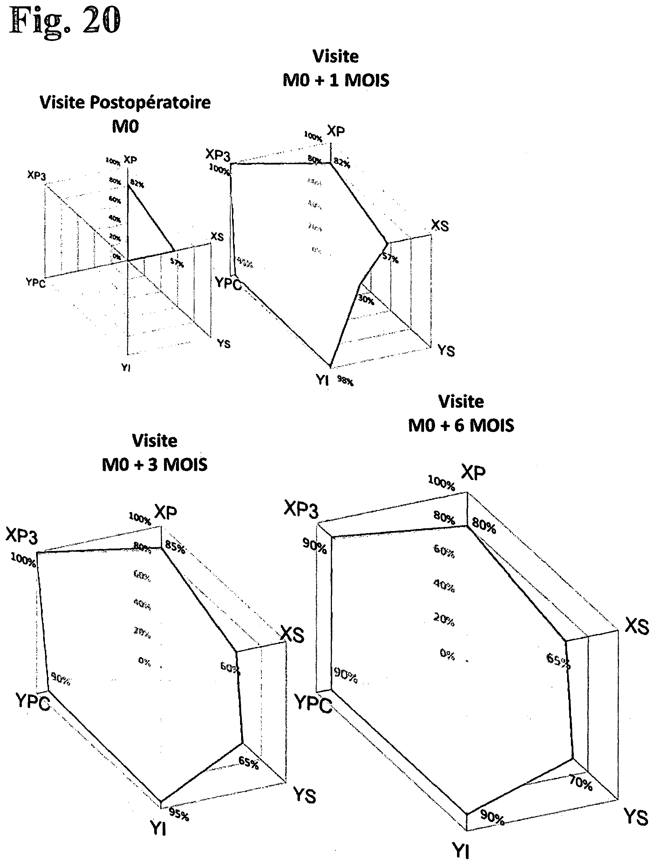

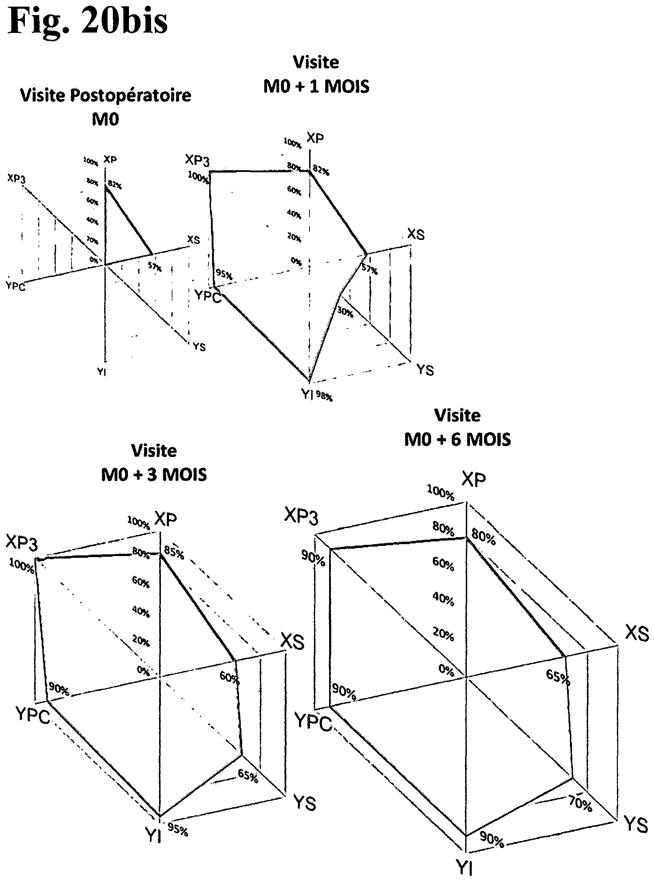

- La figure 20 est une copie d'écran montrant en perspective une suite temporelle de diagrammes semblables à celui de la figure 19, pour une électrode et un patient;

- La figure 20 bis est l'original en couleur de la figure 20 ;

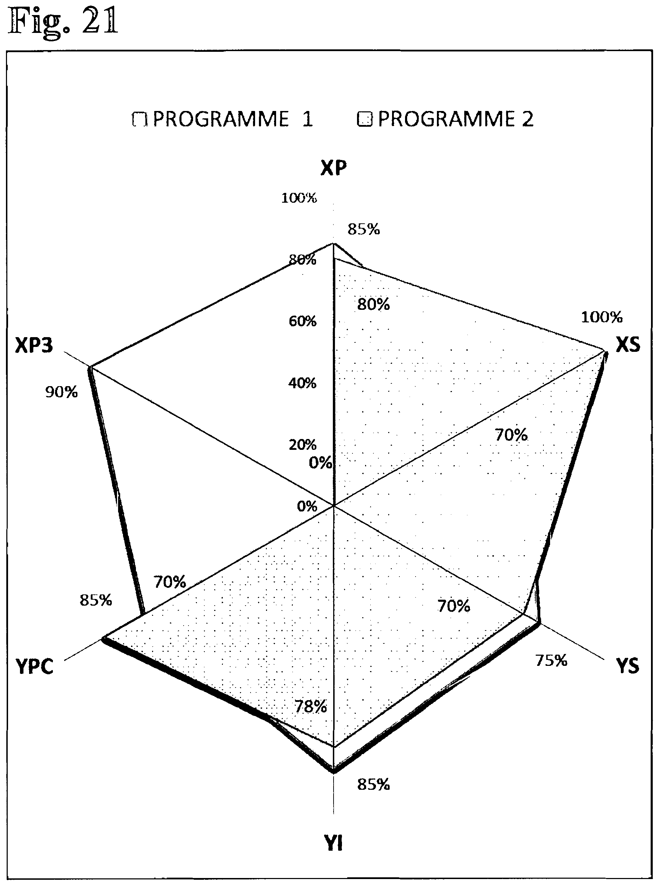

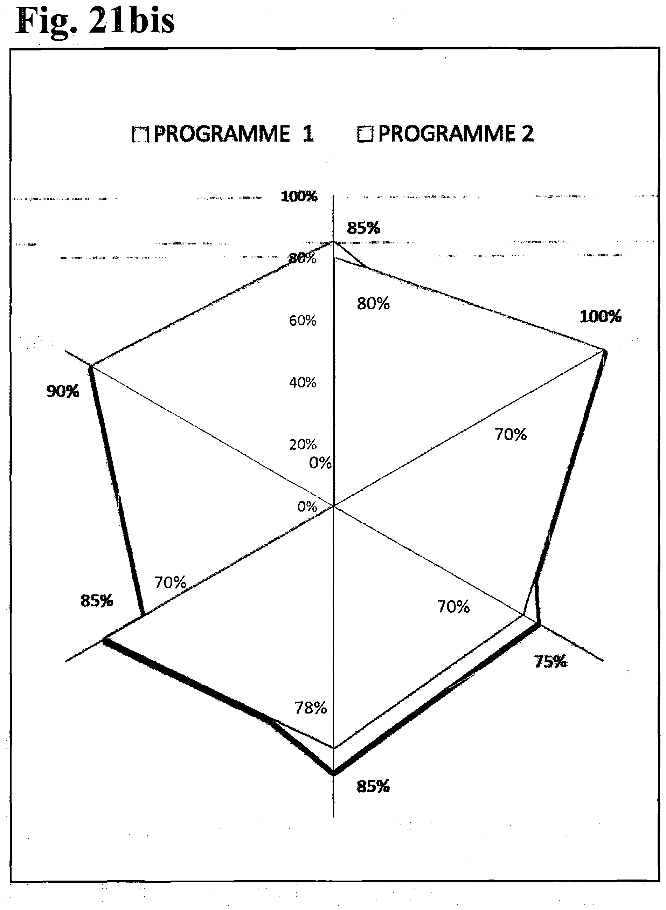

- La figure 21 est une copie d'écran montrant un diagramme hexagonal multiaxial sur lequel sont superposés deux contours relatifs à deux programmes différents d'excitation de l'électrode pour un patient ;

- La figure 21 bis est l'original en couleur de la figure 21 ;

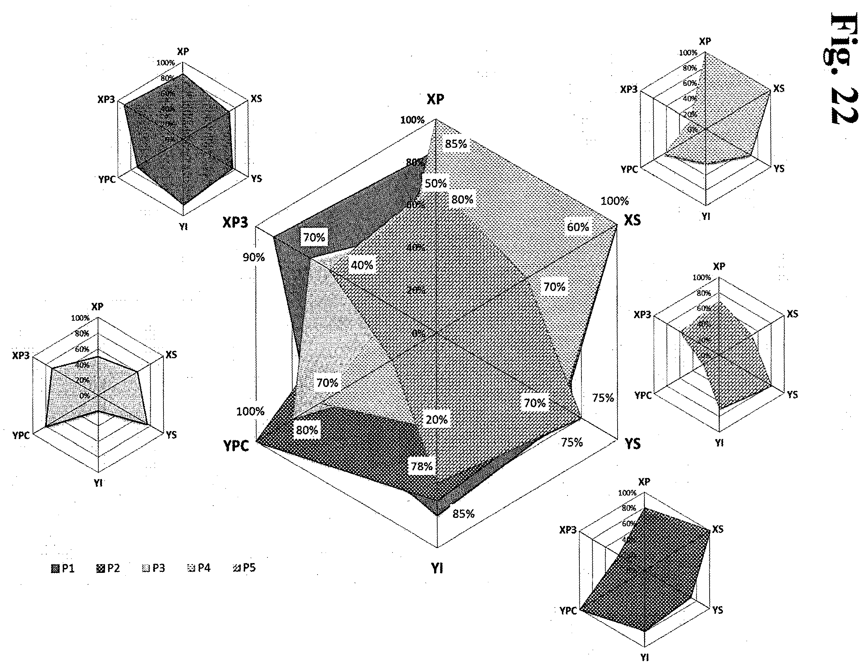

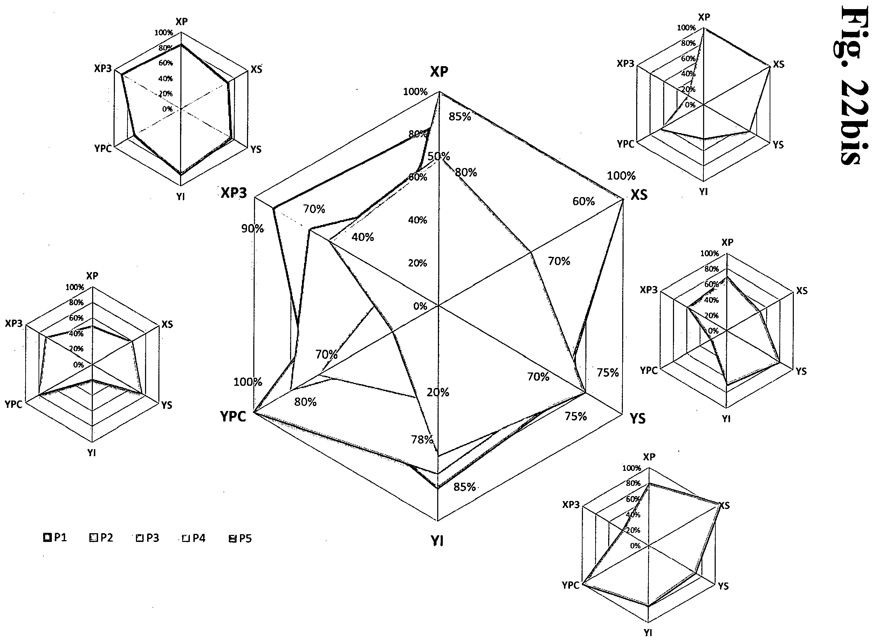

- La figure 22 est une copie d'écran montrant en périphérie cinq diagrammes hexagonaux multiaxiaux, correspondant à cinq programmes différents d'excitation de l'électrode pour un patient, et au centre, dans un diagramme hexagonal multiaxial plus grand, la superposition des cinq contours des diagrammes périphériques ; et

- La figure 22 bis est l'original en couleur de la figure 22.

Les dessins et la description ci-après contiennent, pour l'essentiel, des éléments à caractère certain. Les dessins font partie intégrante de la description et pourront donc non seulement servir à mieux faire comprendre la présente invention, mais aussi contribuer à sa définition, le cas échéant.

La description détaillée qui va suivre comprend d'abord une description résumée de l'invention, tirée en substance de la demande de brevet français No 1358850 déposée le 13 septembre 2013, et intitulée "PROCEDE ET SYSTEME DE CARTOGRAPHIE, PROCEDE ET SYSTEME D'EVALUATION DE L'EFFICACITE D'UNE STIMULATION MEDULLAIRE"

La description détaillée comprend ensuite des modes de réalisation approfondis, actuellement préférés, de plusieurs aspects de l'invention.

**Description résumée de l'invention**

L'invention propose un procédé de cartographie de zones douloureuses, un procédé d'évaluation de l'efficacité d'une stimulation médullaire, un système de cartographie, et un système d'évaluation de l'efficacité d'une stimulation médullaire.

Le procédé de cartographie est destiné à :

- indiquer la localisation de douleurs ressenties par un patient,
- indiquer le type de ces douleurs (neuropathiques ou mécaniques) ressenties, et
- fournir des caractéristiques desdites douleurs. Ces caractéristiques sont notamment la surface cutanée concernée par la douleur, et le pourcentage de surface cutanée douloureuse comprise dans chaque dermatome ou chaque région lombaire.

Ces résultats permettent à un médecin de choisir une ou plusieurs électrodes à implanter au niveau de la ou les zones douloureuses, afin de mettre en oeuvre la technique de stimulation, par exemple médullaire, visant à soulager les douleurs du patient. En effet, en fonction des caractéristiques indiquées précédemment, certaines électrodes ou dispositifs seront plus ou moins adaptés.

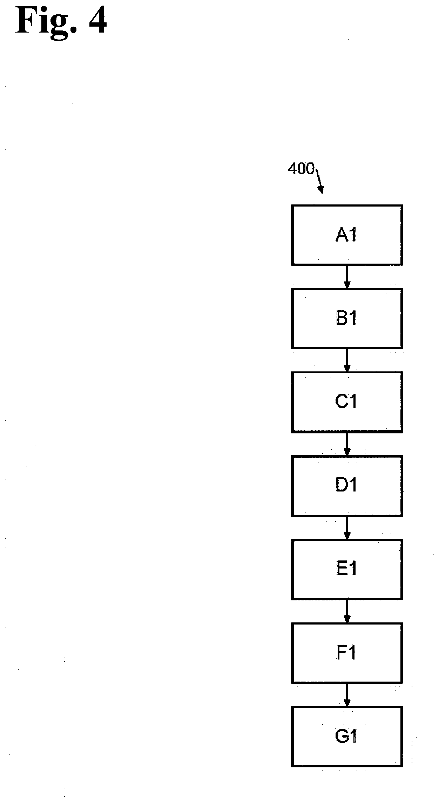

Plus précisément, en référence à la figure 4, le procédé de cartographie 400 comporte les étapes suivantes :

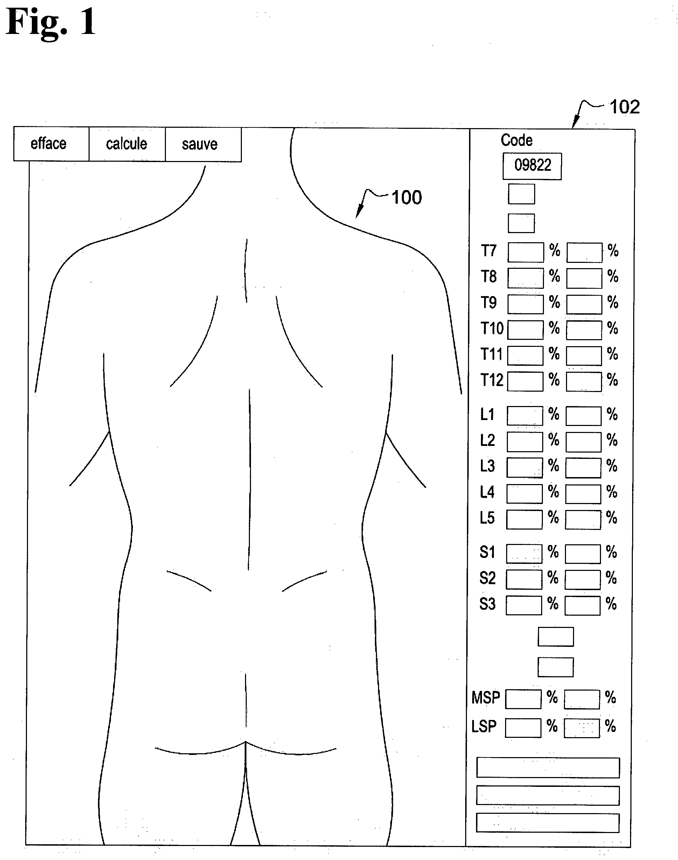

- A1 : en référence à la figure 1, afficher une première silhouette 100 représentant la face postérieure d'un corps, sur un premier écran 102. Le premier écran 102 est par exemple un écran tactile, avantageusement un écran de tablette tactile. En effet, un écran tactile permet une utilisation ergonomique, et une tablette tactile est de taille suffisamment grande pour permettre un affichage agréable, et suffisamment petite pour limiter l'encombrement. Dans le mode de réalisation décrit, la silhouette 100 est un modèle standard, non adaptée à la morphologie du patient. Cependant, un mode de réalisation dans lequel la silhouette 100 est de forme adaptée à la morphologie du patient n'est pas exclu.

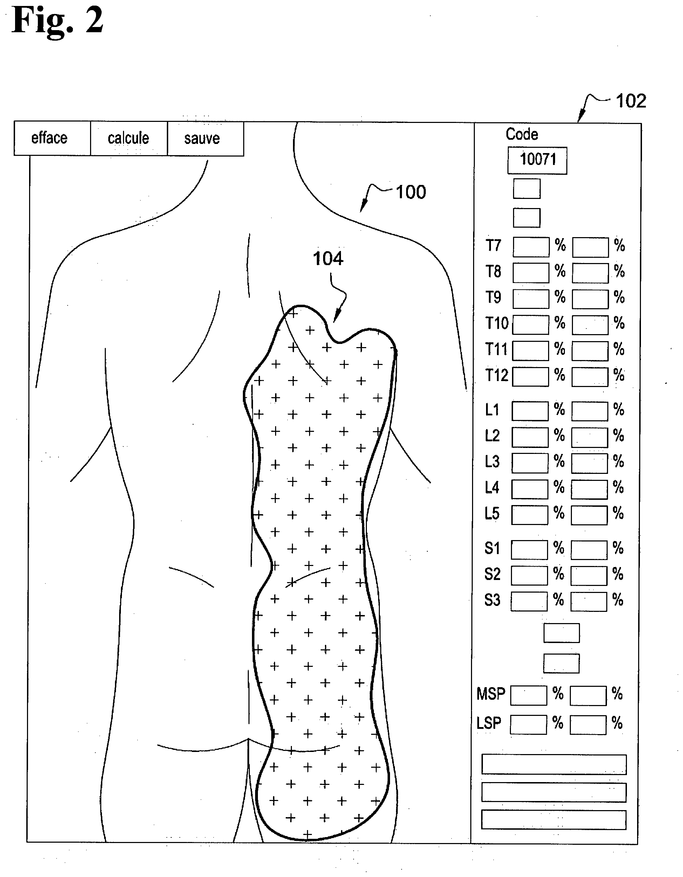

- B1 : en référence à la figure 2, dessiner au moins une zone douloureuse 104 sur ladite première silhouette affichée 100, indiquant la localisation d'une douleur ressentie par le patient. Dans un mode de réalisation non limitatif dans lequel l'écran 102 est tactile, le patient délimite la zone douloureuse 104 en l'entourant avec son doigt.

Plus particulièrement, le patient peut :

- Dessiner d'une première manière une douleur neuropathique, c'est-à-dire une surface douloureuse comportant des caractéristiques neuropathiques ;
- Dessiner d'une deuxième manière une douleur mécanique, c'est-à-dire une surface douloureuse comportant des caractéristiques mécaniques.

Dans un mode de réalisation, le patient dispose d'une palette de deux couleurs (au moins), chaque couleur représentant un type de douleur. Dans un autre mode de réalisation, la douleur est représentée d'une seule et même couleur mais des hachures permettent de différencier le type de douleur. Par type de douleur, on entend notamment une douleur mécanique ou neuropathique. Des critères déterminés regroupés dans un questionnaire permettent de qualifier une douleur comme neuropathique. Un exemple de questionnaire connu est le questionnaire dit "DN4" défini dans la publication : Bouhassira et al. Comparison of pain syndromes associated with nervous or somatic lesions and development of a new neuropathic pain diagnostic questionnaire (DN4). Pain 2005;114:29-36. Le patient peut ainsi indiquer si une zone douloureuse correspond à une douleur neuropathique ou à une douleur mécanique.

En effet, certains scientifiques considèrent que la stimulation par exemple médullaire n'est pas efficace sur les douleurs mécaniques. Ces sous-étapes peuvent permettre de confirmer ou d'infirmer cette considération.

- C1 : déterminer un premier nombre de pixels du premier écran 102, correspondant à la zone douloureuse 104. On note que les nombres de pixels correspondant aux douleurs neuropathiques et mécaniques, ou à différentes zones douloureuses, s'ajoutent sauf dans le cas d'une intersection : dans ce cas, la surface commune n'est comptabilisée qu'une seule fois, au moins pour certains des calculs, comme la performance et la spécificité.

- D1 : mesurer une distance repère sur le patient. Dans le mode de réalisation décrit, la distance repère correspond à la distance entre les deux crêtes iliaques du patient. En effet, cette distance présente l'avantage d'être sensiblement invariante selon la corpulence du patient. On note que d'autres repères morphologiques pourraient être utilisés, par exemple la distance entre les omoplates ou la distance entre les mastoïdes, mais la distance entre les crêtes iliaques est préférée. Il est entendu que l'étape de mesure de la distance repère peut être effectuée avant les étapes précédemment décrites, ou entre deux de ces étapes.

- E1 : convertir le premier nombre de pixels en une surface cutanée douloureuse, la distance repère intervenant comme paramètre de la conversion. La surface cutanée douloureuse est alors affichée sur le premier écran 102, ce qui permet au médecin de disposer de l'information. La surface cutanée douloureuse est par exemple exprimée en centimètres carrés.

Pour réaliser cette étape de conversion, deux coefficients sont calculés :

- un coefficient vertical égal à la taille réelle du patient divisée par le nombre de pixels représentant la taille de la silhouette
- un coefficient horizontal égal à la distance entre les crêtes iliaques du patient divisée par le nombre de pixels représentant la distance entre les crêtes iliaques sur la silhouette.

La surface cutanée douloureuse est alors égale au produit du coefficient horizontal, du coefficient vertical et du premier nombre de pixel correspondant à la zone douloureuse. Le produit du coefficient horizontal par le coefficient vertical donne la surface d'un pixel.

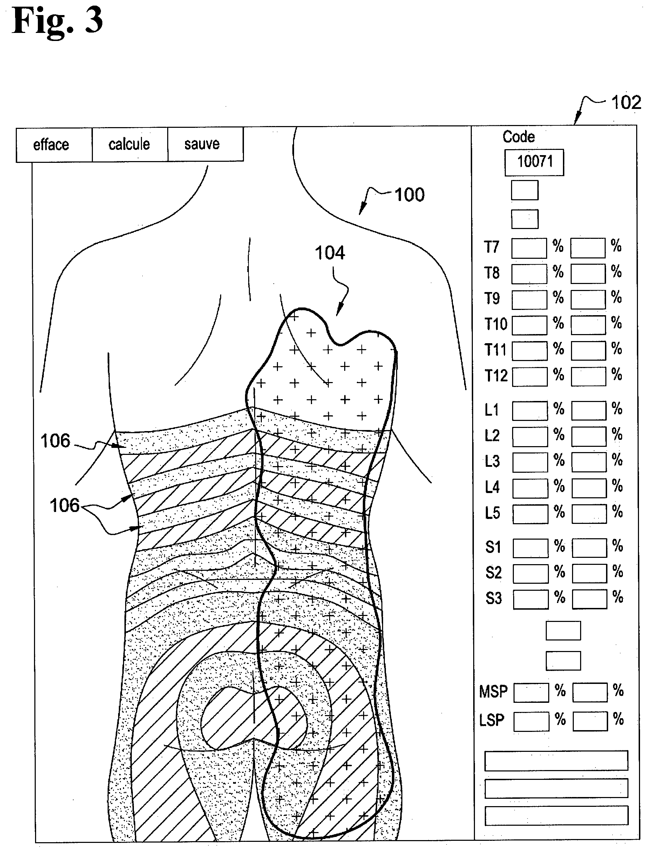

- F1 : déterminer un ratio de surface cutanée douloureuse comprise dans une région 106 de la première silhouette 100. En référence à la figure 3, dans un mode de réalisation, la région 106 considérée correspond à un dermatome. Dans un mode de réalisation, les régions 106 sont affichées sur la silhouette 100, superposées à la zone douloureuse 104 dessinée. La région 106 pourrait également être une région lombaire, ou toute autre région correspondant à une notion topographique connue.

Avantageusement, plusieurs ratios correspondant à plusieurs régions 106 d'un référentiel topographique sont calculés. Dans un mode de réalisation, ces ratios sont exprimés en pourcentage afin d'en faciliter la lecture. Le médecin peut ainsi savoir quels dermatomes sont impliqués dans la surface douloureuse, et en quelles proportions. Dans un mode de réalisation, un tableau de correspondance est affiché à côté de la première silhouette 100, montrant les ratios ou les pourcentages de surface cutanée douloureuse par région considérée. La surface cutanée douloureuse est ainsi exprimée en pourcentages par rapport à la représentation surfacique des différents dermatomes de la région dorso-lombaire en particulier.

- G1 : dans un mode de réalisation, la première silhouette 100 sur laquelle sont dessinées la ou les zones douloureuses 104, ainsi que les caractéristiques de ces douleurs, sont enregistrées sur un serveur.

La surface cutanée correspondant à la zone douloureuse dessinée par le patient est un premier paramètre de quantification de la douleur dont le procédé permet l'évaluation. Ce premier paramètre aide à choisir convenablement l'outil thérapeutique, c'est-à-dire le type d'électrode, à utiliser. En effet, si la surface cutanée douloureuse est estimée à 25 centimètres carrés, une électrode d'une capacité de couverture de 10 centimètres carrés pourra être jugée inadaptée. On note qu'avantageusement, l'écran est tactile, et le patient dessine la zone douloureuse avec ses doigts, ce qui est plus pratique que d'utiliser une souris ou un stylet.

À chaque pression tactile une délimitation de la zone douloureuse est saisie au niveau de la cartographie, plusieurs zones pouvant être dessinées avec ou sans superposition. Cette cartographie est étalonnée par rapport à la distance repère afin d'extrapoler les tracés par rapport à l'individu concerné. Le dessin est simple, rapide à réaliser et permet en plus de délimiter une surface, d'identifier une localisation et une évolution du tracé au fil du traitement de la douleur.

Dans un mode de réalisation non limitatif, le procédé de cartographie comprend l'étape suivante : déterminer un ratio de surface cutanée douloureuse comprise dans une région de la première silhouette. Ledit ratio de surface cutanée comprise dans la région est un deuxième paramètre de quantification de la douleur dont le procédé permet l'évaluation. En effet, il est intéressant de rapporter la surface cutanée douloureuse à des notions topographiques connues de l'organisme, notamment des délimitations agréées par des organismes spécialisés ou la communauté scientifique, telles que les délimitations High Back Pain / Low Back Pain, ou bien des délimitations communément admises sur le plan anatomique, telles que les dermatomes. En corrélant la localisation de la surface cutanée douloureuse avec un référentiel topographique choisi, le médecin a accès à des données lui permettant de choisir convenablement ses outils thérapeutiques, c'est-à-dire les électrodes à implanter.

Par exemple, une électrode peut être efficace dans une région donnée, et moins efficace dans une autre. On note que la quantification de la douleur par surface topographique est importante pour déterminer la prédominance et l'intensité de certains types de douleurs et ainsi expliquer la douleur. Cette étape permet donc en outre de préciser les territoires nerveux périphériques qui sont plus ou moins impliqués dans le mécanisme de douleur.

Dans un mode de réalisation non limitatif, la région correspond à un dermatome. Un dermatome est une bande de peau qui correspond à une innervation sélective d'un nerf donné de l'organisme. Grâce au procédé de cartographie selon l'invention, le médecin a accès aux pourcentages de la surface cutanée douloureuse comprise dans chaque dermatome. Dans un autre mode de réalisation non limitatif, la région correspond à une région lombaire.

Le procédé de cartographie 400 peut être mis en oeuvre à différents moments dans l'espace temporel pour un patient donné. On obtient ainsi une cartographie initiale et des cartographies de suivi, ce qui permet de comparer la région douloureuse avant et après que le patient ait reçu un traitement donné et en particulier une implantation de stimulation, par exemple médullaire. Les évaluations peuvent être également répétées dans le temps et à plusieurs moments de la journée ce qui permet, par implémentation de données, d'arriver à une approximation moyennée des régions douloureuses plus précise dans le temps, avec prise en compte des changements posturaux ou positionnels de l'individu tout au long du nycthémère.

Le procédé d'évaluation de l'efficacité d'une stimulation médullaire est utilisé pour :

- lors d'une opération d'implantation d'une ou plusieurs électrodes sur le patient, savoir si l'électrode est bien positionnée ou s'il est avantageux de la déplacer, et/ou
- en post-opératoire, faire un suivi du patient, c'est-à-dire vérifier que la couverture paresthésiante de la stimulation médullaire est (encore) efficace, et si elle peut prétendre continuer à soulager en effet le patient, en permettant, soit une diminution des surfaces douloureuses, soit de leur intensité.

Dans le premier cas, la stimulation médullaire est réalisée lors d'une opération d'implantation de ladite électrode. Ceci est particulièrement avantageux pour implanter l'électrode à un endroit où les stimulations médullaires seront efficaces. En pratique, le patient est réveillé pendant l'opération d'implantation, et indique au médecin, grâce au procédé d'évaluation de l'efficacité d'une stimulation médullaire selon l'invention, si l'électrode est placée à un endroit approprié. Si ce n'est pas le cas, le médecin peut alors déplacer l'électrode avant de rendormir le patient et terminer l'implantation.

Dans le second cas, la stimulation médullaire est réalisée postérieurement à une opération d'implantation de ladite électrode, par exemple quelques mois ou quelques années après l'opération. Cela permet de vérifier que le patient répond correctement aux stimulations médullaires, et que la zone douloureuse est toujours couverte par la zone de paresthésies générées par la stimulation. En effet, on observe parfois que les paresthésies s'atténuent avec le temps, voire disparaissent. Ainsi, un suivi cartographique du patient pendant la procédure d'implantation est possible en temps réel.

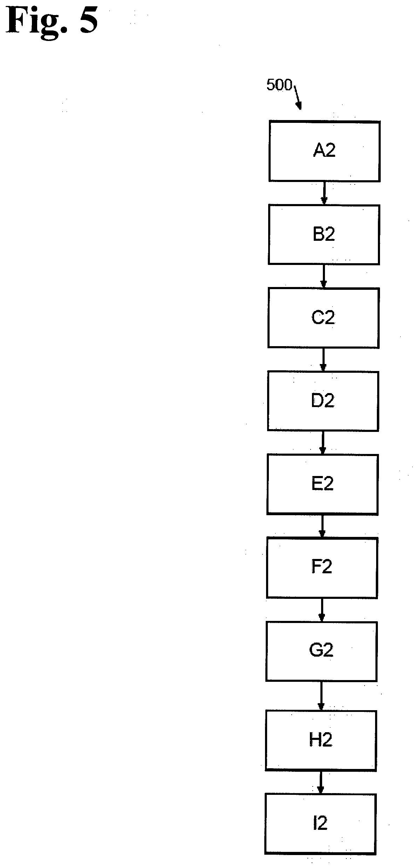

Plus précisément, en référence à la figure 5, le procédé d'évaluation de l'efficacité d'une stimulation médullaire 500 peut comporter les étapes suivantes :

- A2 : réaliser les étapes du procédé de cartographie 400 précédemment décrit.

- B2 : réaliser une stimulation médullaire sur le patient à l'aide d'au moins une électrode. Dans un mode de réalisation, l'électrode a été préalablement implantée lors d'une opération chirurgicale au niveau de la zone douloureuse 104 ressentie par le patient. Dans un autre mode de réalisation, la stimulation médullaire est réalisée lors d'une opération chirurgicale destinée à implanter l'électrode.

- C2 : afficher une deuxième silhouette sensiblement identique à la première silhouette 100, sur un deuxième écran. Le deuxième écran est par exemple un écran tactile, avantageusement un écran de tablette tactile. Dans un mode de réalisation non limitatif, le deuxième écran est le premier écran précédemment évoqué.

- D2 : dessiner au moins une zone de paresthésies sur la deuxième silhouette affichée, indiquant la localisation de paresthésies ressenties par le patient en réponse à la stimulation médullaire.

- E2 : déterminer un deuxième nombre de pixels du deuxième écran, correspondant à la zone de paresthésies.

- F2 : convertir le deuxième nombre de pixels en une surface cutanée de paresthésies, la distance repère précédemment évoquée intervenant comme paramètre de la conversion. La conversion est effectuée de la même manière qu'à l'étape E1 du procédé de cartographie 400. La surface cutanée paresthésique est alors affichée sur le deuxième écran, ce qui permet au médecin de disposer de l'information.

- G2 : comparer la surface cutanée paresthésique à la surface cutanée douloureuse déterminée lors de l'étape E1 du procédé de cartographie 400.

- H2 : superposer la deuxième silhouette et la première silhouette 100 sur le deuxième écran ou sur le premier écran 102.

- 12 : dans un mode de réalisation, la deuxième silhouette sur laquelle sont dessinées la ou les zones de paresthésies, ainsi que la surface cutanée paresthésique, sont enregistrées sur le serveur.

On note que dans un mode de réalisation non limitatif, le deuxième écran est le premier écran évoqué précédemment. Dans un mode de réalisation non limitatif, le deuxième écran est un écran tactile et le patient dessine la zone de paresthésies avec ses doigts. Dans un mode de réalisation non limitatif, la deuxième silhouette est sensiblement identique à la première silhouette évoquée précédemment. Avantageusement, la surface douloureuse a été prédéterminée au moyen du procédé de cartographie évoqué précédemment.

En comparant la surface cutanée paresthésique et la surface cutanée douloureuse, le médecin peut déterminer si la stimulation médullaire a été efficace, en termes de couverture.

Le procédé de cartographie selon l'invention permet donc de déterminer des paramètres de quantifications de la douleur et de son traitement :

- de manière stricte par le calcul de la surface douloureuse, et
- de manière indirecte par l'évaluation de la surface des paresthésies et enfin par le rapport douleur/paresthésies.

Tout ceci est réalisé en fonction des zones et territoires de distribution des fibres nerveuses et de leur représentation au niveau du système nerveux central (somatotopie). On note les deux aspects de l'invention :

- une méthode de diagnostic et thérapeutique (pré, per et post-opératoire) permettant de recueillir et de suivre la douleur du patient et son évolution, et permettre l'indication vers une thérapeutique de choix,
- une méthode d'évaluation d'une surface douloureuse en regard d'un dispositif médical en vue d'évaluer l'efficacité de celui-ci.

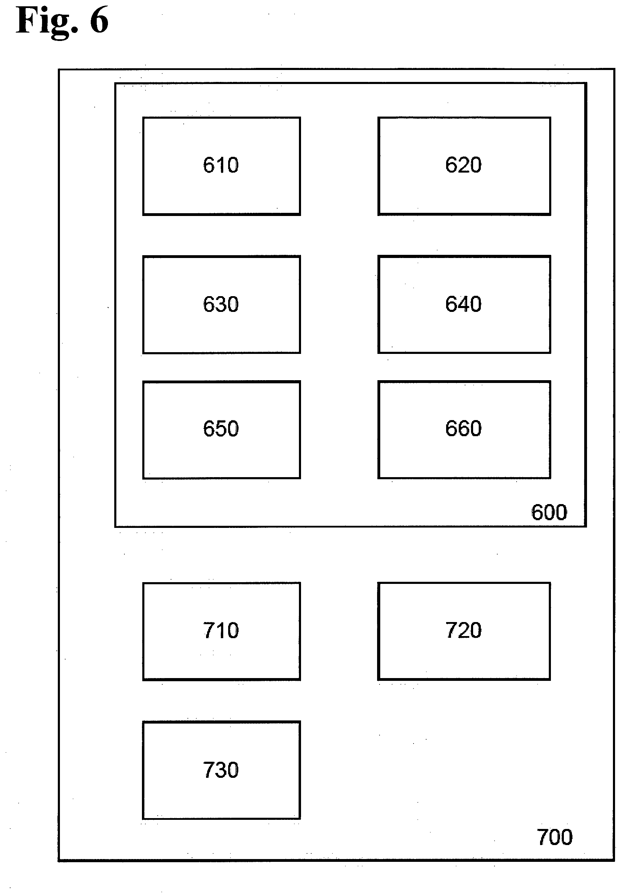

En référence à la figure 6, le système de cartographie 600 comprend :

- Des moyens d'affichage 610 d'une silhouette représentant la face postérieure d'un corps, sur un écran
- Des moyens de dessin 620 d'au moins une zone sur ladite silhouette
- Des moyens de détermination 630 d'un nombre de pixels de l'écran, correspondant à ladite zone
- Des moyens de conversion 640 dudit nombre de pixels en une surface cutanée, au moyen d'une distance repère intervenant comme paramètre de ladite conversion.

Il peut s'y ajouter :

- Des moyens de détermination 650 d'un ratio de ladite surface cutanée comprise dans une région de la silhouette
- Des moyens de comparaison 660 de la surface cutanée à une deuxième surface cutanée préalablement déterminée.
- Des moyens de correction de la surface cutanée au moyen d'un critère de correction.

Le système de cartographie 600 permet de mettre en oeuvre le procédé de cartographie 400 selon l'invention. Les zones dessinées peuvent correspondre à des zones douloureuses ou à des zones de paresthésies ressenties suite à une stimulation médullaire.

Le système d'évaluation de l'efficacité d'une stimulation médullaire 700 comporte :

- Le système de cartographie 600
- Des moyens de stimulation médullaire 710. Classiquement, il s'agit d'au moins une électrode multi contacts implantée sous la peau du patient, reliée à un boîtier de commande.
- Des moyens de comparaison 720 d'une surface cutanée douloureuse à une surface cutanée paresthésique, ladite surface paresthésique étant ressentie en réponse à une stimulation médullaire par les moyens de stimulation médullaire
- Des moyens de calcul 730 d'un pourcentage de recouvrement de la surface cutanée douloureuse par la surface cutanée paresthésique.

Le système d'évaluation de l'efficacité d'une stimulation médullaire 700 permet de mettre en oeuvre le procédé d'évaluation de l'efficacité de couverture d'une stimulation médullaire 500.

La suite de cette description porte sur des modes de réalisation préférentiels des systèmes 600 et 700.

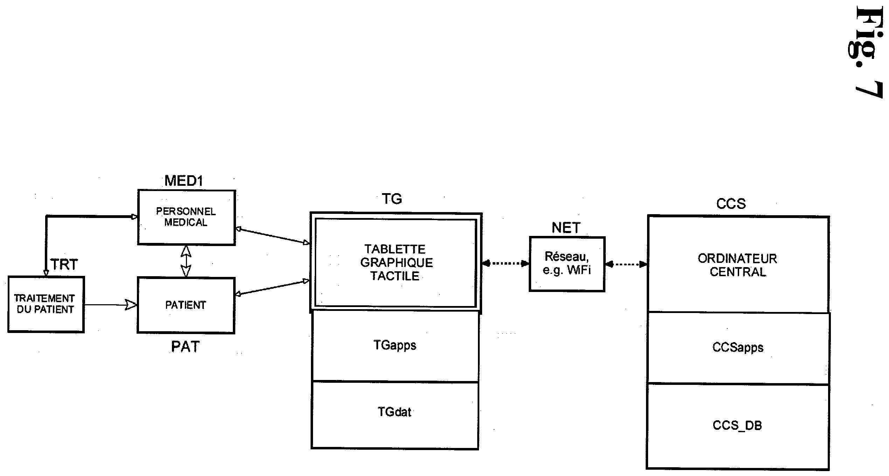

Sur la figure 7, la référence TG désigne un outil graphique tactile portable, ici une tablette graphique tactile de haute résolution, dans laquelle on fait apparaître une mémoire d'applications *TGapps,* et une mémoire de données *TGdat.*

La tablette graphique TG communique par un réseau local NET, par exemple de type WiFi, avec au moins un ordinateur central CCS, pour lequel on fait apparaître une mémoire d'applications *CCSapps,* et une mémoire de données contenant notamment une base de données CCS_DB.

Ici, l'ordinateur central est situé dans un hôpital. Il peut être partagé entre plusieurs hôpitaux, auquel cas le réseau NET est complété par une structure de communication réseau plus étendue. De façon générale, la tablette graphique TG peut donc accéder à la base de données centrale CCS_DB en temps réel par un réseau intranet ou via internet, directement ou à travers d'autres ordinateurs.

Le contexte comprend un patient PAT, au moins un personnel médical MED1, et un dispositif de traitement TRT du patient, sous le contrôle du personnel médical.

Le dispositif de traitement TRT peut être un dispositif de neurostimulation, qui est au contact du corps du patient et qui émet une stimulation électrique agissant sur le système nerveux du patient (ceci peut par exemple produire un picotement chez le patient).

Un dispositif de neurostimulation comprend une électrode de stimulation implantable, généralement à plusieurs contacts, montés dans une configuration géométrique déterminée, des extensions optionnelles reliant l'électrode à l'élément suivant, au besoin, et un générateur électrique implantable, situé par exemple sous la peau du ventre ou sous la fesse, souvent muni d'une télécommande qui permet de commander sélectivement lesquels des contacts sont électriquement alimentés ainsi que les paramètres de stimulation. Cette commande sélective définit une configuration de travail du dispositif de neurostimulation.

Là où les médecins français utilisent le mot "électrode", on utilise le mot "lead" en anglais. Le mot "neurostimulateur" est parfois utilisé en pratique pour désigner le seul générateur électrique, puisqu'il règle la configuration de travail du dispositif de neurostimulation.

Dans la présente description, on utilise de façon synonyme les mots "électrode", "dispositif de neurostimulation", "neurostimulateur" ou plus brièvement "stimulateur" pour désigner l'ensemble du dispositif de neurostimulation. Lorsqu'on utilise le mot "électrode" ou "lead", il est entendu que le reste d'un dispositif de neurostimulation lui est connecté.

Les fonctions 610, 620, 630, 640, 650 et 660 de la figure 6 peuvent être mises en oeuvre, en partie au moins (y compris pour chaque fonction), par les applications *CCSapps.* Des fonctions de calcul complexes peuvent être déléguées à l'ordinateur central CCS (ou l'un d'entre eux), en fonction des puissances de calcul respectives de la tablette graphique TG et de l'ordinateur central CCS. Le dispositif de traitement TRT peut correspondre aux moyens de stimulation médullaire 710 de la figure 6, ou à d'autres modes de traitement. Les fonctions de comparaison 720 d'une surface cutanée douloureuse à une surface cutanée paresthésique, ainsi que de calcul 730 d'un pourcentage de recouvrement de la surface cutanée douloureuse par la surface cutanée paresthésique peuvent être mises en oeuvre dans la tablette TG, dans l'ordinateur CCS, ou partagées entre eux, là aussi en fonction des puissances de calcul respectives.

Comme le montre la figure 7, le personnel médical MED1 et le patient PAT vont interagir avec la tablette graphique TG. Pour cela, dans la tablette graphique TG, les applications *TGapps* peuvent être rendues disponibles au moyen d'un menu arborescent à onglets (dits "*tabs*"). Ce menu peut être vu comme une application pilote *TGappmain,* à disposition du personnel médical MED1.

Ainsi, dans le bandeau vertical gauche de la figure 8 (et figure 8bis) apparaissent 6 onglets ou "Tabs", numérotés de 1 à 6.

L'interaction se produit en plusieurs phases.

Une première application *TG_app1* permet la saisie d'une fiche patient. La saisie est faite par le personnel médical, en interrogeant au besoin le patient.

Une liste de données patients est maintenue dans une base de données centrale telle que CCS_DB. De manière connue, cette liste de données patients (ou fiche patient) est agencée pour respecter les précautions de confidentialité applicables, notamment lorsqu'on y accède. Elle est gérée par une application *CCSapp0* logée du côté de l'ordinateur central CCS, qui peut dialoguer avec la tablette, et notamment l'application *TG_app1* de celle-ci.

L'application *TG_app1* permet de "créer" une fiche patient, ainsi que de compléter les données relatives à un patient dans une fiche déjà existante. Cela peut se faire en liaison directe avec la fiche patient existant dans la base de données centrale CCS_DB, ou par un stockage d'une ou plusieurs fiches patient dans la mémoire TGdat de la tablette graphique TG, la base de données centrale CCS_DB étant mise à jour par la suite.

Pour la clarté, on va distinguer plusieurs sections dans une fiche patient, chacune pouvant avoir plusieurs sous-sections.

**Section S1 - Données relatives au patient**

La fiche patient comprend d'abord une première section S1, générale. Elle sera décrite en référence à la copie d'écran de la figure 8 (et figure 8bis). Celle-ci est en anglais, langue de travail habituelle des scientifiques et des informaticiens, et on en donne la traduction ci-après dans des tableaux.

Pour cette section S1, l'onglet en cours est l'onglet 1, qui correspond aux données patient ("Patient Data") de la section 1. On les trouve sur la figure 8 (et figure 8bis), à droite du bandeau.

La première sous-section S11 de cette section S1 contient des caractéristiques générales du patient ("General characteristics"), qui peuvent être celles du tableau TS11a ci-après.

| Tableau TS11a | | |
|---|---|---|
| Nom anglais | Nom français | Accès |
| Patient ID | Code patient | Saisi |
| Gender | Sexe | Saisi |
| Date of birth | Date de naissance | Saisi |
| Age | Age | Calculé |

La première sous-section S11 contient aussi des caractéristiques morphométriques du patient ("Morphometric characteristics"), telles que celles du tableau TS11b ci-après. Les caractéristiques morphométriques correspondent à une description anatomique du patient comprenant au moins un paramètre anatomique de référence. Dans le présent mode de réalisation, ce paramètre de référence est la distance entre les crêtes iliaques.

| Tableau TS11b | | |
|---|---|---|
| Nom anglais | Nom français | Accès |
| Height | Taille | Saisi |
| Weight | Poids | Saisi |
| BMI | IMC (indice de masse corporelle) | Calculé |
| Distance between iliac crests | distance entre crêtes iliaques | Saisi |

La troisième colonne des tableaux TS11a et TS11b indique si la valeur en question est saisie, ou bien calculée d'après des valeurs déjà saisies.

La deuxième sous-section S12 vise l'étiologie du patient à sa première visite (première visite pour ses problèmes, ou symptômes douloureux en cours). L'étiologie peut être désignée parmi une liste de possibilités par exemple celle du tableau suivant TS12.

| Tableau TS12 | |
|---|---|
| Nom anglais | Nom français |
| Cluster Headache | Algie vasculaire de la face |
| Arnold Neuralgia | Névralgie d'Arnold |
| Chronic Migraine | Migraine chronique |
| Chronic Back Pain | Douleurs chroniques dorso-lombaires ou dorsolombalgies chroniques |
| Chronic Back and Leg Pain (CBLP) | Lombo-radiculalgies chroniques |
| Failed Back Surgery Syndrome (FBSS) | Lombo-radiculalgies post-opératoires (LRPO) |
| Complex Regional Pain Syndrome (CRPS) | Syndrome Douloureux Régional Complexe (SDRC) |
| Peripheral Vascular Disease (PVD) | Maladie vasculaire périphérique Artérite Oblitérante des Membres Inférieurs (AOMI) |
| Phantom Limb Pain | Douleur de membre fantôme |

Une troisième sous-section S13 concerne la localisation générale et actuelle de la douleur parmi une liste de possibilités, comme par exemple celles indiquées dans le tableau suivant TS13.

| Tableau TS13 | |
|---|---|
| Nom anglais | Nom français |
| Headache | céphalées |
| Axial Pain | douleur axiale |
| Back and Leg Pain | douleur du dos et des jambes |
| Lower Extremity(ies) | douleur des extrémités inférieures |
| Upper Extremity(ies) | douleur des extrémités supérieures |

Ceci est illustré sur la figure 8 (et figure 8bis), sous l'intitulé "Pain characteristics", i.e. caractéristiques relatives à la douleur.

Sur la figure 8 (et figure 8bis), on saisit d'abord l'étiologie, ici "Failed Back Surgery Syndrome (FBSS)" (sous-section S12 et tableau TS12 ci-dessus), puis le ressenti douloureux général, ici "Back and Leg Pain" (sous-section S13 et tableau TS13 ci-dessus).

On peut prévoir une quatrième sous-section S14, qui décrit, le cas échéant le ou les neurostimulateurs ("Type of lead") déjà implantés ou à implanter sur le patient, par exemple en notant à chaque fois le nom usuel du neurostimulateur, ainsi que des attributs de l'implant comme ceux indiqués dans le tableau suivant TS14.

| TS14 | |
|---|---|
| Nom anglais | Nom français |
| Monocolumn Percutaneous lead | Electrode monocolonne percutanée |
| Monocolumn Surgical lead | Electrode monocolonne chirurgicale |
| Monocolumn subcutaneous lead | Electrode monocolonne sous-cutanée |
| Multicolumn surgical lead | Electrode multicolonnes chirurgicale |

La figure 8 (et figure 8bis) comprend enfin l'énoncé de jusqu'à quatre neurostimulateurs implantés : "Type of lead No 1" à "Type of lead No 4", ici non renseignés, car le patient n'est pas encore implanté. Si le patient est déjà implanté, chaque zone "Type of lead" peut être renseignée conformément à la sous-section S14 et au tableau TS14.

**Section S2 - Données relatives à l'image support de la cartographie**

La saisie d'une fiche patient se poursuit avec des données d'imagerie, qui définissent le type d'image sur laquelle on va demander au patient de dessiner sa zone douloureuse (notamment).

Ainsi, la seconde section S2 de la fiche patient va comprendre une sous-section S21, qui définit le choix premier de la représentation sur laquelle le patient va dessiner, par exemple comme indiqué dans le tableau TS21.

| TS21 | | |
|---|---|---|
| Choix | Nom anglais | Nom français |
| S21-a | 2D Schematic view | Vue schématique 2D |
| S21-b | 2D Selected view | Vue sélectionnée 2D |
| S21-c | 2D Real view | Vue réelle 2D |
| S21-d | 3D Real view | Vue réelle 3D |

Davantage de détails concernant le choix S21-a à S21-d sont décrits ci-après :

S21-a : La représentation schématique bidimensionnelle dite "Vue schématique 2D" correspond à un gabarit classique tenant uniquement compte du sexe de l'individu et rapporté à sa taille;

S21-b : La représentation sélectionnée bidimensionnelle dite "Vue sélectionnée 2D" est choisie dans une collection de représentations corporelles types, intégrant le sexe et la taille de l'individu, et rapportées à son IMC;

S21-c : La représentation photographique dite "Vue réelle 2D" est une représentation tirée d'une photo du patient prise en temps réel, et traitée par un processus de recalage et de conversion numérique (cf. infra) ; et

S21-d : La représentation tridimensionnelle dite "Vue réelle 3D" peut provenir par exemple d'une reconstruction à partir d'une acquisition tridimensionnelle certifiée médicalement (en anglais « Medical Certified »), à l'échelle réelle du patient.

Sur la figure 9 (et figure 9bis), l'onglet 2 est activé. Le choix se fait dans la boîte à option "acquisition type". Les boutons "Back" et "Front" permettent de choisir entre vue de dos et vue de face.

Pour les choix S21-a et S21-b, deux sous-sections auxiliaires S22 permettent d'interroger la base de données centrale CCS_DB, pour télécharger une silhouette parmi un jeu de silhouettes standard prédéfinies, notamment pour distinguer une silhouette féminine d'une silhouette masculine (S21-a et b). Le choix S21-b offre plus de précision car il est notamment possible tenir compte de la corpulence du patient, en choisissant une silhouette précalibrée en fonction de l'indice de masse corporelle du patient.

Le choix S21-c comportant une silhouette réelle du patient 2D à deux dimensions peut être une vue de dos, ou une vue de face ou les deux. Il s'agit par exemple d'une photo du patient déjà existante dans la base de données CCS_DB. Dans ce cas, la photo peut-être une photo de résolution prédéfinie ou encore peut-être une photo prise selon la qualité définie par un standard médical (standard DICOM notamment) ou encore une photo faisant l'objet d'un traitement de recalage et de conversion spécifique et intégré au logiciel.

Par ailleurs, pour le choix 21-c, une photographie peut être prise lors de la consultation en cours, par exemple avec une caméra intégrée sur la tablette graphique TG. La photographie peut être prise en une résolution et à une distance prédéfinies. La photographie peut aussi être prise avec une première résolution à une distance donnée, puis être recalibrée en une deuxième résolution prédéfinie.

Pour le choix S21-d, une silhouette 3D peut être tirée d'un jeu d'images préexistantes, obtenues par exemple par tomodensitométrie dite aussi tomographie par ordinateur (CT pour "*Computer Tomography*"), ou par imagerie par résonance magnétique (IRM ou en anglais MRI pour "*Magnetic Resonance Imaging*").

Le portrait de la douleur sur une cartographie 3D est possible notamment si l'on dispose d'images scanographiques (CT) ou IRM du patient. Comme on le verra, il est possible de transformer de telles images en silhouette 3D rapportée à une métrique réelle, avec laquelle il devient possible d'établir une cartographie tridimensionnelle de la douleur.

On admet que l'on disposait déjà d'un portrait de la douleur sur une cartographie 2D pour un patient donné. Si l'on venait à disposer d'une image CT ou MRI du patient en cours de traitement, il serait envisageable de passer à une silhouette 3D en métrique réelle, et de récupérer les surfaces de la cartographie 2D antérieure, puisque celle-ci est aussi en métrique réelle, tout en effectuant les recalages 2D/3D appropriés.

La cartographie 3D permet notamment de tenir compte:

- des creux et des bosses corporels et donc de l'aire de la surface;
- des douleurs de type irradiations circulaires ;
- des aires de surfaces estimées sur des tronçons cylindriques, notamment de membres ;
- d'une unité minimale en cm$^2$ exprimée par le biais de la technique dite des triangles (bien plus précise que la technique utilisant des pixels de forme carrée).

La cartographie 3D permet de localiser une douleur et d'enregistrer son évolution en trois dimensions. La cartographie 3D permet ainsi de déterminer le siège exact (y compris la profondeur sous-cutanée) d'une douleur, de suivre son l'évolution dans le temps et d'observer plus précisément, le cas échéant, une migration de la douleur vers un autre siège.

Tout comme la cartographie 2D, la cartographie 3D permet de mener des études descriptives prenant en compte différents types de segmentations spatiales.

Les différentes opérations pour l'acquisition des données de douleurs sont analogues à ce qui sera décrit ci-dessous pour la cartographie 2D (en faisant référence aux figures 9 à 17).

Toutefois, lorsque le patient « peint » ou portraite ses douleurs ou ses paresthésies sur une silhouette 3D, la partie de la surface 2D de l'écran qui est le siège de l'impact tactile se trouve projetée sur la silhouette 3D, dès que le doigt du patient quitte l'écran. Cette projection peut être vue comme un « jet ou spray de peinture », partant de la zone peinte de l'écran 2D, et dirigé sur la silhouette 3D. En résumé, le portrait de douleur tient compte de la forme corporelle réelle et tridimensionnelle du patient.

La silhouette 3D est modélisée par exemple sous forme de triangles contigus. La "peinture", sur la silhouette 3D, des surfaces douloureuses ou paresthésiantes est donc possible, de manière instantanée et homogène. On "peint" ou remplit de petits triangles contigus sur l'écorce cutanée tridimensionnelle de la silhouette 3D, en même temps que des pixels carrés sur l'écran 2D. Si le spray de peinture résultant de l'impact tactile sur l'écran, est hors champ par rapport à la silhouette 3D, il est sans effet sur le corps in fine.

La durée d'application du doigt sur l'écran et le nombre de passages de sprays itératifs au même site anatomique ne modifient pas la délimitation des surfaces, qui ne prendra en compte qu'une seule fois la surface cutanée colorée.

Le calcul surfacique peut s'effectuer sur la base de la représentation 3D par petits triangles (ou autres). Il en est de même pour le calcul de l'écart type.

La cartographie 3D est plus exacte dans l'estimation des surfaces non planes, comme les surfaces curvilignes au niveau du tronc et des membres, ainsi que les reliefs liés aux anfractuosités.

La cartographie 3D convient aussi à la distribution dite « spiroïde » de certains dermatomes, comme ceux des membres : par exemple, le trajet d'une névralgie sciatique résultant d'une compression de la racine nerveuse L5 correspond à la face postérieure de la fesse, la face latérale de la cuisse, la face antéro-latérale du mollet, la face supérieure du cou de pied et le gros orteil. En mode cartographique 2D, il existe un risque de prise en compte de la même surface qui serait à cheval entre la vue antérieure et postérieure de la silhouette. Au pire, elle serait comptabilisée deux fois dans le calcul surfacique ou bien sous-estimée à l'inverse car sa surface « à plat », i.e. en 2D ne correspond pas à son aire sur une surface, correspondant à la réalité (autre exemple de la névralgie intercostale, en bande de la colonne jusqu'à la face antérieure du thorax).

Enfin, la cartographie 3D permet de prendre en compte la notion de profondeur. La douleur est une projection cutanée car c'est la peau qui contient la plupart des nerfs nocicepteurs ; mais certaines douleurs sourdes ou profondes correspondent à des conflits ou stimuli douloureux plus profonds, provenant des capsules articulaires, des disques intervertébraux, par exemple, et leurs projections douloureuses sont beaucoup plus difficiles à apprécier. La cartographie 3D facilite la prise en compte de la profondeur de la douleur, en dessinant d'une manière différente une douleur sourde et/ou profonde, avec un ou des indices relatifs de profondeurs.

C'est applicable au cas de la douleur « transfixiante ». La cartographie 3D peut permettre de décomposer (ou « découpler ») les composantes douloureuses superficielles/profondes. On peut rapprocher cela de l'appréciation qualitative de la douleur, décrite par le patient (« sensation d'étau, de serrement, de broiement, de coup de poignard transfixiant »).

De même, sur traitement, au niveau paresthésique, il devient possible de distinguer une couverture superficielle due à une stimulation sous-cutanée, à quelques millimètres de l'hypoderme, et certaines sensations profondes résultant d'une stimulation centrale (par exemple médullaire) ou encore d'une stimulation combinée hybride, générant une sensation beaucoup plus profonde, diffuse, globale et « enveloppante » selon la description des patients, probablement parce qu'elle concerne alors des fibres centrales et périphériques, des territoires moins spécifiques et une variété de fibres sensitives plus importante.

La capture de cartographie 3D permet donc de représenter métriquement la douleur d'un patient sur l'écran de la tablette graphique TG et de la même manière que pour l'outil de cartographie 2D les intensités et la typologie des douleurs, selon le même code couleur et les mêmes procédés de calcul. Cela permet de quantifier les douleurs d'un patient, de réaliser des calculs dans un système métrique (calculs statistiques par exemple) et de suivre l'évolution ou, le cas échéant, la migration de la douleur du patient.

L'ensemble des données récoltées peuvent-être exportées vers la base de données CCS_DB du serveur neuro-informatique pour effectuer les calculs. Les écrans illustrés comportent en haut et à droite une touche qui commande l'exportation des données vers le serveur.

On notera que les représentations 2D ou 3D ainsi que les photos dont il est question pour les choix S21-a à S21-d, peuvent subir un certain nombre de modifications pour adapter, le cas échéant, leur taille et forme (modification par la fonction « rogner » pour découper une photo afin de représenter uniquement une partie anatomique ciblée, telle que le buste par exemple).

Dans le menu arborescent à onglets, on a décrit ci-dessus l'onglet 1, qui concerne les données patient, et l'onglet 2, qui définit le type d'image sur laquelle on va travailler.

Les sections/onglets suivants 3 à 6 font intervenir le patient, comme on le verra.

Deux opérations de calibration différentes sont prévues. Elles peuvent se dérouler avant que le patient portraite sa douleur. Ces deux calibrations sont réalisées lorsque le patient prend en main la tablette graphique TG (avec le logiciel de calibration) pour la première fois. Il n'est pas exclu que cette étape puisse être en partie réalisée en début de consultation, par exemple dans une salle de consultation prévue à cet effet.

**Section S3 - Calibration 1**

Cette section S3 porte sur l'interaction du patient avec l'écran tactile de la tablette graphique TG, en étudiant comment le patient dessine/portraite sur l'écran.

Les dispositifs de pointage classiques - souris ou stylet - ont une bonne précision, et semblent s'imposer pour du dessin. À l'inverse, le pointage tactile par le doigt suffit pour actionner une touche sur l'écran, mais il est nettement moins précis pour dessiner une surface et/ou un contour, à l'intérieur de la silhouette (2D ou 3D), la vue schématique ou la photo affichée à l'écran.

Les études du Demandeur ont cependant montré qu'il était possible de bénéficier du côté plus intuitif et plus pratique du pointage tactile par le doigt, tout en conservant une précision suffisante en termes de positionnement et de surface. Typiquement, une incertitude de surface allant jusqu'à 4 cm$^2$ est considérée actuellement comme acceptable puisqu'elle correspond aux limites de discrimination moyenne du tact épicritique dans la région dorsale chez l'individu humain.

D'autre part, un mode zoom est proposé au patient pour délimiter précisément certaines régions d'intérêt à la surface limitée.

Cette première calibration, ou Calibration 1, a pour but d'affiner la précision du toucher du patient par rapport à la résolution et aux caractéristiques de l'écran (paramètre propre interface homme-machine, avec un facteur correctif appliqué instantanément pour les saisies ultérieures).

L'application « appCalib1 » affiche plusieurs points sur l'écran tactile de la tablette graphique TG. Dans un exemple, six points sont affichés successivement à l'écran à des emplacements prédéfinis en X et en Y dans le plan de coordonnées d'affichage : quatre points proches (intérieurement) des quatre coins de l'écran, un point au centre de l'écran et un point placé aléatoirement sur l'écran. Ces points "cibles" sont représentés par exemple par une croix cerclée. Pour chaque point, on attache une position désirée $X_0$, $Y_0$ correspondant au centre de la croix.

Pour chaque point, le patient doit toucher le plus précisément possible le centre de la cible affichée sur l'écran, selon sa meilleure intuition. L'application « appCalib1 » enregistre les coordonnées ($X_1$, $Y_1$) du pixel au centre de l'impact du doigt du patient sur l'écran tactile. Cette étape est réalisée pour les six points du processus de calibration.

Pour chaque point, l'application « appCalib1 » calcule les écarts suivants :

$$\Delta X = X_0 - X_1 \qquad \text{et} \qquad \Delta Y = Y_0 - Y_1$$

entre la position désirée du point-cible et la position du pixel au centre de l'impact tactile (touché par le patient). La moyenne et l'écart-type des $\Delta X$ et $\Delta Y$ sont calculés.

L'application appCalib1 définit alors une première série de paramètres correctifs en X et Y, notés Dcal1x et Dcal1y, qui seront appliqués par la suite aux saisies tactiles du patient sur l'écran tactile de la tablette graphique TG.

On peut appliquer la même correction en X et en Y pour tout l'écran, auquel cas Dcal1x et Dcal1y sont des constantes.

En variante, notamment si les écarts ne sont pas les mêmes aux coins et au centre, on peut utiliser une paire de corrections en X et Y pour chacun des points testés. Dans ce cas, Dcal1x et Dcal1y sont des fonctions de X et de Y. Par la suite, lorsque le patient touchera une zone de l'écran, on pourra calculer des corrections en X et Y interpolées selon la situation de ce point par rapport aux quatre coins et au centre.

Le point aléatoire permet notamment de vérifier la cohérence du comportement du patient lorsqu'il pointe sur l'écran.

Un mode de réalisation particulier est illustré sur la figure 10.

L'écran initial est sur la partie gauche de la figure 10. Ensuite, au centre, le patient est invité à toucher la cible (en anglais « Tap on target »). Quand cela a été fait pour les 6 points, la dispersion des touchers est affichée dans l'écran représenté à droite de la figure 10. Les écarts moyens en X "Mean ΔX" et en Y "Mean ΔY" ainsi que leurs écarts types "Sd" respectifs sont alors affichés.

## Section S4 : Calibration 2

Cette seconde calibration est dénommée : « autocalibration » pour le patient (en anglais « *Patient AutoCalibration* »). Elle porte sur la manière dont le patient perçoit dans l'espace des sensations en des points ou zones de son corps, par exemple où il ressent une douleur. Autrement dit, elle a pour but d'analyser la capacité du patient à « géo-localiser » un point de son propre corps.

Lorsque cette géo-localisation concerne par exemple un point situé sur le dos du patient, ce-dernier doit opérer en aveugle. La capacité de géo-localiser des points en aveugle est un exercice très difficile. Ainsi, l'opération d'autocalibration de la présente invention offre une possibilité de mieux la maîtriser.

Le problème est le suivant. On considère par exemple une douleur ponctuelle dans le haut du dos. Le Demandeur a observé que, le plus souvent, le patient définit assez bien la position de cette douleur ponctuelle dans la direction verticale. Par contre, ses sensations spatiales sont beaucoup moins nettes dans la direction horizontale. De même, un patient ne décrira pas systématiquement avec la même précision la géo-localisation d'une douleur thoracique droite et la géo-localisation d'une douleur lombaire gauche.

De façon générale, on procède comme suit :

- un personnel d'assistance ou "hétéro-observateur" va toucher le patient en des points successifs choisis, ou points cibles, dont les coordonnées $X_0(i)$ et $Y_0(i)$ sont connues de la tablette graphique TG ;
- à chaque fois, le patient va toucher la silhouette (dans la suite on entend par « silhouette » une silhouette 2D, 3D, une vue schématique ou une photo) sur la tablette graphique TG, au point $X_1(i)$ et $Y_1(i)$ où il pense avoir ressenti le toucher de l'hétéro-observateur.
- À chaque fois, la tablette détermine les écarts entre le point-cible et le point touché par le patient sur la silhouette :

$$\Delta X = X_1(i) - X_0(i)$$

$$\Delta Y = Y_1(i) - Y_0(i)$$

où i est l'itération.

Le mot "hétéro-observateur" indique que cette personne n'est pas nécessairement un médecin ou un personnel infirmier. Une personne qualifiée en anatomie peut superviser cette Calibration 2.

Ceci permet notamment de prendre en compte les décalages systématiques entre les points du dos du patient tels qu'ils sont physiquement, et les points du dos du patient tels qu'il les ressent (et qu'il les indique à la tablette graphique TG).

L'écart type de la distribution fournit une appréciation de la fiabilité de la localisation par le patient de ses sensations physiques, et par conséquent de la fiabilité de la représentation par le patient sur l'écran de la tablette graphique TG desdites sensations physiques.

Le résultat de cette Calibration 2 comprendra donc des paramètres d'écart propres à l'individu, avec des facteurs correctifs appliqués dans le post-traitement des données de toucher relatives à cet individu (Calibration 1). Le cas échéant, la Calibration 2 va exclure des patients ayant une trop mauvaise perception de leur représentation corporelle, typiquement si 5 à 6 points touchés par le patient sont en dehors de la cible de référence.

Plus précisément pour cette Calibration 2, on appliquera à chaque toucher tactile réalisé par le patient les paramètres correctifs Dcal1x et Dcal1y, tels que définis par l'application appCalib1. À noter que la Calibration 2 s'appliquerait même si le pointage était non tactile, et s'effectuait à l'aide d'un stylet ou d'une souris, par exemple, auquel cas on pourrait se dispenser de la Calibration 1.

Cette section S4 correspond à l'onglet 4 de l'écran de la figure 8 (et figure 8bis). Lorsque l'onglet 4 est activé, on obtient par exemple l'écran de lancement illustré sur la figure 11 (et figure 11bis).

Cette Calibration 2 peut être réalisée selon 2 protocoles :

- Un protocole simplifié dit « calibration rapide ».
- Un protocole optimisé par les résultats issus des tests de validation statistique du logiciel appelé « calibration optimisée ».

La sélection entre les deux protocoles peut se faire par un écran comme celui de la figure 11 (et figure 11bis).

**Calibration rapide (en anglais « *Quick calibration* »)**

Six points du corps du patient prédéfinis dans l'application « appCalib2 » et facilement identifiables par des repères anatomiques standards (cf. infra Tableau TS41) sont montrés à un hétéro-observateur (sur un écran dédié à celui-ci) sur une vue schématique du patient et ne sont pas connus de ce dernier. Le mot « hétéro-observateur » indique une personne qualifiée en anatomie, il n'est pour autant pas nécessaire pour cette phase de recourir systématiquement à un médecin ou un personnel infirmier.

L'hétéro-observateur va par la suite successivement toucher sur le corps du patient ces six différents points que l'on appelle ici "cibles de calibration" (cf. infra Tableau TS41). Le patient devra pour chaque point touché par l'hétéro-observateur, désigner la cible où il ressent le toucher de l'hétéro-observateur sur la silhouette que l'application « appCalib2 » présente à l'écran.

Une variante est illustrée sur la figure 12 (et figure 12bis). L'hétéro-observateur va marquer directement sur le corps du patient, à l'aide d'un stylo-feutre spécifique, des repères anatomiques précis (prédéfinis par le protocole de validation statistique), correspondant aux « cibles de calibration » précitées.

L'hétéro-observateur va ensuite prendre une photo du patient, vu de dos, en utilisant l'appareil photo disponible sur la tablette graphique TG. La photo peut être automatiquement rognée par l'application « appCalib2 » pour ne conserver qu'une partie de corps choisie. Ladite partie de corps peut-être la partie du corps strictement comprise entre le sommet de la voute crânienne (vertex) et l'appui des talons des pieds du patient. Dans le présent exemple de réalisation la partie de corps est la partie strictement comprise entre le sommet de la voute crânienne (vertex) et les plis fessiers.

Dans une première opération, les coordonnées de marques de feutre sur la photo sont recherchées par la tablette graphique TG et enregistrées dans sa mémoire. La saisie et l'enregistrement des coordonnées peuvent être réalisés manuellement par une saisie des coordonnées respectives en abscisse et ordonnée X et Y, puis enregistrement. En variante, l'enregistrement peut être réalisé automatiquement par une reconnaissance photographique des marquages de stylo-feutre ou encore par l'apposition temporaire de sondes autocollantes sur le dos du patient. Dans le présent mode de réalisation il s'agit d'une reconnaissance photographique des marquages de stylo-feutre.

Dans une deuxième opération, le patient désigne sur la photo où il ressent le toucher de l'hétéro-observateur pour chaque cible de calibration. Aucun marquage des cibles de calibrations (et leurs coordonnées) ne sont visibles pour le patient lors de cette opération. À cet effet, les marquages de stylo-feutre sont éliminés sur la photo à l'aide d'un filtre spécifique (par exemple un filtre de couleur) avant de soumettre l'affichage au patient.

Un exemple de distribution des écarts entre points-cibles et points-patient est illustré en bas et à droite de la figure 13 (et figure 13bis).

**Calibration optimisée (en anglais « _Optimized calibration_ »)**

La calibration optimisée s'effectue à plusieurs reprises, par exemple sur 5 cycles. L'ordre dans lequel l'hétéro-observateur devra toucher les cibles et faire indiquer au patient le ressenti précis de chaque cible est rendu quasi aléatoire (ou procédé au hasard). L'ordre qui résulte de cette randomisation est indiqué à l'hétéro-observateur à chaque début de cycle ou de test sur un écran confidentiel. Ceci permet de limiter le biais d'apprentissage « chronologique » de la géo-localisation des cibles.

Dans un mode de réalisation, l'ordre dans lequel sont présentées les cibles est automatiquement généré par un algorithme utilisant pour clé des données d'identité du patient. Ceci permet d'avoir le même ordre sur deux ordinateurs avec deux écrans séparés respectifs, utilisés simultanément : l'un pour guider l'hétéro-observateur tout au long du test, et l'autre (la tablette) pour prendre en compte les impacts tactiles du patient, procéder à l'analyse et afficher ensuite les écarts et facteurs correctifs à appliquer, en fonction des régions.

C'est donc une photo vierge de toute trace de feutre qui sera présentée au patient, à chaque pas du test de calibration (figure 13 en bas à gauche).

L'hétéro-observateur va par la suite toucher successivement les six différentes cibles de calibration sur le corps du patient, en fonction de l'ordre de randomisation affecté par l'application à chaque cible prédéfinie. Le patient devra à chaque fois désigner à l'écran, le point où il ressent le toucher de l'hétéro-observateur sur sa propre photo. Cette étape va être réalisée à cinq reprises.

Un exemple de distribution des écarts entre points-cibles et points-patient est illustrée en bas et à gauche de la figure 13 (et figure 13bis).

Le procédé « _optimized calibration_ » nécessite plus de temps que le procédé de calibration rapide (« _quick calibration_ ») mais permet une estimation plus précise de la capacité du patient à représenter sa propre perception corporelle, appliquée au dos et ainsi d'attribuer des facteurs correctifs fiabilisés sur les données sources, en post-traitement et en fonction des sous-régions dorso-lombaires concernées.

**Cibles de calibration**

Les cibles de calibration peuvent être celles du tableau suivant TS41 :

| Tableau TS41 | |
| --- | --- |
| Nom anglais | Nom français |
| Right iliac crest | Apex de la crête iliaque droite |
| Left iliac crest | Apex de la crête iliaque gauche |
| Right scapula inferior angle | Angle inférieur de la scapula droite |
| Left scapula inferior angle | Angle inférieur de la scapula gauche |

(suite)

| Tableau TS41 | |
|---|---|
| Nom anglais | Nom français |
| Right gluteal fold center | Milieu du pli fessier droit |
| Left gluteal fold center | Milieu du pli fessier gauche |
| 3rd lumbar vertebra (L3) | Sommet de l'épineuse de la 3ème vertèbre lombaire (L3) |
| Junction between 7th cervical vertebra (C7) and 1st thoracic vertebra (T1) | Sommet de la jonction ligamentaire entre l'épineuse de la 7ème vertèbre cervicale (C7) et celle de la 1ère vertèbre thoracique (T1) |

En résumé, cette calibration optimisée permet notamment de prendre en compte les décalages entre les cibles de calibration du dos du patient telles qu'elles sont définies physiquement (cibles réelles de coordonnées $X_0$ et $Y_0$), et la perception de ces cibles de calibration (ressenti des cibles de coordonnées $X_1$ et $V_1$) par le patient, tels qu'il les indique sur la tablette graphique TG. À chaque fois que le patient touche le plus précisément possible l'écran, l'application « appCalib2 » localise la position (coordonnées X et Y) de la zone touchée par le patient.

On obtient six valeurs d'écarts de coordonnées en X et en Y

$$\Delta X = X_1(i) - X_0(i)$$

$$\Delta Y = Y_1(i) - Y_0(i)$$

Avec i allant de 1 à 6.

Cela peut être représenté sous la forme de 6 mires, comparant les emplacements des pixels localisés au centre des "cibles de calibration" touchées par l'hétéro-observateur et les emplacements des pixels au centre des impacts tactiles correspondant aux positions ressenties par le patient. C'est ce qu'on voit en bas et à droite de la figure 13, pour la calibration rapide.

Des considérations statistiques sont applicables, par exemple s'il y a des écarts trop importants, ou encore si l'écart type montre que la distribution des écarts est trop éloignée d'une distribution gaussienne. Elles permettront à l'application appCalib2 de décider d'accepter ou de refuser la calibration.

Si la calibration est acceptée, l'application appCalib2 définit une deuxième série de paramètres correctifs en X et Y, Dcal2x et Dcal2y, qui seront appliqués aux données saisies, dans la phase de post-traitement. Ces paramètres Dcal2x et Dcal2y pourront varier en fonction de la région du dos (ou plus généralement de son corps) dans laquelle le patient saisira les données sources.

La calibration rapide peut suffire pour les patients qui ont une bonne sensation spatiale de leur corps, ou encore pour vérifier une calibration déjà faite antérieurement.

La calibration optimisée ou approfondie permet de mieux apprécier si le patient a une bonne sensation spatiale de son corps. En passant de 1 à 5 cycles, on obtient des informations plus riches, au moins statistiquement.

En outre, les inventeurs ont observé qu'il existe un effet d'apprentissage, permettant généralement au patient d'être pleinement opérationnel après 5 séances d'entraînement, même s'il n'avait jamais fait d'exercice de géo-localisation.

Les patients ayant une représentation significativement altérée de leur schéma corporel, et pour lesquels une utilisation fiable de l'outil ne peut être garantie, pourront être identifiés puis, soit exclus de l'analyse des résultats découlant du logiciel, soit analysés en tenant compte de leurs spécificités, qui n'excluent pas nécessairement qu'ils puissent faire des dessins de leurs sensations douloureuses et/ou paresthésiques, par exemple à l'aide d'une fonction de conversion propre à un tel patient.

Pour le reste, en fonction de la capacité de chaque patient à "géo-localiser" les points de son corps, on pourra classer les patients en plusieurs groupes, par exemple 3 groupes : « bon localisateur » si moins de 3 points touchés par le patient sont en dehors de la cible de référence, « localisateur moyen » si 3 à 4 points touchés par le patient sont en dehors de la cible de référence et « mauvais localisateur » si 5 à 6 points touchés par le patient sont en dehors de la cible de référence.

Les écarts de calibration de l'un et l'autre type peuvent être appliqués dans la tablette et/ou lors du traitement ultérieur des données.

**Travail en métrique réelle**

Les actions de la section S3 et/ou de la section S4 peuvent aussi comporter des calculs pour passer en métrique réelle, c'est-à-dire pour connaître l'équivalent en taille réelle (hauteur et largeur) en cm d'un pixel à l'écran.

Si l'on part d'une photo, on peut déterminer la taille en cm d'un pixel à l'écran à partir des réglages au moment de la prise de vues, et/ou à partir de la taille du patient, s'il s'agit d'une photo complète.

Dans le cas d'une silhouette schématique 2D (choix S21-a plus haut), les formes de la silhouette sont approximatives, et les pixels de la silhouette sont définis en unités arbitraires par rapport aux dimensions physiques réelles du patient. Il convient de convertir la taille du pixel en métrique réelle, rapportée aux dimensions physiques du patient. On peut utiliser à cet effet une distance-repère.

Dans un exemple simple, la distance-repère correspond à la distance entre les deux crêtes iliaques du patient. En effet, cette distance varie peu d'un individu à l'autre, selon sa corpulence. On peut utiliser une estimation de la distance entre les crêtes iliaques, en la corrigeant au besoin d'après la taille et/ou le poids du patient. Plus simplement, on peut utiliser la distance physique mesurée entre ses crêtes iliaques, si elle a été saisie dans les caractéristiques morphologiques du patient (sous-section S11, plus haut). La mesure de la distance repère peut être effectuée au préalable, ou bien pendant la cartographie.

Dans un autre mode de réalisation on peut aussi mesurer la taille du patient (entre la base des talons de pied et le haut de la tête) pour effectuer le calcul du passage en métrique réelle.

La conversion des unités arbitraires des pixels en $cm^2$ peut se faire à partir de deux coefficients calculés :

- un coefficient vertical égal à la taille réelle du patient divisée par le nombre de pixels représentant la taille de la silhouette,
- un coefficient horizontal égal à la distance entre les crêtes iliaques du patient divisée par le nombre de pixels représentant la distance entre les crêtes iliaques sur la silhouette. Comme le patient a pointé ses crêtes iliaques sur l'écran, l'application appCalib2 connaît la distance entre les crêtes iliaques en nombre de pixels, en X (et au besoin en Y).

On observera que, même si le pixel est carré, ses dimensions réelles (équivalents en cm) en X et en Y ne sont pas forcément les mêmes, dans le cas d'une silhouette schématique, qui n'est pas à l'échelle.

Ces deux coefficients représentent l'échelle d'un pixel en métrique réelle, par exemple en centimètres. Le produit du coefficient horizontal et du coefficient vertical donne la surface d'un pixel en centimètres carrés ($cm^2$).

La métrique réelle d'une surface dessinée sur l'écran peut alors être considérée comme égale au produit de la surface d'un pixel en centimètres carrés par le nombre de pixels que comprend la surface dessinée.

Le même genre de traitement peut être effectué sur tout ou partie des autres localisateurs définis plus haut, comme la distance entre les omoplates ou la distance entre les mastoïdes.

Un traitement statistique permet d'améliorer la précision. On peut aussi utiliser la taille et le poids du patient pour améliorer l'estimation des distances physiques entre deux repères morphologiques localisateurs.

Dans une variante, on utilise une collection de silhouettes schématiques que l'on peut mieux adapter au patient en cours.

Des gabarits spécifiques peuvent être prédéfinis en fonction du sexe et de l'IMC (indice de masse corporelle) des patients, par exemple via un logiciel de modélisation 3D «Makehuman » (open source, logiciel version CC0). Dans un mode de réalisation, six modèles présélectionnés pour chaque sexe sont disponibles pour les intervalles d'IMC suivants : <18, [18-20[, [20-22[, [22-25[, [25-30], >30. Le logiciel de cartographie adapte automatiquement le gabarit à utiliser en fonction du sexe, du poids et de la taille du patient saisis dans l'onglet « Data ». Le modèle de gabarit est choisi en fonction du sexe et de l'IMC; La taille du modèle de gabarit est adaptée à celle du patient par homothétie, afin d'occuper la totalité de l'espace dédié à l'image.

La silhouette 3D (ou 2D) peut être tirée d'une image préexistante du patient, obtenue par exemple par tomographie informatique (CT), ou encore par imagerie par résonance magnétique (MRI). Ces images sont généralement enregistrées au format de la norme dite DICOM (en anglais : *Digital Imaging and Communication in Medicine*).

Le fichier DICOM contient des données d'image du patient, ainsi que de nombreuses autres données de travail qui dépendent du mode d'imagerie. Le Demandeur a observé qu'il est possible d'extraire les données d'image tridimensionnelle du patient, à l'échelle réelle, pour former un modèle tridimensionnel du patient.

À cet effet, on peut utiliser un logiciel de la famille dite *ImageJ*, articulé sur *Java*, et disponible en mode *Open Source* auprès de différents auteurs, comme indiqué en : http://fiji.sc/ImageJ. On utilise de préférence le progiciel *Fiji*, qui contient une version d'*ImageJ*, une déclinaison de *Java* pour la supporter, ainsi que de nombreuses extensions logicielles ("*plugins*").

Dans un mode de réalisation, au lieu d'avoir un seul serveur, le serveur CCS est décomposé en un serveur de travail (CCS-T, non représenté), auquel est reliée la tablette tactile TG, et un serveur central d'imagerie (CCS-I, non représenté) qui sert comme banque de données pour tout un établissement hospitalier.

Dans ce cas, le serveur de travail CCS-T va obtenir les données DICOM pour le ou les patients considérés, depuis le serveur central d'imagerie CCS-I. Et c'est le serveur de travail CCS-T qui assure les transformations d'images et autres calculs, permettant de reconstituer l'enveloppe corporelle en 3D, en conservant les métriques réelles, et en supprimant les informations inutiles (intérieur du corps, notamment).

L'enveloppe corporelle en 3D ainsi reconstituée est un contour défini par juxtaposition de mailles triangulaires, dont chacune correspond à des mesures réelles exactes du corps. Ces mêmes triangles sont utilisés pour construire l'image affichée sur la tablette. On sait donc rapporter un triangle affiché sur la tablette à des mesures réelles, et déterminer sa surface réelle, par exemple en considérant que chaque pixel de l'écran se projette sur un ou des triangles de l'image 3D. Ces calculs peuvent être effectués dans le serveur de travail CCS-T.

L'image tridimensionnelle obtenue est stockée dans le serveur de travail CCS-T, qui en donne l'accès à la tablette graphique TG.

**Section S5 - Outil Portrait douloureux du patient (dit en anglais « *NeuroPain'T*»)**

La section S5 va gérer les actions sous forme historique, par une application *appHisto.*

Comme visible sur la figure 14 (et figure 14bis), l'application *appHisto* définit une liste ordonnée de consultations du patient, prédéfinies par exemple comme :

$M_{-1}$, $M_0$, $M_{+1}$, $M_{+2}$, $M_{+3}$, $M_{+6}$, $M_{+12}$, $M_{+24}$, où
$M_0$ est une séance de référence, typiquement une séance per-opératoire le jour de l'implantation d'un neurostimulateur,
$M_{-1}$ est une consultation préalable,
$M_{+1}$, $M_{+2}$, $M_{+3}$, $M_{+6}$, $M_{+12}$, $M_{+24}$ sont des consultations de suivi.

L'échelonnement prédéfini peut être en jours, où $M_{+1}$ est la séance 1 jour après l'implantation, $M_{+2}$ est la séance 2 jours après l'implantation, etc... et $M_{+24}$ est la séance 24 jours après l'implantation.

L'échelonnement prédéfini est le plus souvent en mois où $M_{+1}$ est la séance 1 mois après l'implantation, $M_{+2}$ est la séance 2 mois après l'implantation, etc... et $M_{+24}$ est la séance 24 mois après l'implantation (avec une période de tolérance de $\pm 2$ à 5 jours).

D'autres consultations peuvent être ajoutées à cet échelonnement. Chaque consultation est associée à un dossier qui va contenir tout ou partie des dessins, données, mesures et calculs obtenus lors de cette consultation.

Après sélection d'une consultation (ou création d'une nouvelle consultation), une touche de lancement "Go" permet de lancer les dessins de cartographie proprement dits.

Par ces dessins de cartographie, le patient va dépeindre ou "portraiter" sa douleur. Il va même, en fait, "peindre" sa douleur, par des couleurs et/ou d'autres attributs graphiques.

**Fonctions de dessin**

La tablette graphique TG tactile permet au patient et/ou au clinicien de réaliser une caractérisation par dessin d'une zone de douleur sur la représentation bidimensionnelle schématique standard (S21-a), sur une des représentations bidimensionnelles pré-calibrée (S21-b), sur la représentation tirée d'une photo prise en temps réel (S21-c) ou encore sur la représentation tridimensionnelle (S21-d). La représentation choisie est appelée génériquement silhouette. On considère une silhouette vue de dos, pour des douleurs dorsales.

On entend par « caractérisation par dessin d'une zone de douleur » la définition donnée par le patient du ressenti de douleur. Pour cela, le patient et le clinicien ont à leur disposition, différentes fonctions logées dans une application graphique appGraph utilisée pour décrire précisément la zone de douleur.

Chaque fonction peut être sélectionnée tactilement par un bouton/icône sur la tablette graphique TG. Parmi ces fonctions, on peut trouver notamment :

- une fonction de délimitation pour démarquer une zone de douleur (par exemple en l'encerclant avec un doigt sur la représentation) ;
- une fonction de choix de marqueur pour varier le mode de marquage des pixels (par exemple un marqueur peut marquer des pixels adjacents : outil pinceau ; ou des pixels espacés : outil spray) ;
- une fonction de taille de marqueur pour faire varier le nombre de pixel par unité de résolution lors d'un marquage ou démarquage (par exemple la sensibilité tactile peut être variée en fonction de la taille du doigt) ;
- une fonction de coloration ;
- une fonction de gommage pour corriger une manipulation faussement réalisée ;

- une fonction de variation de taille de gomme pour faire varier le nombre de pixel par unité de résolution lors d'une correction ;
- une fonction d'annulation pour annuler une action et rétablir un état initial, par exemple pour une correction d'une sélection faussement marquée ;
- une fonction de répétition pour réitérer une action effectuée ;
- une fonction de réinitialisation (en anglais *reset*) pour revenir dans un état de base et de ce fait annuler toute action exécutée.

Chaque fonction est généralement utilisée distinctement. Toutefois, chaque résultat obtenu par la manipulation tactile d'une fonction donnée peut se superposer avec un ou plusieurs autre(s) résultat(s) obtenu(s) par des manipulations tactiles effectuées avec une autre fonction.

Pour la capture de cartographie de douleurs d'un patient, il est demandé au patient de délimiter tactilement sur la tablette la ou les douleurs, par exemple dorsales, qu'il ressent au quotidien. Il pourra même saisir un état des lieux des douleurs de manière itérative, à différents moments de la journée ("nycthémère"), pour renseigner sur le caractère éventuellement positionnel (dépendant de la position du patient) des douleurs, et de leurs surfaces.

La cartographie proposée permet d'évaluer à différents moments de la journée les douleurs, notamment « positionnelles ». Accroupi, le patient peut ressentir une douleur mécanique du dos, alors que couché, il ressent une douleur neuropathique de la jambe droite.

Quant aux surfaces, on comprend que le ventre n'a pas la même surface quand un individu est allongé sur le dos ou bien penché en avant. Donc les surfaces de douleur peuvent être appréciées différemment en fonction de la posture du patient.

À cet effet, l'application graphique appGraph est complétée par une application de sélection appSelec qui établit une correspondance entre des attributs graphiques (ou "indicateurs") et des qualifications de douleurs. Selon un mode de réalisation, l'application de sélection sur l'écran tactile met à disposition plusieurs jeux d'attributs graphiques, qui comprennent :

- un jeu d'attributs graphiques relatifs à l'intensité de la douleur ;
- un jeu d'attributs graphiques relatifs au type de douleur; et
- un jeu d'attributs graphiques relatifs à une zone faisant l'objet d'une thérapie à visée antalgique, comme l'implantation d'une stimulation médullaire (et donc, le cas échéant : des paresthésies).

Par attribut graphique, on entend notamment une couleur et/ou une texture, y compris des hachures. Dans la suite de la présente description il est fait référence à un mode de réalisation utilisant différentes couleurs pour caractériser les douleurs. Pour des raisons de forme, les couleurs peuvent être représentées sur des dessins par des pointillés, hachures ou autres signes graphiques distinctifs. Les couleurs indiquées dans les tableaux ci-après sont données à titre d'exemples.

Il est maintenant fait référence à la figure 15 (et à la figure 15bis). À droite de la figure 15 (et figure 15bis) il est représenté une colonne comportant des fonctions graphiques de l'application appGraph représentées par des pictogrammes, de haut en bas :

- Zoom ;
- Sauvegarder/Enregistrer;
- Rotation du tronc à 180° pour passer d'une vue antérieure à une vue postérieure et vice versa ;
- Effacer l'ensemble des informations de douleur saisies (global reset) ;
- Revenir une étape en arrière (undo) ;
- Gomme large;
- Gomme fine;
- Pinceau large ;
- Pinceau fin ;
- Pinceau très fin.

En bas, la figure 15 (et figure 15bis) comprend un bandeau horizontal. Sa partie haute comporte trois des quatre onglets : "Intensité", "Typologie", "Paresthésies" et "Résumé", qui font partie de l'application appSelec. L'onglet "Paresthésies" est ici non activé, invisible, ou supprimé, comme on le verra plus loin.

On note enfin que, sur la figure 15 (et figure 15bis), c'est l'onglet "Intensité" qui est activé.

**Intensité de douleur**

L'intensité de la douleur peut être définie à l'écran par des touches marquées chacune par sa couleur et un texte indiquant un niveau de douleur. L'exemple de la figure 15 (et figure 15bis) utilise quatre niveaux de douleur (DL1 à DL4), associés à quatre couleurs différentes, selon le tableau suivant TS51.

| Tableau TS51 - Niveau de douleur | | | |
|---|---|---|---|
| Niveau | Nom anglais | Nom français | Couleur |
| DL1 | Low | Faible | Bleu clair |
| DL2 | Medium | Moyenne | Bleu moyen |
| DL3 | Intense | Intense | Orange |
| DL4 | Very intense | Très intense | Rouge |

Sur la figure 15, la douleur de niveau faible LOW est représentée par une zone de pointillés espacés d'une distance D ; la douleur de niveau moyen MED. est représentée par une zone de pointillés espacés d'une distance d (où d<D) ; la douleur de niveau intense INT. est représentée par une zone de pointillés espacés d'une distance L (où L<d) ; et la douleur de niveau très intense V.INT. est représentée par une zone de pointillés espacés d'une distance I (où I<L).

Sur la figure 15bis, la douleur de niveau faible LOW est représentée par une zone de couleur bleu clair; la douleur de niveau moyen MED. est représentée par une zone de couleur bleu moyen ; la douleur de niveau intense INT. est représentée par une zone de couleur orange ; et la douleur de niveau très intense V.INT. est représentée par une zone de couleur rouge.

À chaque niveau de douleur peut être associée la surface calculée correspondante, ainsi qu'un pourcentage de cette surface calculée par rapport à la surface douloureuse totale. Ces variables sont calculées en temps réel. C'est illustré au bas de la figure 15 (et figure 15bis), en pointillés correspondants (ou en couleur correspondante pour la figure 15bis). Le bouton "Data" laisse le choix à l'hétéro-observateur de les afficher ou pas, afin de ne pas influencer le patient pendant la saisie.

Pour la clarté de la présente description, le niveau de douleur est rappelé près de la silhouette, en regard de chaque zone pointillée/colorée.

Les commandes de dessin sont contraintes par des règles, comme :

- la surface dessinée ne peut pas déborder de la silhouette,
- les niveaux de douleur sont exclusifs,
- le dernier niveau de douleur dessiné l'emporte.

On peut y ajouter des règles optionnelles, comme : la gomme n'efface que la couleur/texture en cours.

Sur la figure 16 (et figure 16bis), les fonctions graphiques à droite de l'écran sont construites à l'identique de la figure 15 (et figure 15bis). En bas, c'est maintenant l'onglet "Type" qui est activé dans l'application appSelec.

**Types de douleur**

L'indicateur relatif au type de douleur peut être défini par sélection entre quatre types de douleurs, à chacun desquels est associée une couleur correspondante, par exemple selon le tableau suivant TS52.

| Tableau TS52 - Type de douleur | | |
|---|---|---|
| Nom anglais | Nom français | Couleur |
| Nociceptive pain | Douleur par excès de Nociception | Rose |
| Trigger pain | "Trigger" ("Gâchette") | Violet |
| Neuropathic pain | Douleur Neuropathique | Jaune |
| Mechanical pain | Douleur Mécanique | < néant> |

Dans ce qui suit, l'expression "Douleur par excès de Nociception" pourra être abrégée en "douleur nociceptive".

Seules les zones possédant une intensité de douleur peuvent être typées. C'est-à-dire que l'on ne peut donner un type qu'à une zone présentant un niveau de douleur. On remarque donc que les surfaces marquées de la figure 16 (et figure 16bis) sont comprises dans celles de la figure 15 (et figure 15bis). De plus, les types ne sont pas exclusifs, en ce sens qu'on peut avoir plusieurs types de douleur dans une même zone de douleur.

En partie basse de la figure 16 (et figure 16bis), il n'y a que trois touches de commande, qui correspondent aux trois premiers types du tableau TS52.

À chaque type de douleur peut être associé un pourcentage de la surface calculée correspondante, par rapport à la surface douloureuse totale. C'est illustré au bas de la figure 16 (et figure 16bis), dans la couleur correspondante. Le pourcentage de douleur mécanique est obtenu par addition des pourcentages "nociceptif" et "trigger", à l'arrondi près.

Sur la figure 16, la douleur par excès de nociception (en anglais « nociceptive pain »), notée "NOCI." est représentée par une zone de pointillés espacés ; la douleur de type « trigger » ou « gâchette » (en anglais «trigger pain »), notée "TRIG." est représentée par une zone de croix espacées; et la douleur neuropathique (en anglais « neuropathic pain »), notée "NEURO." est représentée par une zone de tridents espacés.

Sur la figure 16bis, la douleur par excès de nociception (en anglais « nociceptive pain ») NOCI. est représentée par une zone de couleur rose ; la douleur de type « trigger » ou « gâchette » (en anglais « trigger pain ») TRIG. est représentée par une zone de couleur violet ; et la douleur neuropathique (en anglais « neuropathic pain ») NEURO. est représentée par une zone de couleur jaune.

La touche "View all" de la figure 16 (et figure 16bis) sert à visualiser les intensités douloureuses superposées aux différents types de douleurs.

On fera maintenant référence à la figure 17 (et figure 17bis). Dans celle-ci, les fonctions graphiques à droite de l'écran sont construites comme sur les figures 15 et 16.

En ce qui concerne les onglets du bandeau horizontal (application appSelec), ils pourraient être les mêmes sur les figures 15, 16 et 17 (et figures 15bis, 16bis et 17bis), à savoir "Intensité", "Typologie", "Paresthésies" et "Résumé".

Le Demandeur préfère que les sections S5 et S6 soient singularisées, c'est-à-dire que :

- la section S5 est spécialisée sur le dessin et l'évaluation des douleurs pré-traitement ou post-traitement, qu'il s'agisse de neurostimulation, ou non. L'onglet "Paresthésies" est donc désactivé, invisible, ou supprimé dans les écrans de la section S5, cf. figures 15 et 16 (et figures 15bis et 16bis).
- la Section S6 est dédiée à la neurostimulation, et comporte les quatre onglets "Intensité", "Typologie", "Paresthésies" et "Résumé", comme le montre la figure 17 (et figure 17bis).

**Section S6 - Outil Cartographie des paresthésies générées par la neurostimulation implantée à visée antalgique (en anglais « *Neuro-Mapping Locator*»)**

Similairement à la section S5, la section S6 va gérer les actions sous forme historique, par l'application appHisto.

Comme précédemment (figure 14 et figure 14bis), l'application appHisto définit une liste ordonnée de consultations du patient, prédéfinies par exemple comme :

$M_{-1}$, $M_0$ (séance per-opératoire le jour de l'implantation), $M_{+1}$, $M_{+2}$, $M_{+3}$, $M_{+6}$, $M_{+12}$, $M_{+24}$ (visites de suivi)

Après sélection d'une consultation (ou création d'une nouvelle consultation), une touche de lancement "Go" permet de lancer les dessins de cartographie proprement dits.

En bas de la figure 17 (et figure 17bis), c'est l'onglet "Paresthésies" qui est activé dans l'application appSelec.

Sur la figure 17, la paresthésie "PARES." générée sur le patient par le neurostimulateur est représentée par une zone hachurée. Cette zone hachurée se superpose aux zones de douleurs de la figure 15.

Sur la figure 17bis, la paresthésie "PARES." généré sur le patient par le neurostimulateur est représenté par une zone de couleur verte. Cette zone de couleur verte se superpose aux zones de douleurs de la figure 15bis.

Par ses dessins de cartographie, le patient va alors dépeindre ou "portraiter" les paresthésies ressenties, résultant de la stimulation médullaire. Le patient va alors à l'identique de ses douleurs, "peindre" ses paresthésies représentées en couleur verte et/ou à l'aide d'autres attributs graphiques.

**Paresthésies**

La neurostimulation se traduit par des sensations physiques particulières, que l'on peut qualifier de « picotements/fourmillements agréables ou bienfaisants ». La cartographie peut aussi porter sur ces sensations, qui sont de type paresthésique.

L'indicateur relatif aux paresthésies prévoit la différenciation entre différents programmes de stimulation ou même différents types de stimulations implantées chez un même individu. Ainsi, on différencie une stimulation médullaire implantée en regard de la moelle épinière (cf. supra) (SCS en Anglais pour *Spinal Cord Stimulation*) et une stimulation nerveuse périphérique, implantée en région sous-cutanée, en regard de la zone douloureuse, par exemple lombaire, intercostale, faciale ou occipitale (Sub-Q Stim ou PfNS ou encore PNS, de l'anglais *Peripheral (field) Nerve Stimulation*). Là aussi, des couleurs différentes peuvent être utilisées, comme indiqué dans le tableau TS53.

| Tableau TS53 - Type de paresthésies | | |
|---|---|---|
| Nom anglais | Nom français | Couleur |
| SCS Spinal Cord Stimulation ou Program 1 | SME Stimulation Médullaire Epidurale, ou Programme 1 | Vert clair |
| PNS Peripheral Nerve Stimulation ou Program 2 | SNP Stimulation Nerveuse Périphérique ou Programme 2 | Vert foncé |

Il est possible d'opérer en hybride, à savoir en utilisant les différents types de stimulateur simultanément ou successivement. Le patient dessine à chaque fois la surface cutanée sur laquelle il ressent les picotements caractéristiques de tel ou tel type de neurostimulation, mise en fonction.

La figure 17 illustre un exemple.

Dans le mode réalisation de la figure 17 (et figure 17bis), le type de paresthésies n'est pas indiqué par une couleur sur la silhouette affichée par la tablette graphique TG. Il est spécifié lorsque l'on sauvegarde les autres données de l'image de la figure 17 (et figure 17bis).

En résumé, si l'on compare les rôles des sections S5 et S6 :

- La section S5 constitue une analyse du ressenti du patient au niveau des douleurs,

- La section S6 passe à une analyse du ressenti du patient par rapport à une thérapie délivrée, ici une stimulation médullaire implantée, notamment lorsque l'on fait varier les paresthésies générées par la stimulation implantée. La variation des paresthésies générées par la stimulation implantée peut-être réalisée, soit le jour de l'implantation d'un stimulateur lors de la programmation initiale en temps réel au bloc opératoire, soit au cours du suivi longitudinal dans le temps.

Comme la section S5, cette section S6 se base sur un historique relatif au patient. L'écran d'accueil de la section S6 est donc une liste ordonnée de visites du patient, comme pour la figure 14 (et figure 14bis). Cet écran n'est pas représenté à nouveau.

Il y a plusieurs façons de modifier le traitement du patient (paresthésies), et notamment :

- modifier la configuration d'excitation neuronale du neuro-stimulateur; il s'agit de modifier le programme de stimulation, en faisant varier, soit la configuration électrique d'anode(s) et de cathode(s), soit l'amplitude/Intensité de stimulation, soit la durée d'impulsion de l'onde de stimulation, soit la fréquence de stimulation, soit la répartition de densité de courant sur les différents contacts si la génération du courant est multisources, soit une combinaison de ces paramètres ; ou
- modifier la position de l'électrode dans l'axe cranio-caudal ou encore latéralement, lors de son implantation.

On rappellera maintenant les techniques de neurostimulation implantée, à visée antalgique.

**Techniques évoluées de neurostimulation implantée à visée antalgique**

Dans la technique de stimulation médullaire proprement dite, on agit au niveau de la moelle épinière. L'abréviation usuelle est SCS pour "*Spinal Cord Stimulation*". Puissante, cette technique peut-être utilisée pour soulager des patients souffrant de douleurs neuropathiques sévères. Selon cette technique, un générateur programmable émet des impulsions électriques en direction des fibres nerveuses de la moelle épinière d'un patient, par l'intermédiaire d'au moins une électrode multi contacts. On agit directement sur le système nerveux central.

L'électrode de stimulation peut être monocolonne (au moins deux contacts), et l'on peut l'implanter par voie percutanée. Elle peut être multicolonnes (au moins deux colonnes de contacts), auquel cas on la pose en principe par voie chirurgicale, l'électrode ayant la forme d'une plaque, plus large que les électrodes monocolonnes, qui sont cylindriques et filaires.

Le positionnement optimal de l'électrode de stimulation se situe au niveau d'une certaine région de la moelle épinière dans le canal vertébral, au niveau de laquelle sont centralisées les projections afférentes d'une zone douloureuse cutanée périphérique (par exemple, une région douloureuse du membre inférieur se projette au niveau de la région médullaire, située au niveau des vertèbres thoraciques T9 à T11).

On connaît aussi la technique de stimulation nerveuse sous-cutanée, qui agit sur le système nerveux périphérique. L'abréviation usuelle est PNS pour "*Peripheral Nerve Stimulation*". Cela peut être considéré comme une stimulation médullaire indirecte, dans la mesure où la stimulation nerveuse sous-cutanée remonte par les fibres nerveuses périphériques jusqu'à la partie de la moelle épinière qui est associée à la zone périphérique excitée par la stimulation. Dans ce cas, l'(les) électrode(s) implantée(s) dans les tissus sous-cutanés est (sont) généralement mono-colonne (s) (au moins deux contacts), bien que l'usage d'une électrode multi-colonnes ne soit pas formellement exclu. L'électrode est implantée en regard de la zone douloureuse.

Dans tous les cas, l'électrode est reliée à un neurostimulateur implanté dans les tissus sous-cutanés de la région abdominale ou de la fesse. Un boîtier externe de commande associé, permet d'interroger le dispositif implanté par radiofréquence et d'activer sélectivement ses programmes de stimulation, mettant en jeu les plots électriques (ou contacts) de l'électrode, afin de générer un champ électrique contrôlé, en tension, en intensité et en portée dans l'espace. Ce champ électrique stimule une population neuronale, ce qui provoque un effet physique dit "paresthésies", susceptible d'entraîner un soulagement de la douleur. Cet effet physique est souvent ressenti par le patient comme un picotement ou fourmillement, bienfaisant et agréable dès lors qu'il se substitue à la sensation douloureuse.

Jusqu'à présent, le médecin devait recourir à son intuition et à son expérience pour le choix des électrodes et du neurostimulateur à implanter.

Or le choix de la ou des électrodes (et des neurostimulateurs qui leur sont associés) est crucial. En effet les résultats sont variables en fonction de l'outil thérapeutique utilisé (électrodes plus ou moins sophistiquées ; électrodes percutanées ou chirurgicales, électrodes conventionnelles ou de nouvelle génération, électrodes mono colonnes ou multi colonnes, électrodes de surface de couverture plus ou moins limitée, etc).

En effet, les électrodes médullaires directes (SCS) sont souvent efficaces lorsque l'indication est rigoureusement posée par un implanteur expert, eu égard au fait qu'elles agissent directement sur la moelle épinière et donc le système nerveux central. Mais, dans certains cas au moins, elles ne sont pas très sélectives. En effet, la moelle épinière loge des faisceaux de fibres nerveuses, affectées à différentes régions du corps, et que l'on stimule ensemble. Par exemple, une électrode SCS implantée pour stimuler un côté du bas du dos va généralement exciter aussi la population neuronale médullaire, responsable de l'innervation du membre inférieur du même côté, ce qui va produire des paresthésies inutiles, sur un territoire non douloureux, puisque la douleur, dans cet exemple précis, n'évolue qu'aux dépends de la composante axiale de l'individu, soit le niveau lombaire bas et non au niveau des membres.

À l'inverse, les électrodes de stimulation indirecte (PNS) peuvent être moins efficaces dans certains cas, et n'agissent qu'indirectement sur la moelle épinière. Par contre, elles sont plus sélectives. En effet, parmi les différents faisceaux de fibres nerveuses que la moelle épinière loge, sont seules stimulées celles qui correspondent à la zone où est implantée l'électrode PNS. Il semble qu'il existe aussi un phénomène d'accoutumance (ou tolérance) pour une électrode PNS, dont la stimulation perd de son efficacité avec le temps.

Le choix de l'électrode est donc particulièrement délicat. Il l'est encore plus si l'on considère d'une part la variété de modèles d'électrodes qui sont proposés, d'autre part la variété de configurations de stimulation que permet chaque modèle. Le programmateur permet en effet de nombreuses combinaisons d'activation des plots ou contacts de l'électrode, avec par exemple des effets angulaires et de focalisation du champ électrique généré. À titre d'exemple, on peut noter qu'en ne tenant compte que du nombre de combinaisons possibles, indépendamment de toute notion d'amplitude, de durée d'impulsion, de fréquence de stimulation, etc., les possibilités de choix de contacts sur une électrode multicolonnes à 16 plots dépassent 49 millions de combinaisons possibles, pour une électrode donnée, implantée à un niveau donnée, chez un individu donné, pour une indication donnée.

Pour plus de détails, on se référera aux articles :

- "Transverse tripolar spinal cord stimulation: theoritical performance of a dual channel system", Struijk JJ. and Holsheimer, J. Med Biol Eng Comput, 34(4):273-279, 1996;
- "Spinal cord stimulation versus repeated lumbosacral spine surgery for chronic pain: a randomized, controlled trial". North RB et al., Neurosurgery, 56:98-106, 2005.
- "Spinal cord stimulation versus conventional medical management for neuropathic pain: a multicentre randomized controlled trial in patients with failed back surgery syndrome", Kumar K., et al., Pain, 132:179-88, 2007.
- "The effects of spinal cord stimulation in neuropathic pain are sustained: a 24-month follow-up of the prospective randomized controlled multicenter trial of the effectiveness of spinal cord stimulation", Kumar K., et al., Neurosurgery, 63:762-70, 2008.
- "Back Pain, A Real Target for Spinal Cord Stimulation", P. Rigoard et al., Neurosurgery 70:574-585, 2012.

À l'aide des fonctions décrites sous la section S5, le choix de l'outil thérapeutique est effectué en fonction de paramètres mesurés de quantification de la douleur, tels que la taille de la surface ou encore l'emplacement de la zone douloureuse. En effet, l'évaluation de ces paramètres est une étape clef permettant de choisir convenablement la ou les électrodes à implanter. C'est un premier progrès.

La section S6 permet d'aller plus loin, en proposant une comparaison dynamique de la zone douloureuse et de la zone de recouvrement paresthésique, générée par la stimulation implantée.

La définition d'un ou plusieurs dispositifs de neurostimulation implantée conformément au tableau TS14 peut comprendre :

- un identifiant de l'électrode et du stimulateur qui définit complètement le nombre et la distribution de ses plots/contacts,
- les paramètres de stimulation (amplitude/intensité/durée d'impulsion, fréquence de stimulation),
- les caractéristiques du générateur (mono ou multi-sources etc...),
- le nombre, le type et la position d'implantation de(s) l'électrode(s) de stimulation dans le corps et en général le canal rachidien du patient (stimulation médullaire),
- la configuration de stimulation des plots/contacts du stimulateur,
- les caractéristiques anatomiques et électriques du site d'implantation et de la cible neuronale (dimensions du canal rachidien, distance de l'électrode par rapport à la moelle, implantation des racines, épaisseur de la couche de liquide céphalo-rachidien s'interposant entre l'électrode et le tissu neural, forme du canal, conductivité des tissus nerveux adjacents, etc...).

On peut alors enregistrer historiquement, en fonction des traitements, l'évolution de la zone douloureuse, et l'évolution de la relation entre la zone paresthésique et la zone douloureuse, comme on le verra plus loin.

**Action sur la programmation de l'électrode de stimulation implantée**

La cartographie permet de comparer la zone de couverture (en $cm^2$) des paresthésies générées par le stimulateur par rapport à la zone de douleur (en $cm^2$). Cela permet de calculer le recouvrement des paresthésies sur la zone de douleur. Ainsi, le clinicien peut réorganiser le champ électrique du stimulateur, en principe pour augmenter le recouvrement, en modifiant la programmation de l'électrode.

De plus, la cartographie permet d'adapter la spécificité des paresthésies. En effet, il arrive que les paresthésies couvrent à la fois une zone de douleur et une zone saine. Ainsi, bien qu'un recouvrement paresthésiant puisse être satisfaisant (par rapport à la zone douloureuse cible), la spécificité des paresthésies peut ne pas l'être. Cette situation se présente notamment lorsque les paresthésies couvrent la totalité d'une zone de douleur mais qu'en parallèle elle couvre également une trop importante zone saine. Dans ces conditions la spécificité des paresthésies n'est généralement pas satisfaisante. Le clinicien pourra au moyen de la cartographie réorganiser le champ électrique du stimulateur pour en augmenter la spécificité.

Ceci peut être précisé en considérant des groupes de patients, sur lesquels peuvent être conduites des analyses statistiques évoluées : coefficient de corrélation, distribution, études des variables quantitatives en fonction du temps, comparaison de moyennes, analyse de la variance (dite "ANOVA"), notamment. On peut utiliser par exemple le logiciel SAS v 9.3, disponible notamment auprès de SAS Institute Inc., 100 SAS Campus Drive, Cary, NC 27513-2414, USA. Ce logiciel fournit des procédures (telles que PROC MEAN et autres), pour analyser statistiquement des variables, ainsi que leur "normalité" (conformité à la loi de Gauss).

**Action sur le positionnement du stimulateur**

Les mesures de recouvrement/spécificité des paresthésies sont très utiles lorsqu'elles sont effectuées pendant une opération d'implantation de l'électrode chez un patient. En effet, le système nerveux (central ou périphérique) étant extrêmement complexe, il est souvent difficile de localiser l'emplacement idéal pour positionner une électrode. La tablette graphique TG permet d'interroger de manière quantitative et fiabilisée un patient pendant l'implantation. Pour cela le patient est localement anesthésié mais se trouve en état éveillé (ou réveillé), ce qui lui permet de coopérer avec l'équipe médicale pour délimiter ses paresthésies, qu'il s'agisse d'une électrode implantée par voie chirurgicale ou par voie percutanée.

Lors de l'opération le patient peut indiquer en temps réel sur la tablette graphique TG les paresthésies ressenties du fait de l'implant posé. Selon la mesure de spécificité à partir de la cartographie réalisée par le patient, la pertinence de l'emplacement de l'électrode peut être évaluée. Le cas échéant, l'électrode peut être repositionnée en temps réel, avec un retour direct du patient, confirmant l'optimisation de la couverture paresthésiante de sa zone douloureuse.

Ceci permet de rechercher la position de l'électrode qui est efficace, ou la plus efficace, au vu d'une ou plusieurs cartographies paresthésiques dessinées par le patient.

Ainsi, le positionnement de l'électrode peut, en mode per-opératoire, être ajusté en fonction d'une interaction en temps réel entre le patient et le dispositif de l'invention (ici la tablette), en fonction des comparaisons entre les surfaces des paresthésies et des zones douloureuses. A chaque fois, le chirurgien en recevra une représentation visuelle très parlante, qui lui facilite la décision du positionnement et/ou de la programmation de l'électrode. On peut alors considérer qu'il s'agit d'une sorte de "Stéthoscope Médullaire Électronique", qui permet d'abord une interaction avec le patient, mais surtout de mettre en rapport un balayage électrique ("screening") des fibres des cordons postérieurs (configurations électriques de stimulation, et position de l'électrode) et ce qui en résulte quant au ressenti du patient, au niveau des douleurs comme de la paresthésie.

Selon un mode de réalisation, l'écran de la tablette graphique TG peut comporter un double affichage, avec symétrie par rapport à un axe central. Un demi-écran permet au patient de dépeindre ou "peindre" sa douleur. L'autre demi-écran permet au clinicien d'observer les manipulations réalisées par le patient lors de l'opération, et les calculs de recouvrement/spécificité de paresthésies (notamment) qui s'ensuivent.

En variante, la tablette graphique TG ne sert qu'au patient. L'affichage réservé au clinicien s'effectue alors sur un écran distinct d'un autre ordinateur ou d'une autre tablette graphique.

**Fiche synthétique (ou en anglais « Summary »)**

Une synthèse qui affiche l'ensemble des données récoltées auprès du patient peut être affichée sur l'écran tactile dans les onglets "Summary" des écrans S5 et/ou S6.

Pour l'écran S5, l'affichage peut comprendre notamment la surface par intensité de douleur, la surface par type de douleur et la surface douloureuse globale. Ces données peuvent être simplement rassemblées en liste sous forme de texte.

Pour permettre au clinicien une appréciation visuelle à la fois rapide et complète, la fiche synthétique est de préférence exprimée sous forme graphique, par exemple comme décrit plus loin ("paramètres de douleur Y").

Pour la fiche synthétique (« Summary ») de l'écran S6, l'affichage peut comprendre notamment la surface par intensité de douleur, la surface par type de douleur et la surface douloureuse globale, à quoi s'ajoutent, le cas échéant, les données de surface pour les paresthésies générées par le dispositif implanté, avec les ratios définis plus haut.

Là aussi, ces données peuvent être simplement rassemblées en liste sous forme de texte, ou mieux exprimée sous forme graphique, par exemple comme décrit plus loin ("paramètres de traitement X").

Une touche à l'écran peut permettre de basculer entre l'affichage texte et l'affichage graphique, qui est l'affichage par défaut.

**Avantages**

En résumé, la caractérisation par dessin d'une zone de douleur aboutit à une cartographie de douleur propre à un patient donné. La cartographie peut-être dimensionnée directement en $cm^2$ ou encore en pourcentage de surface corporelle d'un patient (mesure en $cm^2$ de la zone de douleur par rapport à la surface corporelle totale d'un patient exprimée en $cm^2$). Il s'agit donc d'une cartographie nettement plus sophistiquée que les solutions proposées dans l'art qui permet notamment de réaliser des calculs statistiques qui ont pour base des mesures en taille réelle de patients.

Lorsque la cartographie de douleur est établie, celle-ci peut être directement sauvegardée sur la base de données centrale CCS_DB, en regard de l'identifiant de visite en cours, défini sur l'écran de la figure 14 (et figure 14bis).

En d'autres termes, une cartographie de la douleur d'un patient est représentée métriquement sur l'écran de la tablette graphique TG au niveau de l'onglet S5. Cette cartographie peut-être enregistrée dans la tablette TG, puis être transférée dans la fiche patient de l'ordinateur CCS (CCS-T et/ou CCS-I).

La cartographie peut-être en 3D, comme on l'a vu.

Ainsi, la caractérisation par dessin d'une zone de douleur ou de paresthésies peut-être réitérée lors de chaque consultation du patient chez le clinicien. De cette manière, un suivi de l'évolution de la douleur et de la couverture douloureuse est assuré. Il s'ensuit que chaque cartographie de douleur et/ou de paresthésies réalisée lors d'une consultation est sauvegardée sur la base de données centrale CCS_DB. La comparaison et l'évolution dans le temps de différentes cartographies offrent notamment la possibilité au clinicien d'analyser et de mieux comprendre la douleur ressentie par le patient. Le clinicien peut donc adapter le traitement du patient en fonction des cartographies réalisées.

La cartographie proposée est nettement plus évoluée qu'une échelle visuelle analogique classique, avec laquelle il est difficile d'analyser et de comprendre les différentes composantes de la douleur sur des bases fiables.

La cartographie des douleurs selon la section S5 (NeuroPain'T) peut se faire au chevet du patient, en consultation ou même en ambulatoire, sous la forme d'une auto-évaluation à distance. D'autres cartographies peuvent se faire en mode per-opératoire, c'est-à-dire en salle d'opération durant l'implantation de l'électrode de stimulation, comme on l'a vu dans la section S6, correspondant au "Neuro-mapping Locator".

## Variantes de réalisation

De manière générale, l'outil graphique de l'invention comporte préférentiellement un écran tactile qui permet une utilisation intuitive pour le patient, comme on l'a vu. Toutefois, d'autres types d'organes de pointage sur un écran graphique ne sont pas exclus, au moins dans certains cas.

La tablette graphique est normalement visible par le patient et par le clinicien. Elle est contrôlée par le clinicien. Pour les écrans de toucher par le patient, pour calibration et cartographie, le clinicien donne le contrôle de la tablette au patient.

Dans certaines situations, comme on l'a vu, il peut être souhaité que le patient et le clinicien ne voient pas la même chose. Suivant le degré de confidentialité désiré, cela peut se faire en divisant l'écran de la tablette graphique en deux moitiés symétriques, ou bien en prévoyant dans la tablette un contrôle d'accès qui réserve au clinicien ce que lui seul doit connaître, ou encore en utilisant un écran et/ou un ordinateur séparé pour le clinicien. Dans ce dernier cas, l'affichage séparé peut être géré par la tablette graphique TG, directement ou bien en interaction avec l'ordinateur central CCS (ou l'un d'entre eux, ou un autre ordinateur), par le réseau.

Le transfert d'information peut se faire notamment de l'une des manières suivantes :

1- Les données sont créées sur la tablette, et, dans le cas de l'existence d'une borne wifi (ou autre connexion réseau), l'utilisateur peut envoyer ses données en temps réel sur le serveur de travail,

2- En l'absence de connexion, la tablette enregistre en local les données et les transmet par la suite, quand une connectivité (par exemple wifi) redevient disponible. Le volume de données peut alors être limité par l'espace disque disponible de la tablette.

3- Une autre solution en cas de non connectivité est d'utiliser localement un PC interconnecté à la tablette, qui reçoit les données de la tablette avant de les envoyer lui-même au serveur de travail.

Par ailleurs, la tablette graphique TG peut devoir recourir au serveur de travail CCS-T pour exécuter des traitements de calcul lourds, comme on l'a vu.

En outre, les opérations effectuées par la tablette graphique TG (au moins lorsqu'elles sont validées) sont systématiquement stockées dans la fiche patient globale du serveur central CCS et/ou du serveur de travail CCS-T.

Sur un autre plan, l'invention peut aussi s'appliquer avec des traitements de la douleur autres que la neurostimulation. Par exemple, l'application de topiques locaux comme la capsaïcine, provoque une sensation cutanée de brûlure, avec rougeur, ce qui permet au patient de délimiter la zone traitée, telle qu'il la ressent.

## Applications évoluées

L'application graphique calcule la surface stimulée Spar. Celle-ci est comparée à une surface douloureuse correspondante Sdol, par exemple la surface douloureuse globale ou plus spécifiquement de type Neuropathique. Tout ou partie des calculs suivants peuvent être effectués :

| Variable | Expression(s) | Commentaire |
|---|---|---|
| Scom | Spar ∩ Sdol | Surface de la partie commune à la surface stimulée Spar et à la surface douloureuse Sdol |
| Stot | Spar ∪ Sdol (Spar + Sdol) - Scom | Surface totale de la réunion de la surface stimulée Spar avec la surface douloureuse Sdol, sans compter deux fois la surface commune Scom |

Différents ratios peuvent être calculés, de préférence comme suit.

Le premier ratio est un coefficient de performance Cperf:

$$Cperf = (Spar \cap Sdol) / Sdol = Scom / Sdol.$$

Ce coefficient Cperf peut être vu comme le ratio de couverture paresthésiante par rapport à la douleur. Il ne prend pas en compte le fait que Spar peut être en dehors d'une douleur. Il calcule donc une paresthésie efficace.

Le second ratio est un coefficient de Sélectivité (Csel) :

$$Csel = Scom / Spar$$

Il exprime le ratio de paresthésies utiles sur la surface totale des paresthésies.

Le coefficient de sélectivité Csel peut être vu comme une prise en compte de ce que l'on appelle parfois le « spreading électrique » (ou étalement électrique) de la stimulation. Cette notion est assez mal appréhendée aujourd'hui. Selon le Demandeur, ce spreading découle d'une diffusion ou dispersion de la stimulation électrique dans le corps humain. Une partie du spreading ou étalement atteint effectivement des aires douloureuses. C'est un spreading adapté ("matching"). Csel en représente la proportion.

Le reste, en proportion (1 - Csel), va vers des aires non douloureuses ; c'est un spreading inadapté ("non matching"), donc inutile, voire nuisible au confort du patient. Le Demandeur considère que c'est un indicateur pertinent en second niveau, une fois que l'exigence de performance apparaît satisfaite. Ce point sera développé ci-après. L'inverse de Csel peut être vu comme un coefficient de dispersion, Cdisp =Spar/Scom.

Ces applications évoluées utilisent des "paramètres de traitement" de la forme X* (leur première lettre est X), ainsi que des "paramètres de douleur" de la forme Y* (leur première lettre est Y).

Les paramètres de douleur (Y) concernent la ou les douleurs, et sont calculés à partir de mesures de surface faites dans la section S5. Un exemple de paramètres de douleur est donné dans le tableau TS71 ci-dessous.

| Tableau TS71- Paramètres Y | |
|---|---|
| Paramètre | Signification |
| $Y_S$ | $Y_S$ est la diminution d'une surface douloureuse d'une visite à une autre |
| $Y_I$ | $Y_I$ est la diminution d'intensité douloureuse sur une surface donnée d'une visite à une autre |
| $Y_{PC}$ | changement de typologie ou de caractérisation de la douleur (« pain characterization ») sur une surface donnée d'une visite à une autre |

## Paramètres de traitement ; stimulation par électrodes

Les paramètres de traitement (X) visent à représenter l'efficacité de la stratégie antalgique appliquée, par exemple dans le cas d'un dispositif de stimulation tel qu'une électrode implantée. Ils sont déterminés à partir de mesures de la section S5 et S6 pour un patient. Le tableau TS72 ci-dessous en présente un exemple, dans le cas des électrodes de stimulation médullaire.

| TS72 - Paramètres de traitement X | | |
|---|---|---|
| Paramètre | Expression | Signification |
| $X_P$ | Cperf | Paramètre dit « d'efficacité électrode », tels que l'efficacité de recouvrement paresthésique des douleurs, correspondant à la performance du dispositif, ou en anglais « Device Performance » |
| $X_S$ | Csel | Paramètre de sélectivité de la stimulation, correspondant à la spécificité du dispositif, ou en anglais « Device Specificity » |
| $X_{P3}$ | Spar(t2)/Spar(t1) | Paramètre de persistance, correspondant à l'évolution de la surface paresthésique entre les instants t1 et t2. C'est l'évolution de la perception de la stimulation électrique par le patient dans le temps, donc une évaluation du phénomène de tolérance |

L'ensemble de ces paramètres est calculé et rendu disponible instantanément, à un temps donné, grâce au traitement des données en temps réel effectué par l'application de calculs du logiciel. Le résultat peut être représenté sous plusieurs formes visuelles, pour en permettre une lecture rapide mais précise par l'opérateur qui pose l'implant sur le patient (par exemple un neurochirurgien, également dénommé clinicien implanteur).

## Exemple particulier

On considère un patient donné. Entre les visites $M_{-1}$ et $M_0$, ce patient est muni d'une électrode donnée, en une position donnée, et avec une configuration d'excitation donnée.

Les données brutes de surfaces dépeintes par le patient, en fonction des niveaux et types de douleur, ainsi que le taux de couverture "Paresthésies/douleur $M_{-1}$" sont donnés dans le tableau TS80 ci-après.

| Tableau TS80 | | | | | |
|---|---|---|---|---|---|
| | Visite M$_{-1}$ | Visite M$_0$-post-op | | | Visite M$_1$ |
| Niveau douleur | douleur | Paresthésies | Paresthésies/ douleur M$_{-1}$ | douleur restante | Paresth. |
| | Cm$^2$ | Cm$^2$ | % | Cm$^2$ | |
| DL1 | 248 | 242 | 98% | 0 | |
| DL2 | 244 | 242 | 99% | 100 | |
| DL3 | 63 | 15 | 24% | 40 | |
| DL4 | 63 | 9 | 14% | 0 | |
| Sdol Scom Sout | 618 | - 508 247 | | 140 | |
| Spar | | 755 | | | 689 |
| Stot | = Sdol + Sout | 865 | | | |
| Type douleur | | | | | |
| Noci. | 122 | | | 0 | |
| Trigg. | 126 | | | 0 | |
| Neuropath | 370 | | | 140 | |
| Total | 618 | | | 140 | |

Dans cet exemple :

- le paramètre Xp = Cperf = Scom/Sdol vaut 0.82
- le paramètre XS = Csel = Scom/Spar vaut 0.67
- le paramètre X$_{P3}$ considéré entre la visite M1 (la surface totale de paresthésies Spar est 689 cm$^2$), et M0 (Spar = 755 cm$^2$), s'établit à 0.91 de persistance. Cela signifie que la paresthésie en M0 a perduré presque totalement à la visite M1.

**Coefficient de performance pondéré**

On a considéré plus haut le coefficient de performance Cperf, défini comme :

$$X_P = Cperf = Scom/ Sdol.$$

Le Demandeur a observé qu'à performance et spécificité identiques, un patient ne sera probablement pas satisfait d'un recouvrement paresthésiant qui ne prend pas en compte le maximum de ses douleurs les plus intenses.

C'est pourquoi l'on définit aussi un coefficient de performance pondéré X$_{PW}$, pour lequel on détermine séparément le recouvrement quantitatif paresthésiant pour chaque zone d'intensité de douleur. A chaque fois, le recouvrement est pondéré par un coefficient qui dépend de la zone d'intensité, par exemple :

- coefficient w = 4 pour les douleurs très intenses,
- coefficient z= 2 pour les douleurs intenses,
- coefficient y = 1 pour les douleurs moyennes/modérées et
- coefficient x= 0,5 pour les douleurs faibles.

De même, le Demandeur a observé qu'à performance et spécificité identiques, un patient ne sera peut-être pas satisfait d'un recouvrement paresthésiant qui ne prend pas en compte son ressenti selon les types de douleurs. Par exemple, on donne le poids 2 aux douleurs "trigger", et le poids 1 aux douleurs nociceptives et neuropathiques.

**Index directement exploitables; représentation graphique préférentielle**

Dans ce qui suit, on utilisera des paramètres dits de Manoé, définis par le tableau TS83 ci-après.

| Tableau TS83 | | |
|---|---|---|
| **Paramètres de Manoé** | **Valeurs possibles** | **Descriptions** |
| **Coef Perf** | **[0-1]** | Correspond à la capacité d'un dispositif à générer des paresthésies sur un territoire douloureux initial.<br>CP = (Sd∩Sp)/Sd |
| **Coef Perf Pondéré** | **[0-1]** | Correspond à la capacité d'un dispositif à générer des paresthésies pondérées par l'intensité du territoire douloureux couvert.<br>CPP = [x*(Sp∩Sd Low)+y*(Sp∩Sd Medium)+z*(Sp∩Sd Intense)+w*(Sp∩Sd Very Intense)] / [x*(Sd Low)+y*(Sd Medium)+z*(Sd Intense)+w*(Sd Very Intense)] |
| **Coef Sélectivité** | **[0-1]** | Correspond à la tendance d'un dispositif à générer des paresthésies ciblées sur le territoire douloureux initial.<br>CSel= (Sd∩Sp)/Sp |

Dans ce tableau, Sd_Low, Sd_Medium, Sd_Intense et Sd_Very Intense désignent respectivement les surfaces de douleur pour chaque intensité de douleur, dans l'ordre croissant.

On utilise maintenant trois indicateurs correspondant respectivement à la comparaison de trois paramètres à trois seuils respectifs, par exemple selon le tableau TS85 suivant.

| Tableau TS85 | | | | | |
|---|---|---|---|---|---|
| Paramètre | Exemple | Seuil | Conclusion | Symbole | Couleur |
| CPerf | 0,83 | 0,7 | Satisfaisant | (+) | Vert |
| CPerf Pondéré | 0,57 | 0,7 | Insatisfaisant | (-) | Rouge |
| CSel | 0,71 | 0,77 | Insatisfaisant | (-) | Rouge |

Cet ensemble est dénommé ici "R index", et représenté sous cette forme sur la figure 18 par des voyants en partie basse, en rapport avec des diagrammes sur lesquels on reviendra.

La conception du "R index" est fondée d'abord sur les considérations médicales et pratiques suivantes, au vu des propriétés actuelles des électrodes :

(a) un seuil minimum de couverture de l'ensemble des zones douloureuses fixé à 70% (et donc un coefficient de performance, Cperf, de 0.7) est considéré comme acceptable, pour parler de « couverture satisfaisante » du patient.

(b) En revanche, et ceci représente toute la justification de l'utilisation de coefficients pondérés, comme évoqué ci-dessus, un patient couvert à 95% de ses douleurs, mais pour lequel il manquera la couverture de la petite région la plus douloureuse estimée à 5% de la surface douloureuse totale (le gros orteil par exemple), ne se sentira pas soulagé. Malgré une performance globale de couverture (X1) acceptable, la stimulation risque d'être perçue comme un échec.

On considérera donc d'un point de vue pratique qu'un seuil minimum de couverture de la zone la plus douloureuse (coefficient de performance pondéré à l'intensité douloureuse) fixé également à 70%, est considéré comme acceptable, pour parler de couverture « satisfaisante », pondérée à l'intensité douloureuse.

Pour donner des indicateurs simples, en temps réel à l'opérateur, des voyants verts ou rouges (assortis du symbole associé, "+" ou "-") correspondant à chaque paramètre du R index (Cperf, Cperfpondéré, Csel), sont situés à droite de chaque score et définis en fonction des seuils préétablis ci-dessus pour la performance et ci-après pour la sélectivité.

En d'autres termes :

- un coefficient de performance globale supérieur à 0.7 (voyant vert) et un coefficient de performance pondéré inférieur à 0.7 (voyant rouge), constituent un échec relatif de la stimulation et impliquent un changement de programme de stimulation, un repositionnement de l'électrode ou un changement d'électrode, en fonction des paramètres anatomiques du patient et des paramètres techniques du dispositif.

- un coefficient de performance globale inférieur à 0.7 (voyant rouge) et un coefficient de performance pondéré supérieur à 0.7 (vert), constituent un succès de la stimulation sur la zone la plus douloureuse mais avec une couverture restant insuffisante de l'ensemble de la plainte douloureuse. On parlera de succès relatif de couverture mais à adapter secondairement aux attentes du patient, en l'état, et on recommandera néanmoins un changement ou ajout de programme de stimulation voire un repositionnement de l'électrode.
- un coefficient de performance globale supérieur à 0.7 (voyant vert) et un coefficient de performance pondéré supérieur à 0.7 (voyant vert), constituent le scénario idéal et permettent de conclure à un succès de couverture. On recommandera donc de s'abstenir de tout changement de programme de stimulation, repositionnement de l'électrode ou changement d'électrode.

On rappelera les points suivants :

- plus la sélectivité de couverture sel se rapprochera de 100%, plus les chances de satisfaction du patient seront importantes, même à coefficients de performances (brute et pondérée) considérés comme « acceptables », c'est à dire supérieurs à 0.7.
- On parle ici de succès de « couverture » (paramètres traitements) et non d'efficacité antalgique de la technique (paramètres douleur). En effet, une zone de paresthésie correspond au ressenti du traitement par le patient ; il n'est pas certain que cette zone de paresthésie corresponde exactement à une zone d'effet antalgique.
- Un des intérêts potentiels de cette analyse de paramètres est qu'elle ouvre la voie à l'établissement d'une corrélation entre les paramètres traitement et les paramètres douleur.

Une fois l'étude de la performance réalisée, par les paramètres Cperf et Cperfpondéré, le troisième paramètre du R index peut être pris en considération. Il s'agit de la sélectivité de la

stimulation, étudiée par son coefficient de sélectivité Csel (on pourrait en variante utiliser un coefficient de dispersion).

La zone de dispersion est représentée sur le diagramme de droite de la figure 18 par une zone hachurée de bandes jaunes et noires. Son aire sur le diagramme est définie par le rapport entre l'aire de la surface de dispersion Sout, et l'aire totale de la surface des paresthésies Spar, rapport qui est appliqué à l'aire de la surface du rectangle de fond qui correspond à la surface totale des paresthésies générées.

Au vu des propriétés actuelles des électrodes, on s'appuiera sur les considérations médicales et pratiques suivantes :

- un seuil minimum de sélectivité de la stimulation, fixé à 0.77 est considéré comme acceptable, pour parler de « couverture sélective » du patient.
- Un coefficient de sélectivité inférieur à 0.77, sans être rédhibitoire, indiquera que la stimulation sera grevée d'inconforts pour le patient, liés à une couverture non adaptée et non désirée ("non matching spreading") de la stimulation sur des territoires non douloureux. Dans la mesure du possible, on recommandera de changer de programme de stimulation pour être plus sélectif, à performances égales voire de modifier la position de l'électrode, en fonction des paramètres anatomiques du patient et des paramètres techniques du dispositif, voire de changer de dispositif, si cette dispersion n'est pas tolérée par le patient.

On précisera les points suivants :

- Les notions de sélectivité et de dispersion recouvrent la même entité, à savoir la capacité d'un dispositif à générer le plus de paresthésies utiles (« matching spreading »), ce qui correspond à la sélectivité, ou bien, exprimée de manière différente, la capacité d'un dispositif à générer le moins de paresthésies inutiles (« non matching spreading »), ce qui correspond à la dispersion.
- Le paramètre utilisé ici pour la représentation du Diagramme de Manoé est la sélectivité.

## Importance du R index et de ses voyants

Le **R index** constitue un outil d'aide à la décision quasi immédiat, en particulier en mode per-opératoire, lorsqu'on est en train d'implanter une électrode sur un patient. Les différentes situations sont illustrées dans le tableau TS86 ci-dessous. On l'illustrera sur l'exemple des voyants et diagrammes de la figure 18.

| Tableau TS86 | | |
|---|---|---|
| **Voyants** | **Conclusion** | **Recommandation** |
| Vert vert vert (+++) | Parfait. Performant et sélectif | On ne change rien |
| Vert vert rouge (++-) | bonne performance, sélectivité insuffisante ou dispersion trop importante | changement de programme, si insuffisant, déplacement de l'électrode. |
| Vert rouge vert (+-+) | améliorer la couverture du territoire le plus douloureux | changement de programme, si insuffisant déplacement de l'électrode, si insuffisant et si possible : changement d'électrode. |
| Vert rouge rouge (+--) | améliorer la couverture du territoire le plus douloureux et la spécificité de couverture | changement de programme, si insuffisant déplacement de l'électrode, si insuffisant et si possible : changement d'électrode. |
| Rouge vert vert (-++) | performance pondérée bonne mais pas globale. Bonne sélectivité. | changement ou ajout de programme |
| Rouge rouge vert (--+) | sélectivité bonne la performance n'est pas là | changement de programme, si insuffisant déplacement de l'électrode, si insuffisant et si possible : changement d'électrode. |
| Rouge rouge rouge (---) | Rien ne va | changement de programme, si insuffisant déplacement de l'électrode, si insuffisant et si possible : changement d'électrode. |

Dans ce tableau, l'expression "changement de programme" signifie que la position de l'électrode dans le corps du patient n'est pas modifiée ; par contre, on change la configuration de stimulation électrique des différents contacts de l'électrode.

Ce "R index" est donc immédiatement parlant pour l'implanteur (anesthésiste ou chirurgien). C'est essentiel compte-tenu notamment des nombreux sujets sur lesquels l'implanteur doit rester concentré.

La représentation peut utiliser aussi un diagramme de recouvrement paresthésiant, relatif aux intensités douloureuses sur une zone douloureuse donnée, prenant en compte la pondération.

Comme illustré sur la figure 18 en partie gauche, ce peut être une forme particulière de graphique formée de rectangles juxtaposés dans un grand rectangle ou "socle", de taille standardisée. Chaque rectangle intérieur correspond à une intensité de douleur : Bleu clair, Bleu moyen, Orange et Rouge, dans l'exemple donné plus haut. La surface de chaque rectangle est définie par le poids attaché à l'intensité de douleur qu'il représente. Comme on le voit, les superficies des surfaces Bleu clair, Bleu moyen, Orange et Rouge sont dans des rapports 0.5, 1, 2 et 4.

Les surfaces de recouvrement paresthésiant sont illustrées en vert, par des rectangles inclus dans chaque rectangle d'intensité de douleur, par exemple dans un coin de celui-ci.

En partie droite, un autre diagramme illustre par un rectangle vert la surface de paresthésies utiles Scom, avec autour une équerre zébrée en jaune et gris, qui correspond à la surface Sout.

Le Demandeur a choisi de donner à la représentation de la figure 18 le nom de "diagramme de Manoé".

La combinaison du "R index" et de diagrammes constitue un outil d'aide à la décision particulièrement puissant. Le diagramme de Manoé est une représentation graphique remarquablement efficace. D'autres représentations graphiques sont envisageables.

**INDEX F et Index RFG**

Ce que l'on appelle ici index F est semblable à une intégrale dans le temps. C'est un enregistrement historique, en fonction des traitements, de l'évolution de la zone douloureuse, et de l'évolution de la relation entre la zone paresthésique et la zone douloureuse.

L'Index RFG est plus complet sur le plan clinique, et intéresse particulièrement le chercheur clinicien et le scientifique, en ce qu'il permet d'établir pour la première fois des relations entre les paramètres techniques du traitement et l'efficacité clinique de la thérapie antalgique (paramètres cliniques de douleur), avec suivi dans le temps.

Dans la suite, on utilisera ce qu'on appelle les paramètres RFG. Pour la représentation, ils sont définis en pourcentages, conformément au tableau TS87 ci-après.

Dans ce tableau, Sd désigne une surface de douleur, et Sp une surface de paresthésies ; t1 et t2 désignent deux instants de visite, par exemple les visites M0 et M1.

Pour une visite donnée, on part des pourcentages d'intensité des douleurs résiduelles par rapport à la douleur initiale, pour chaque niveau d'intensité :

$$\%\text{Low} \quad \%\text{Medium} \quad \%\text{Intense} \quad \%\text{Very Intense}$$

Ou, selon une notation équivalente

$$\text{DL1\%} \quad \text{DL2\%} \quad \text{DL3\%} \quad \text{DL4\%}$$

La grandeur Score_Int est la somme de ces pourcentages d'intensité, pondérés par le poids associé au niveau d'intensité concerné (x, y, z et w), l'ensemble divisé par w.

Par exemple x, y, z et w sont 0.5, 1, 2,4, comme précédemment.

YI est alors la diminution de ce score d'intensité entre la visite t1 et la visite t2.

| Tableau TS87 | | |
|---|---|---|
| **Paramètres RFG** | **Valeurs retenues** | **Description** |
| **XP = Cperf** (Performance) | [0%-100%] | Représente la capacité d'un dispositif à générer des paresthésies sur un territoire douloureux initial. $\text{Cperf} = (Sd \cap Sp)/Sd \times 100$ |
| **XS = Csel** (Sélectivité) | [0%-100%] | Représente la capacité d'un dispositif à générer des paresthésies spécifiquement sur le territoire douloureux initial. $\text{Csel} = (Sp \cap Sd)/Sp \times 100$ |
| **XP3** (Persistance de la perception des paresthésies) | [0%-100%] plafonné à 100% si $\geq$ 100% | Représente le maintien entre 2 visites (t1, t2) de l'ensemble des paresthésies générées par un dispositif, entre 2 visites (t1, t2) $\text{XP3} = Sp_{(t2)}/Sp_{(t1)} \times 100$ |
| **YS** (Diminution de la surface) | [0%-100%] Plancher à 0% si $\leq$ 0% | Représente la diminution en % des surfaces de douleurs résiduelles par rapport aux douleurs initiales, entre 2 visites (t1, t2) $\text{YS} = [Sd_{(t1)} - Sd_{(t2)}]/Sd_{(t1)} \times 100$ |
| **YI** (Diminution intensité) | [0%-100%] Plancher à 0% si $\leq$ 0% | Représente la diminution du score de % d'intensité pondéré par le niveau d'intensité (x, y, z et w) des douleurs résiduelles ((x*%Low + y*%Medium + z*%Intense +w*%Very Intense)/w) par rapport à la douleur initiale $\text{YI} = [\text{Score Int}_{(t1)} - \text{Score Int}_{(t2)}]/\text{Score Int}_{(t1)} \times 100$ |
| **YPC** (Changement de typologie) | [0%-100%] | Représente la variation de la caractéristique prédominante en % Neuropathique/Mécanique des douleurs entre 2 visites. $\text{YPC} = |\%\text{Neuropathique}_{(t1)} - \%\text{Neuropathique}_{(t2)}|$ |

**Variantes de représentations graphiques**

Les paramètres utilisés ici peuvent également être illustrés sous forme de diagrammes polygonaux multi-axiaux, également dénommés "diagrammes en étoile". Dans la suite, on aura généralement six paramètres. On obtiendra donc à chaque fois un diagramme hexagonal multi-axial.

La figure 19 est un tel diagramme hexagonal multi-axial, construit à partir des paramètres en X et Y des tableaux TS71 et TS72. Cette figure 19 illustre l'évaluation d'une thérapie (SCS) à 3 mois de l'implantation.

Il s'agit d'une représentation graphique hexagonale de l'index RFG chez un patient souffrant de lombo-radiculalgies réfractaires et implanté d'une stimulation médullaire multicolonnes, à 3 mois de suivi postopératoire.

En ce qui concerne les paramètres « traitement », on observe tout d'abord une performance de couverture de la surface douloureuse très satisfaisante pour cette électrode implantée (Xp = 82%). En revanche, la sélectivité de l'électrode reste très moyenne puisque à peine plus de la moitié des paresthésies générées est utile (Xs = 57%), ce qui signifie que 43% des paresthésies générées sont hors de la région douloureuse. Il n'existe pratiquement aucun effet de tolérance puisque la stabilité de la couverture dans le temps avoisine 100% (XP3=92%).

En ce qui concerne les paramètres cliniques du patient (« douleurs »), le soulagement paraît très appréciable avec une diminution des surfaces douloureuses de 77% (YS) s'accompagnant d'une diminution des intensités de douleur de 75% (YI). Cet effet antalgique permet une régression de la composante neuropathique des douleurs (YPC) de 86%, composante prédominante jusque là.

La figure 20 est une vue en perspective qui enchaîne quatre diagrammes hexagonaux multi-axiaux, considérés en M0, M0 + 1 mois, M0 + 3 mois et M0 + 6 mois, pour un patient et une électrode donnée. M0 est une visite per-opératoire, et n'a donc que les axes XP et XS.

Cela constitue un exemple de l'index F, et aussi de l'index RFG.

Ce diagramme montre une stabilité de la performance (XP) dans le temps alors que la sélectivité (XS) a une discrète tendance à augmenter. La stabilité de la couverture paresthésiante est bonne dans le temps (XP3).

En ce qui concerne les paramètres de douleur, une diminution progressive des surfaces douloureuses est caractérisée par une augmentation du YS qui passe de 0 à 30% à 1 mois post-implantation puis à 70% à 6 mois post-implantation. L'augmentation quasi immédiate en post-opératoire, et sub-optimale, du YI témoigne très probablement d'une bonne couverture, d'emblée, des régions douloureuses les plus intenses. On s'attendrait ainsi à ce que le coefficient de performance pondéré de l'électrode chez ce patient, et donc le R index, non représentés sur ce diagramme, soient élevés.

La typologie des douleurs change drastiquement dès le premier mois post-implantation avec une diminution de 95% du YPC, le quel caractérisait une composante prédominante mécanique des douleurs chez ce patient.

La figure 21 est un diagramme hexagonal multi-axial, qui compare deux programmes de stimulation, pour un même patient et une même électrode.

Il s'agit d'une représentation comparative de 2 index RFG correspondant à 2 programmes différents (P1 et P2), essayés chez un même patient. Le patient était préalablement porteur du programme 1, dont la sélectivité n'était pas satisfaisante malgré une bonne efficacité antalgique globale.

La construction de ce diagramme intervient une semaine après la mise en place du programme P2 (en vert), ce qui explique qu'on ne dispose pas encore du paramètre XP3 pour le programme 2, faute de recul.

On observe que les programmes 1 et 2 ont une performance de couverture à peu près comparable. En revanche la sélectivité du programme 2 est nettement supérieure au programme 1.

Les paramètres de douleur sont encourageants avec une diminution des surfaces douloureuses à peu près comparable (Score YS légèrement inférieur pour le programme 2). Il en est de même pour l'intensité douloureuse (85% pour le programme 1 vs 78% pour le programme 2).

Il est intéressant de noter que la diminution de la composante neuropathique des douleurs est en faveur du programme 2 (YPC = 85% vs 70%). Comme expliqué ci-dessus, il n'est en revanche pas possible d'apprécier un éventuel phénomène de tolérance aux paresthésies générées par le programme 2 tout juste mis en place.

La figure 22 comporte en périphérie cinq diagrammes construits comme celui de la figure 21, pour cinq programmes différents P1 à P5, illustrés par des couleurs différentes. Au centre, les contours associés aux cinq programmes sont superposés dans un seul diagramme hexagonal multi-axial.

Chaque programme a été essayé pendant une semaine, ce qui a permis à l'implanteur d'isoler deux programmes extrêmement performants (P2 et P4), en termes de couverture mais d'efficacité malheureusement fugace (paramètre XP3). Ces programmes ont dû être abandonnés pour la sélection finale.

Le programme P5 était grevé du même problème de tolérance et surtout d'un manque de sélectivité ne permettant pas d'observer un changement de typologie des douleurs (YPC).

Le programme P3 aux performances techniques moyennes ne s'accompagnait malheureusement d'aucune diminution significative des intensités douloureuses.

C'est donc au final le programme P1 (en violet) qui a été retenu pour la programmation définitive de ce patient.

La neurostimulation du système nerveux central comprend notamment la neurostimulation médullaire, mais aussi la neurostimulation corticale. Ce qui est décrit ici s'applique à tous les types de neurostimulation, qu'il y ait ou non paresthésie.

## Paramètres de traitement X ; cas autres que la neurostimulation

La section S5 montre comment le patient délimite sa ou ses douleurs. Elle peut servir dans des situations autres que le traitement par neurostimulation. On peut citer les deux exemples suivants, à titre non limitatif :

- une chirurgie rachidienne telle qu'une exérèse de hernie discale (paramètres de traitement dans le tableau TS93)
- la prise en charge de douleurs cervicales irradiant vers le membre supérieur par une mécanothérapie de type Mc Kenzie (paramètres de traitement dans le tableau TS94).

Dans ces cas, les paramètres de traitement ne sont pas définis par des paresthésies, mais par d'autres grandeurs techniques liées au traitement.

| Tableau TS93 - Paramètres de traitement $X_n$ - Hernie discale | | | |
|---|---|---|---|
| Paramètre | Définition | Mise en oeuvre | Mesure |
| $X_1$ | Qualité du résultat | décompression disco-radiculaire | Examen per-opératoire de la racine décomprimée (souplesse, oedème, couleur) |
| $X_2$ | Volume herniaire enlevé | IRM ou Scanner pré/post-opératoire | Evaluation volumétrique du vide laissé |
| $X_3(1)$ | Evolution discale dans le temps | IRM post-opératoire | Evolution de l'état inflammatoire péri-discal (signaux IRM "MODIC") |
| $X_3(2)$ | Evolution discale dans le temps | Scanner | Evolution de l'hydratation du disque (classification de PFIRMANN) |
| $X_3(3)$ | Evolution discale dans le temps | Radiographies standards | Evolution de la hauteur discale |
| $X_3(4)$ | Evolution discale dans le temps | Radiographies dynamiques | Evolution de la stabilité discale dans le plan sagittal |

Un ou plusieurs des paramètres $X_3(1)$, $X_3(2)$, $X_3(3)$ et $X_3(4)$ du tableau TS93 peuvent être utilisés.

| Tableau TS94 - Paramètres de traitement $X_n$ - Mécanothérapie Mc Kenzie | |
|---|---|
| Paramètre | Mise en oeuvre |
| $X_1$ | Evaluation initiale des douleurs en fonction de positions répétées et imposées au patient ⇨ Préférence positionnelle/directionnelle |
| $X_2$ | Rémanence de l'effet antalgique après une séance |
| $X_3$ | Nombre de séances requis pour obtenir une efficacité durable |

Ainsi, il devient possible de comparer un traitement par neurostimulation médullaire à un autre traitement non électrique, comme la chirurgie rachidienne.

**Traitements statistiques multi-patients**

Pour chaque patient, la tablette graphique TG et l'ordinateur CCS vont permettre de nourrir une base de données « Neuro-Data-Base », en bref Neuro-DB, avec :

- des données personnelles cliniques sur le patient;
- une représentation du patient en silhouette, avec une conversion en taille réelle des zones de douleurs et de paresthésies, ressenties par le patient ;
- la réalisation d'une cartographie avec dessin de zones de douleur, définies en intensité et type de la douleur;
- la réalisation d'une cartographie avec dessin de zones de paresthésies ;
- la conversion de ces cartographies sur base métrique, en dimensions réelles ;
- le suivi historique de ces cartographies,
- le même suivi historique en fonction du positionnement de l'électrode implantée chez le patient,
- le même suivi historique en fonction du programme de stimulation utilisé chez le patient.

Cette Neuro-DB peut être logée dans la base de données d'ensemble CCS_DB.
Les index R, F et RFG, ainsi que leurs évolutions pour chaque patient vont être calculés à chaque actualisation de la base de données Neuro-DB. Des études statistiques peuvent notamment être conduites sur des groupes étendus de patients, en utilisant des méthodes statistiques de stratification de classes, méthodes dites « de Cluster », ou encore des méthodes computationnelles de type dit "Data Mining", afin d'isoler des prédicteurs de réponses au sein des différentes sous-populations.

Les données d'évaluations quantitatives douleurs/paresthésies sur les différents patients sont directement comparables, puisqu'elles sont exprimées en métrique réelle.

## Applications en pratique clinique et pour le processus décisionnel de stratégie thérapeutique

Du suivi historique, on peut répondre rapidement par une analyse statistique à une question comme : en présence d'une configuration donnée de dessin de zones de douleur, définies en intensités et types de la douleur; quels ont été les traitements statistiquement les plus efficaces, en termes de choix du (des) dispositif(s) implanté(s), du positionnement de l'électrode et de sa programmation ? Les données récoltées fournissent la réponse.

En compilant ce type d'analyses statistiques, on peut définir des traitements préconisés, en fonction de chaque configuration rencontrée d'intensités et de types de douleurs.

En utilisant l'invention comme outil source de saisie d'informations quantitatives, la Neuro-DB qui y est adossée comme outil de stockage évolutif et en appliquant les concepts développés ci-dessus au traitement des données sur une population large, il devient possible de préconiser directement, en partie au moins et pour un patient donné :

- le type d'électrode à implanter,
- sa position d'implantation, et
- sa configuration de programmation.

L'invention peut être considérée comme un double outil de mesure et d'évaluation comparative des douleurs et des éventuels dispositifs implantés à visée antalgique, chez un individu, permettant, via un traitement de l'information à grande échelle grâce à la Neuro-DB qui leur est adossée, d'identifier puis d'extraire des prédicteurs de réponse à la thérapie, sur une sous-population cible, pour guider les choix thérapeutiques de demain en matière de neurostimulation.

Chaque dispositif implantable, pour une indication donnée, sur une population cible donnée, pourra hériter d'un Index RFG global. Cet index RFG global est comparable d'un dispositif à l'autre.

Ainsi, chaque patient présentant une zone de douleur particulière pourra se voir attribuer de façon préférentielle le dispositif le plus adapté aux prédicteurs de réponse identifiés pour cet individu.

Ce qui précède intéresse le plan clinique du traitement des patients.

Les comparaisons entre modèles d'électrodes (implantation et programmes) peuvent se faire sur de nombreux patients. Cela ouvre la voie à des tests et/ou bancs d'essais d'un grand intérêt pour toute la communauté des professionnels de la santé, depuis les chercheurs cliniciens jusqu'aux fabricants d'électrodes, en passant par les autorités de santé, comme la Haute Autorité de Santé en France.

## SYNTHÈSE DES AVANTAGES ET SOLUTIONS APPORTÉES PAR L'INVENTION

Comme on vient de l'illustrer, les applications découlant de l'utilisation de l'invention, avec une métrique fiable, sont multiples, nouvelles et d'implications majeures pour le patient. On peut ainsi :

- Représenter sous forme instantanée et synthétique la performance d'une thérapie délivrée, à l'aide de paramètres techniques quantitatifs liés à la thérapie délivrée. Cette évaluation est permise par l'Index R et la représentation graphique des paramètres dits de « Manoé ».
- Déterminer si cette «efficacité technique», instantanée et initiale, s'accompagne d'un effet antalgique significatif de la thérapie délivrée à un instant t (Figure 19) et d'apprécier s'il est durable dans le temps (Figure 20), ce qui en constitue la finalité. Cette projection temporelle est permise par l'évolution longitudinale de l'index RFG et sur le plan purement technique, par l'intégration de la performance pure du dispositif dans le temps, matérialisée par l'index F.
- En matière de stimulation médullaire plus particulièrement, modifier l'emplacement et/ou la programmation d'une électrode tout juste implantée, patient en conditions de chirurgie éveillée, en utilisant la fonction de balayage des fibres médullaires cibles (fonction de stéthoscope médullaire électronique), par l'appréciation de l'évolution de la performance et de la sélectivité instantanées, liée à un changement de configuration électrique du dispositif ou à une modification per-opératoire de son positionnement au sein du canal rachidien.
- Modifier la programmation de ce même dispositif au cours du suivi longitudinal du patient dans le temps, en appréciant l'évolution des paramètres Traitement et Douleur, sur des index RFG itératifs et comparatifs (Figures 21 et 22), en fonction de l'utilisation de tel ou tel programme.

In extenso, en utilisant ces fonctionnalités non plus seulement à l'échelon individuel mais en les projetant à l'échelon de masse grâce au traitement statistique multi-patients, l'outil logiciel proposé permet les applications suivantes :

∘ Les dispositifs les plus récents et sophistiqués comportent une électrode à 16, 20 ou 32 contacts. Pour 16 contacts, on a un choix de configurations électriques de plus de 49 millions de combinaisons, à paramètres de stimulation constants. Il en découle des difficultés de choix de programmation, ce qui peut rendre leur utilisation très contraignante pour l'opérateur, voire aléatoire pour le patient : on pourrait déterminer, pour chaque dispositif et chaque programme, le sous-groupe de population susceptible d'en tirer le meilleur bénéfice et de fait, pour un patient donné, raffiner les algorithmes de programmation jusqu'à des perspectives de programmation automatisée. La finalité de l'invention est bien d'extraire un certain nombre de prédicteurs de réponse à tel ou tel programme par l'analyse secondaire des données. Ceci constitue un des intérêts majeurs de la Neuro-DB, couplée à ce logiciel.

∘ Toujours dans le contexte de la stimulation médullaire, l'espoir de couvertures de plus en plus étendues et sélectives, de soulagements optimisés par les nouvelles générations d'électrodes, devrait permettre aux cliniciens d'en élargir potentiellement les indications. Néanmoins, la variabilité des résultats globaux de ces techniques publiés à ce jour illustre à quel point la sélection des patients, le choix du type de neurostimulation et celui qui concerne le type de matériel, semblent être des paramètres tout aussi décisifs que les paramètres d'implantation et de programmation des électrodes. Enfin, la notion de réponse à long terme de ces différentes techniques, dans le cadre de douleurs chroniques, n'a jamais été évaluée. Les enjeux médico-économiques qui en résultent, imposent une rationalisation des indications et, là encore, conditionneront le choix du matériel.

C'est donc bien le couplage des outils interactifs proposés à un traitement statistique multi-patients, permis par l'utilisation de la neuro-DB, qui apparait pertinent et prometteur. Dans ce champ d'applications, celui-ci a pour but d'identifier de manière prospective, au sein des populations cibles de candidats à une neurostimulation implantée, des « phénotypes chirurgicaux » de répondeurs/non répondeurs/répondeurs partiels, à telle ou telle technique de neurostimulation, afin :

- de comparer les différents outils de neurostimulation. Ceci permettra de guider le choix de ces outils, de hiérarchiser les utilisations, et de mettre à disposition les données prospectives aux autorités de tutelle, en charge du remboursement de ces dispositifs.
- d'isoler des prédicteurs de réponse à la technique, par modélisation computationnelle.
- de redéfinir, à terme, les indications et le choix de ces techniques.

A supposer qu'une diffusion du support interactif sus-décrit soit effective dans un réseau clinique d'envergure, l'évaluation rationnelle des différents systèmes de neurostimulation implantable permise par ce procédé inventif aura un impact direct sur les systèmes de soins.

Certaines caractéristiques de l'outil proposé peuvent être définies généralement comme suit:

- dans le second mode (S6), l'application locale (TGapps) est agencée pour permettre la délimitation de zones de paresthésies sélectivement selon qu'il s'agit d'une électrode de neurostimulation du système nerveux central ou d'une électrode de neurostimulation du système nerveux périphérique.
- l'application locale (TGapps) est agencée pour évaluer des premières données de surface (Sdol) relatives à au moins une zone de douleur et des deuxièmes données de surface (Spar) relatives à au moins une zone de paresthésies, tandis que l'application pilote (TGappmain) est agencée pour comparer ces données de surface, ainsi que pour calculer un taux de couverture paresthésiante et un taux de spécificité de la couverture paresthésiante, ce qui permet d'adapter la couverture paresthésiante en améliorant la spécificité.
- l'application locale (TGapps) est agencée pour déterminer, entre les résultats de deux visites, un coefficient de performance brute, un coefficient de performance pondérée, en fonction de la relation entre une zone de traitement et des zones d'intensité de douleur, et un coefficient de spécificité/dispersion, en fonction de la relation entre la zone de traitement et certaines au moins des zones d'intensité de douleur, l'ensemble de ces coefficients (R index), fournissant un puissant indicateur de l'adéquation d'un traitement.
- les coefficients ci-dessus sont représentés graphiquement par des voyants incluant une comparaison à des seuils respectifs, accompagné d'au moins un diagramme de rectangles qui représente d'une part les relations entre les zones d'intensité de douleur et la partie de la zone de traitement qui les affecte respectivement, et d'autre part la partie de la zone de traitement qui n'affecte pas certaines au moins des zones d'intensité de douleur (Figure 18).
- l'application locale (TGapps) est agencée pour déterminer, entre les résultats de deux visites, au moins trois coefficients (YS, YI, YPC), représentant respectivement la diminution de la surface de douleur, la diminution de l'intensité de douleur, et le changement de typologie de la douleur, et au moins trois coefficients (XP, XS, XP3), représentant respectivement la performance du dispositif de traitement, la sélectivité du dispositif de traitement, et la persistance des effets du dispositif de traitement, l'ensemble de ces coefficients (index RFG), fournissant un autre puissant indicateur de l'adéquation d'un traitement.

- les coefficients ci-dessus sont représentés graphiquement sous la forme d'au moins un diagramme polygonal multiaxial ou diagramme en étoile.
- le diagramme polygonal multiaxial supporte la représentation d'au moins deux groupes desdits coefficients, avec des attributs graphiques différents, en particulier de couleur, les deux groupes étant relatifs à des conditions de traitements au moins partiellement différentes.
- la représentation graphique comporte au moins deux diagrammes polygonaux multiaxiaux juxtaposés, correspondant à des conditions de traitements au moins partiellement différentes, ou des temps différents.

L'invention est également susceptible d'être exprimée sous forme de procédé.

Ainsi, elle peut être vue comme un procédé qui comporte les étapes suivantes :

P1 - afficher une silhouette d'un corps, sur un écran de tablette graphique, de préférence un écran de tablette tactile,

P2 - effectuer une auto-calibration patient pour déterminer les écarts entre une pluralité de points-cibles désignés par contact sur le corps du patient, et des points correspondants indiqués par le patient sur l'écran,

P3 - faire délimiter sur l'écran par un patient au moins une zone de la silhouette correspondant à un ressenti local de ce patient,

P4 - corriger cette délimitation en fonction des écarts d'auto-calibration patient.

L'étape P2 peut également comprendre une calibration patient/écran, capable de déterminer les écarts entre des points à viser sur l'écran et les points que le patient touche effectivement, ces écarts étant à combiner avec les écarts d'auto calibration patient.

Les étapes de l'utilisation de l'outil décrit ici, en ses différentes variantes, peuvent elles aussi être exprimées sous forme de procédé.

**Revendications**

1. Outil d'aide à l'évaluation/surveillance de la douleur, comprenant :

   - un outil graphique (TG), tel qu'une tablette, équipé d'un écran (102) et capable de dialoguer avec un serveur,
   - une application locale (TGapps) installée dans l'outil graphique, pour afficher à l'écran (102) une représentation cartographique (100) d'un corps d'un patient, tout en permettant à un usager tel que le patient de délimiter une zone (104) sur la représentation cartographique (100) affichée à l'écran (102),
   - une application pilote (TGappmain) coopérant avec l'application locale (TGapps) pour enregistrer (CCSapps) chaque zone délimitée (104) en association avec des données de surface correspondantes, **caractérisé en ce que** l'application pilote (TGappmain) est agencée pour piloter une fonction d'auto-calibration patient (appCalib2), capable de déterminer les écarts entre une pluralité de points-cibles désignés par contact sur le corps physique du patient, et des points correspondants indiqués par pointage par le patient sur la représentation cartographique (100) affichée à l'écran(102), et

   **en ce que** la délimitation sur l'écran (102) d'une zone (104) sur la représentation cartographique (100) par le patient est modifiée en fonction de ces écarts d'auto-calibration patient.

2. Outil selon la revendication 1, **caractérisé en ce que** les points-cibles sont désignés successivement par contact sur le corps physique du patient, tandis que le patient indique à chaque fois sur l'écran de la tablette graphique (TG) le point de la représentation cartographique (100) de son corps où il ressent le contact.

3. Outil selon l'une des revendications 1 et 2, **caractérisé en ce que** les points-cibles sont choisis parmi une liste prédéfinie de repères morphologiques.

4. Outil selon l'une des revendications précédentes, **caractérisé en ce que** la fonction d'auto-calibration patient (appCalib2) est répétée plusieurs fois pour un même patient.

5. Outil selon l'une des revendications précédentes, dans lequel la tablette graphique (TG) comprend un écran (102) tactile,

**caractérisé en ce que** l'application pilote (TGappmain) est également agencée pour piloter une fonction de calibration patient/écran (appCalib1), capable de déterminer les écarts entre des points à viser sur l'écran (102) et les zones de l'écran (102) tactile que le patient touche effectivement, ces écarts étant combinés avec les écarts d'auto calibration patient.

6. Outil selon la revendication 5, **caractérisé en ce que** les points à viser sur l'écran (102) comprennent au moins quatre points proches de quatre coins de l'écran (102) et un point au centre de l'écran (102).

7. Outil selon l'une des revendications précédentes, **caractérisé en ce que** l'application locale (TGapps) est agencée pour permettre une délimitation par le patient de zones de douleur (104) sur la représentation cartographique du corps ou silhouette (100) et d'afficher à l'écran (102) lesdites zones de douleur (104), et le calcul de données de surface correspondantes.

8. Outil selon la revendication 7, **caractérisé en ce que** la délimitation graphique de zones de douleur (104) par le patient sur la silhouette (100), est différenciée d'après l'intensité de la douleur d'une part, et le type de douleur d'une autre part.

9. Outil selon la revendication 8, **caractérisé en ce que** les types de douleurs comprennent certains au moins des types du groupe : Douleur Nociceptive, Douleur "Trigger" ("Gâchette"), Douleur Neuropathique, Douleur Mécanique.

10. Outil selon l'une des revendications 7 à 9, **caractérisé en ce que** l'application locale (TGapps) est agencée pour permettre également une délimitation graphique d'au moins une zone de paresthésies.

11. Outil selon la revendication 10, **caractérisé en ce que** l'application locale (TGapps) est agencée pour effectuer des calculs comparatifs entre la ou les surfaces de paresthésies et certaines au moins des surfaces de zones de douleur.

12. Outil selon l'une des revendications 7 à 11, **caractérisé en ce que** chaque délimitation graphique est réalisée par remplissage de zone avec des attributs graphiques sélectifs, en particulier de couleur, certaines au moins des zones étant partiellement transparentes.

13. Outil selon l'une des revendications 7 à 11, **caractérisé en ce que** l'application locale (TGapps) est agencée pour permettre d'associer une métrique réelle auxdites délimitations graphiques.

14. Outil selon la revendication 13, **caractérisé en ce que** la métrique réelle est obtenue en appliquant une échelle réelle à la silhouette (100) du patient, à partir d'une grandeur réelle mesurée, telle que la taille du patient.

15. Outil selon la revendication 13, **caractérisé en ce que** la métrique réelle est obtenue par traitement d'images du patient, issues de l'imagerie médicale.

16. Outil selon l'une des revendications 7 à 15, **caractérisé en ce que** les données de délimitation graphique et de surfaces sont rangées dans un historique daté rapporté à une fiche patient.

17. Outil selon l'une des revendications précédentes, **caractérisé en ce que** l'outil graphique est monté en réseau avec au moins un ordinateur central (CCS), d'une manière permettant la mobilité.

**Patentansprüche**

1. Hilfsgerät zur Bewertung/Überwachung von Schmerz, Folgendes umfassend:

- ein Grafik-Apparat (TG), wie etwa ein Tablet, das mit einem Bildschirm (102) ausgestattet ist und mit einem Server in Datenaustausch treten kann,
- eine lokale Anwendung (TGapps), die im Grafik-Apparat installiert ist, um auf dem Bildschirm (102) eine kartografische Darstellung (100) eines Körpers eines Patienten anzuzeigen und es dabei einem Nutzer, wie etwa dem Patienten, zu ermöglichen, einen Bereich (104) auf der am Bildschirm (102) eingeblendeten kartografischen Darstellung (100) einzugrenzen,
- eine Steueranwendung (TGappmain), die mit der lokalen Anwendung (TGapps) kooperiert, um jeden eingegrenzten Bereich (104) in Verbindung mit entsprechenden Oberflächendaten aufzuzeichnen (CCSapps),

**dadurch gekennzeichnet, dass** die Steueranwendung (TGappmain) dazu eingerichtet ist, eine Patientenautokalibrierungsfunktion (appCalib2) zu steuern, welche die Abweichungen zwischen mehreren Zielpunkten, die durch Kontakt am physischen Körper des Patienten bezeichnet werden, und entsprechenden Punkten bestimmen kann, die durch Zeigen des Patienten auf der am Bildschirm (102) eingeblendeten kartografischen Darstellung (100) angegeben werden, und dass die Eingrenzung eines Bereichs (104) in der kartografischen Darstellung (100) durch den Patienten auf dem Bildschirm (102) in Abhängigkeit von den Patientenautokalibrierungsabweichungen modifiziert wird.

2. Hilfsgerät nach Anspruch 1, **dadurch gekennzeichnet, dass** die Zielpunkte sukzessive durch Kontakt am physischen Körper des Patienten bezeichnet werden, während der Patient jedes Mal auf dem Bildschirm des Grafik-Tablets (TG) den Punkt der kartografischen Darstellung (100) seines Körpers angibt, wo er den Kontakt spürt.

3. Hilfsgerät nach einem der Ansprüche 1 und 2, **dadurch gekennzeichnet, dass** die Zielpunkte aus einer vordefinierten Liste morphologischer Orientierungspunkte ausgewählt werden.

4. Hilfsgerät nach einem der vorhergehenden Ansprüche, **dadurch gekennzeichnet, dass** die Patientenautokalibrierungsfunktion (appCalib2) mehrmals für denselben Patienten wiederholt wird.

5. Hilfsgerät nach einem der vorhergehenden Ansprüche, wobei das Grafik-Tablet (TG) einen berührungssensitiven Bildschirm (102) umfasst,
**dadurch gekennzeichnet, dass** die Steueranwendung (TGappmain) auch dazu eingerichtet ist, eine Patient/Bildschirm-Kalibrierungsfunktion (appCalib1) zu steuern, welche die Abweichungen zwischen den auf dem Bildschirm (102) anzuvisierenden Punkten und den Bereichen des berührungssensitiven Bildschirms (102) bestimmen kann, die der Patient tatsächlich berührt, wobei diese Abweichungen mit den Patientenautokalibrierungsabweichungen kombiniert werden.

6. Hilfsgerät nach Anspruch 5, **dadurch gekennzeichnet, dass** die auf dem Bildschirm (102) anzuvisierenden Punkte mindestens vier Punkte nahe den vier Ecken des Bildschirms (102) und einen Punkt in der Mitte des Bildschirms (102) umfassen.

7. Hilfsgerät nach einem der vorhergehenden Ansprüche, **dadurch gekennzeichnet, dass** die lokale Anwendung (TGapps) dazu eingerichtet ist, eine Eingrenzung von Schmerzbereichen (104) auf der kartografischen Darstellung (100) des Körpers oder der Silhouette durch den Patienten zu ermöglichen und diese Schmerzbereiche (104) auf dem Bildschirm (102) anzuzeigen, und die Berechnung entsprechender Oberflächendaten zu ermöglichen.

8. Hilfsgerät nach Anspruch 7, **dadurch gekennzeichnet, dass** die grafische Eingrenzung von Schmerzbereichen (104) auf der Silhouette (100) durch den Patienten je nach der Stärke des Schmerzes einerseits und der Schmerzart andererseits differenziert wird.

9. Hilfsgerät nach Anspruch 8, **dadurch gekennzeichnet, dass** die Arten von Schmerzen zumindest bestimmte Arten der Gruppe umfassen: nozizeptiver Schmerz, "Trigger"-Schmerz ("Auslöser"-Schmerz), neuropathischer Schmerz, mechanischer Schmerz.

10. Hilfsgerät nach einem der Ansprüche 7 bis 9, **dadurch gekennzeichnet, dass** die lokale Anwendung (TGapps) dazu eingerichtet ist, auch eine grafische Eingrenzung mindestens eines Bereichs von Parästhesien zu ermöglichen.

11. Hilfsgerät nach Anspruch 10, **dadurch gekennzeichnet, dass** die lokale Anwendung (TGapps) dazu eingerichtet ist, Vergleichsberechnungen zwischen der oder den Flächen von Parästhesien und zumindest bestimmten der Schmerzbereichflächen durchzuführen.

12. Hilfsgerät nach einem der Ansprüche 7 bis 11, **dadurch gekennzeichnet, dass** jede grafische Eingrenzung durch Ausfüllen des Bereichs mit ausgewählten grafischen Attributen, insbesondere Farbe, erfolgt, wobei zumindest einige der Bereiche teilweise transparent sind.

13. Hilfsgerät nach einem der Ansprüche 7 bis 11, **dadurch gekennzeichnet, dass** die lokale Anwendung (TGapps) dazu eingerichtet ist, zu ermöglichen, den grafischen Eingrenzungen eine reale Metrik zuzuordnen.

**14.** Hilfsgerät nach Anspruch 13, **dadurch gekennzeichnet, dass** die reale Metrik erhalten wird, indem ein realer Maßstab an die Silhouette (100) des Patienten ausgehend von einer realen gemessenen Größe, wie etwa der Körpergröße des Patienten, angelegt wird.

**15.** Hilfsgerät nach Anspruch 13, **dadurch gekennzeichnet, dass** die reale Metrik durch Bearbeitung von aus der medizinischen Bildgebung hervorgegangenen Bildern des Patienten erhalten wird.

**16.** Hilfsgerät nach einem der Ansprüche 7 bis 15, **dadurch gekennzeichnet, dass** die Daten der grafischen Eingrenzung und Flächen in einer datierten Vorgeschichte abgelegt werden, die in einem Patentendatenblatt eingetragen wird.

**17.** Hilfsgerät nach einem der vorhergehenden Ansprüche, **dadurch gekennzeichnet, dass** der Grafik-Apparat mit mindestens einem Zentralrechner (CCS) auf eine Weise vernetzt ist, die Mobilität ermöglicht.

**Claims**

**1.** A tool assisting in the evaluation/monitoring of pain, comprising:

- a graphics tool (TG), such as a tablet, equipped with a screen (102) and capable of interacting with a server,
- a local application (TGapps) installed in the graphics tool for the purpose of displaying on the screen (102) a cartographic representation (100) of a patient's body, while allowing a user, for example the patient, to delimit a zone (104) on the cartographic representation (100) displayed on the screen (102),
- a control application (TGappmain) cooperating with the local application (TGapps) in order to record (CCSapps) each delimited zone (104) together with corresponding surface data, **characterized in that** the control application (TGappmain) is configured to control a patient auto calibration function (appCalib2) capable of determining the deviations between a plurality of target points designated by contact on the physical body of the patient and corresponding points indicated by the patient pointing to the cartographic representation (100) displayed on the screen (102), and **in that** the delimitation, on the screen (102), of a zone (104) on the cartographic representation (100) by the patient is modified as a function of these patient auto calibration deviations.

**2.** The tool according to claim 1, **characterized in that** the target points are designated successively by contact on the physical body of the patient, while the patient indicates each time, on the screen of the graphics tablet, the point on the cartographic representation (100) of his body where he feels the contact.

**3.** The tool according to one of claims 1 and 2, **characterized in that** the target points are chosen from a predefined list of morphological markers.

**4.** The tool according to one of the preceding claims, **characterized in that** the patient auto calibration function (appCalib2) is repeated several times for the same patient.

**5.** The tool according to one of the preceding claims, wherein the graphics tablet (TG) comprises a touch-sensitive screen (102),
**characterized in that** the control application (TGappmain) is also configured to control a patient/screen calibration function (appCalib1) capable of determining the deviations between target points on the screen (102) and the zones of the touch-sensitive screen (102) that the patient actually touches, these deviations being combined with the patient auto calibration deviations.

**6.** The tool according to claim 5, **characterized in that** the target points on the screen (102) include at least four points near four corners of the screen (102) and one point at the center of the screen (102).

**7.** The tool according to one of the preceding claims, **characterized in that** the local application (TGapps) is configured to allow a patient to delimit pain zones (104) on the cartographic representation of the body or silhouette (100) and to display on the screen (102) said pain zones (104), and the calculation of corresponding surface data.

**8.** The tool according to claim 7, **characterized in that** the graphical delimitation of pain zones (104) by the patient on the silhouette (100) is differentiated according to, on the one hand, the intensity of the pain and, on the other hand, the type of pain.

9.  The tool according to claim 8, **characterized in that** the types of pain comprise at least some of the types from the following group: nociceptive pain, trigger pain, neuropathic pain, mechanical pain.

10. The tool according to one of claims 7 to 9, **characterized in that** the local application (TGapps) is configured to also allow a graphical delimitation of at least one paresthesia zone.

11. The tool according to claim 10, **characterized in that** the local application (TGapps) is configured to perform comparative calculations between the paresthesia surface area(s) and at least some of the surface areas of the pain zones.

12. The tool according to one of claims 7 to 11, **characterized in that** each graphical delimitation is realized by filling zones with selective graphical attributes, in particular of color, at least some of the zones being partially transparent.

13. The tool according to one of claims 7 to 11, **characterized in that** the local application (TGapps) is configured to allow a real metric to be assigned to said graphical delimitations.

14. The tool according to claim 13, **characterized in that** the real metric is obtained by applying a real scale to the silhouette (100) of the patient, on the basis of a measured real size, such as the height of the patient.

15. The tool according to claim 13, **characterized in that** the real metric is obtained by processing images of the patient that are obtained from medical imaging.

16. The tool according to one of claims 7 to 15, **characterized in that** the data relating to graphical delimitation and to surfaces are stored in a dated record attached to a patient file.

17. The tool according to one of the preceding claims, **characterized in that** the graphics tool is set up in a network with at least one central computer (CCS), in a manner permitting mobility.

# Fig. 1

~ 102

efface | calcule | sauve

~ 100

Code

09822

T7 [ ] % [ ] %
T8 [ ] % [ ] %
T9 [ ] % [ ] %
T10 [ ] % [ ] %
T11 [ ] % [ ] %
T12 [ ] % [ ] %

L1 [ ] % [ ] %
L2 [ ] % [ ] %
L3 [ ] % [ ] %
L4 [ ] % [ ] %
L5 [ ] % [ ] %

S1 [ ] % [ ] %
S2 [ ] % [ ] %
S3 [ ] % [ ] %

MSP [ ] % [ ] %
LSP [ ] % [ ] %

# Fig. 2

# Fig. 3

# Fig. 4

400

```
┌──────────┐
│    A1    │
└──────────┘
      │
      ▼
┌──────────┐
│    B1    │
└──────────┘
      │
      ▼
┌──────────┐
│    C1    │
└──────────┘
      │
      ▼
┌──────────┐
│    D1    │
└──────────┘
      │
      ▼
┌──────────┐
│    E1    │
└──────────┘
      │
      ▼
┌──────────┐
│    F1    │
└──────────┘
      │
      ▼
┌──────────┐
│    G1    │
└──────────┘
```

# Fig. 5

500

```
┌─────────┐
│   A2    │
└─────────┘
     │
     ▼
┌─────────┐
│   B2    │
└─────────┘
     │
     ▼
┌─────────┐
│   C2    │
└─────────┘
     │
     ▼
┌─────────┐
│   D2    │
└─────────┘
     │
     ▼
┌─────────┐
│   E2    │
└─────────┘
     │
     ▼
┌─────────┐
│   F2    │
└─────────┘
     │
     ▼
┌─────────┐
│   G2    │
└─────────┘
     │
     ▼
┌─────────┐
│   H2    │
└─────────┘
     │
     ▼
┌─────────┐
│   I2    │
└─────────┘
```

# Fig. 6

# Fig. 7

# Fig.8

| 1 | DONNÉES PATIENT | 🌐 |

| 1 DONNÉES |  |
| 2 IMAGES |  |
| 3 CALIBR. 1 |  |
| 4 CALIBR. 2 |  |
| 5 CARTOGRAPHIE |  |
| 6 LOCALISATEUR |  |

Code patient: **Bou-vin**   Liste des patients   Nouveau patient

Caractéristiques générales

Sexe: | Date de naissance: | Âge:

M J A
**01** **31** **1973** **41**

Caractéristiques morphométriques

Taille: | Poids
cm | pieds | pouces | kg | livres | IMC (kg.m$^{-2}$)
**185** | **6** | **0,8** | **82** | **180** | **23.96**

Caractéristiques relatives à la douleur

Étiologie de la douleur | **Lombo-radiculalgies chroniques (LRC)** ▼

Localisation de la douleur | **Douleur du dos et des jambes** ▼

Type d'électrode n°1 | **8 contacts** ▼

Type d'électrode n°2 | **Choisir l'électrode n°2** ▼

Type d'électrode n°3 | **Choisir l'électrode n°3** ▼

Type d'électrode n°4 | **Choisir l'électrode n°4** ▼

Réinitialiser

Sauvegarder et poursuivre

51

# Fig.8bis

| 1 | DONNÉES PATIENT | |

| | | |
|---|---|---|
| 1 | DONNÉES | |
| 2 | IMAGES | |
| 3 | CALIBR. 1 | |
| 4 | CALIBR. 2 | |
| 5 | CARTOGRAPHIE | |
| 6 | LOCALISATEUR | |

Code patient:  **Bou-vin**   Liste des patients   Nouveau patient

Caractéristiques générales

Sexe:          Date de naissance:          Âge:

M    J    A

**01**  **31**  **1973**          **41**

Caractéristiques morphométriques

Taille:                    Poids

cm    pieds    pouces    kg    livres    IMC (kg.m$^{-2}$)

**185**  **6**  **0,8**  **82**  **180**  **23.96**

Caractéristiques relatives à la douleur

Étiologie de la douleur    **Lombo-radiculalgies chroniques (LRC)** ▼

Localisation de la douleur    **Douleur du dos et des jambes** ▼

Type d'électrode n°1    **8 contacts** ▼

Type d'électrode n°2    **Choisir l'électrode n°2** ▼

Type d'électrode n°3    **Choisir l'électrode n°3** ▼

Type d'électrode n°4    **Choisir l'électrode n°4** ▼

Réinitialiser          Sauvegarder et poursuivre

# Fig.9

| 2 | IMAGES PATIENT | |

Type d'acquisition: **Vue schématique ▼**

| | | |
|---|---|---|
| **1** | DONNÉES | |
| **2** | IMAGES | |
| **3** | CALIBR. 1 | |
| **4** | CALIBR. 2 | |
| **5** | CARTOGRAPHIE | |
| **6** | LOCALISATEUR | |

Télécharger les images

Téléchargement OK

# Fig.9bis

| 2 | IMAGES PATIENT | 🌐 |
|---|---|---|

Type d'acquisition: **Vue schématique ▼**

Télécharger les images

Téléchargement OK

Left tabs: 1 DONNÉES, 2 IMAGES, 3 CALIBR. 1, 4 CALIBR. 2, 5 CARTOGRAPHIE, 6 LOCALISATEUR

# CALIBRATION ÉCRAN

**Fig.10**

| 3 | CALIBRATION ÉCRAN |
|---|---|
| 1 | Calibration en Cours |
| 2 | POURSUIVRE |
| 3 | |
| 4 | |
| 5 | |
| 6 | |

Toucher le point-cible

| 3 | CALIBRATION ÉCRAN |
|---|---|

CALIBRATION OK

| Δy moyen = -0,95 Ét = 0,15 | Δx moyen = -1,27 Ét = 0,22 |

Si le patient est hors plage

Réinitialiser

EP 3 043 706 B1

**Fig.10bis**

# Fig.11

## CALIBRATION PATIENT

| 4 | AUTOCALIBRATION PATIENT |
|---|---|

| | | |
|---|---|---|
| 1 DONNÉES | Calibration rapide (x1) | Calibration optimisée (x5) |
| 2 IMAGES | | |
| 3 CALIBR. 1 | ✖ Calibration en Cours | |
| 4 CALIBR. 2 | | |
| 5 CARTOGRAPHIE | | |
| 6 LOCALISATEUR | POURSUIVRE | |

# Fig.11bis

## CALIBRATION PATIENT 2

| 4 | AUTOCALIBRATION PATIENT |
|---|---|

| | | |
|---|---|---|
| 1 DONNÉES | Calibration rapide (x1) | Calibration optimisée (x5) |
| 2 IMAGES | | |
| 3 CALIBR. 1 | ✖ Calibration en Cours | |
| 4 CALIBR. 2 | | |
| 5 CARTOGRAPHIE | POURSUIVRE | |
| 6 LOCALISATEUR | | |

# Fig. 12

Marquage
des cibles de
calibration
au feutre sur
le patient

Filtrage de
la photo
pour
l'affichage

Représentation
par le patient des
points ressenties

# Fig. 12bis

Marquage
des cibles de
calibration
au feutre sur
le patient

Filtrage de
la photo
pour
l'affichage

Représentation
par le patient des
points ressenties

# Fig.13

Calibration optimisée

Calibration RAPIDE

# Fig.13bis

| INTENSITÉ | TYPOLOGIE | PARES/THÉSES | RÉSUMÉ | — |
| FAIBLE | MOYENNE | INTENSE | TRÈS INTENSE | |

Calibration optimisée

Calibration RAPIDE

| 4 | AUTO-CALIBRATION PATIENT |
|---|---|

| 1 DONNÉES | Δx moyen;Ét / Δy moyen;Ét | | Δx moyen;Ét / Δy moyen;Ét |
| 2 IMAGES | Δx moyen;Ét / Δy moyen;Ét | | Δx moyen:Ét / Δy moyen;Ét |
| 3 CALIBR. 1 | Δx moyen;Ét / Δy moyen;Ét | | Δx moyen;Ét / Δy moyen;Ét |
| 4 CALIBR. 2 | | | |
| 5 CARTOGRAPHIE | | | |
| 6 LOCALISATEUR | | | |

GROUPE 1  GROUPE 2  GROUPE 3

✔ Calibration OK

Réinitialiser

Δx moyen;Ét
Δy moyen;Ét

# Fig.14

| 5 | CARTOGRAPHIE | |
|---|---|---|

| 1 | DONNÉES |
|---|---|
| 2 | IMAGES |
| 3 | CALIBR. 1 |
| 4 | CALIBR. 2 |
| 5 | CARTOGRAPHIE |
| 4 | LOCALISATEUR |

Télécharger une consultation précédente

Créer une nouvelle consultation

$M_{-1}$

$M_0$

$M_{+1}$

$M_{+2}$

$M_{+3}$

$M_{+6}$

$M_{+12}$

$M_{+24}$

Autre

POURSUIVRE

# Fig.14bis

| 5 | CARTOGRAPHIE | |

**Télécharger une consultation précédente**

**Créer une nouvelle consultation**

| 1 DONNÉES |
| 2 IMAGES |
| 3 CALIBR. 1 |
| 4 CALIBR. 2 |
| 5 CARTOGRAPHIE |
| 4 LOCALISATEUR |

$M_{-1}$

$M_0$

$M_{+1}$

$M_{+2}$

$M_{+3}$

$M_{+6}$

$M_{+12}$

$M_{+24}$

Autre

POURSUIVRE

# Fig.15

INTENSITÉ   TYPOLOGIE   RÉSUMÉ

| FAIBLE | MOYENNE | INTENSE | TRÈS INTENSE | DONNÉES |
|--------|---------|---------|--------------|---------|
| 29,6% | 10,6% | 26,3% | 33,2% | |
| 193cm$^2$ | 69cm$^2$ | 171cm$^2$ | 217cm$^2$ | |

Données de M$_{-1}$

# Fig.15bis

| INTENSITÉ | TYPOLOGIE | RÉSUMÉ |
|---|---|---|

| FAIBLE | MOYENNE | INTENSE | TRÈS INTENSE |
|---|---|---|---|
| 29,6% | 10,6% | 26,3% | 33,2% |
| 193cm² | 69cm² | 171cm² | 217cm² |

DONNÉES

Données de $M_{-1}$

# Fig.16

INTENSITÉ | TYPOLOGIE | RÉSUMÉ

NOCICEPTIVE | GÂCHETTE | NEUROPATHIQUE

27,2% | 7,5% | 67,1%

Douleur Mécanique
34,8%

DONNÉES | TOUT VOIR

Données de $M_{-1}$

# Fig.16bis

# Fig.17

# Fig.17bis

| INTENSITÉ | TYPOLOGIE | PARESTHÉSIES | RÉSUMÉ |
|---|---|---|---|

Performance 99,7%  Spécificité 75,9%

SAUVEGARDER PARESTHÉSIES
VOIR HYBRIDE
VOIR INTENSITÉ
RÉINITIALISER PAR.

Données de $M_0$

## Fig. 18

# Fig. 18bis

Performance

Sélectivité

Coef Perf
CP = 0,82

Coef Perf Pondéré
CPP = 0,57

Coef sélectivité
CS = 0,72

# Fig. 19

# Fig. 19bis

# Fig. 20

# Fig. 20bis

Visite Postopératoire
M0

Visite
M0 + 1 MOIS

Visite
M0 + 3 MOIS

Visite
M0 + 6 MOIS

# Fig. 21

# Fig. 21bis

☐ PROGRAMME 1    ☐ PROGRAMME 2

# Fig. 22

# Fig. 22bis

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8046241 B, Dodson
- US 7374536 B, Taylor
- US 20090005649 A, Baird
- US 20010007950 A, North
- WO 2004041080 A
- FR 1358850

**Littérature non-brevet citée dans la description**

- **BOUHASSIRA et al.** Comparison of pain syndromes associated with nervous or somatic lesions and development of a new neuropathic pain diagnostic questionnaire (DN4). *Pain,* 2005, vol. 114, 29-36
- **STRUIJK JJ. ; HOLSHEIMER, J.** Transverse tripolar spinal cord stimulation: theoritical performance of a dual channel system. *Med Biol Eng Comput,* 1996, vol. 34 (4), 273-279
- **NORTH RB et al.** Spinal cord stimulation versus repeated lumbosacral spine surgery for chronic pain: a randomized, controlled trial. *Neurosurgery,* 2005, vol. 56, 98-106
- **KUMAR K. et al.** Spinal cord stimulation versus conventional medical management for neuropathic pain: a multicentre randomized controlled trial in patients with failed back surgery syndrome. *Pain,* 2007, vol. 132, 179-88
- **KUMAR K. et al.** The effects of spinal cord stimulation in neuropathic pain are sustained: a 24-month follow-up of the prospective randomized controlled multicenter trial of the effectiveness of spinal cord stimulation. *Neurosurgery,* 2008, vol. 63, 762-70
- **P. RIGOARD et al.** Back Pain, A Real Target for Spinal Cord Stimulation. *Neurosurgery,* 2012, vol. 70, 574-585